(19) Europäisches Patentamt
European Patent Office
Office européen des brevets

(11) **EP 3 847 459 B1**

(12) # EUROPEAN PATENT SPECIFICATION

(45) Date of publication and mention
of the grant of the patent:
**26.02.2025  Bulletin 2025/09**

(21) Application number: **19858371.8**

(22) Date of filing: **06.09.2019**

(51) International Patent Classification (IPC):
**G01N 33/497** (2006.01)     **G01N 33/64** (2006.01)
**G01N 31/22** (2006.01)      **A61B 5/08** (2006.01)
**A61B 5/097** (2006.01)      **G01N 21/78** (2006.01)
**G01N 21/77** (2006.01)      **G01N 21/80** (2006.01)

(52) Cooperative Patent Classification (CPC):
**G01N 31/22;** G01N 21/78; G01N 21/80;
G01N 2021/7786; G01N 2021/7793;
G01N 2201/1241

(86) International application number:
**PCT/AU2019/050955**

(87) International publication number:
**WO 2020/047606 (12.03.2020 Gazette 2020/11)**

---

(54) **SYSTEMS, SENSORS AND METHODS FOR DETERMINING A CONCENTRATION OF AN ANALYTE**

SYSTEME, SENSOREN UND VERFAHREN ZUR BESTIMMUNG EINER KONZENTRATION EINES ANALYTEN

SYSTÈMES, CAPTEURS ET PROCÉDÉS DE DÉTERMINATION D'UNE CONCENTRATION D'UN ANALYTE

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.09.2018  AU 2018903323
20.12.2018  AU 2018904856
20.06.2019  AU 2019902157**

(43) Date of publication of application:
**14.07.2021  Bulletin 2021/28**

(73) Proprietor: **Ausmed Global Limited
Hong Kong (CN)**

(72) Inventors:
• **YI, Xiaoke
Sydney, New South Wales 2006 (AU)**
• **COLLINS, Michael James
Sydney, New South Wales 2006 (AU)**
• **WU, Jiarong
Sydney, New South Wales 2006 (AU)**
• **HAN, Yang
Sydney, New South Wales 2006 (AU)**
• **AUSTIN, Mitchell
Sydney, New South Wales 2006 (AU)**

(74) Representative: **Boult Wade Tennant LLP
Salisbury Square House
8 Salisbury Square
London EC4Y 8AP (GB)**

(56) References cited:
EP-A1- 3 260 860          WO-A1-2018/027309
WO-A1-2018/081877      BR-A- 8 704 258
JP-B2- 6 171 470           SU-A1- 877 425
US-A1- 2004 043 527    US-A1- 2007 048 180
US-A1- 2009 049 890    US-A1- 2012 156 099
US-A1- 2016 033 450    US-A1- 2017 284 999
US-A1- 2018 056 302

---

EP 3 847 459 B1

**Description**

**Field**

The invention relates to systems, sensors and methods for determining a concentration of acetone.

**Background**

Sensors are used to sense various analytes for a variety of applications. These analytes may be biomarkers, contaminants, narcotics, or any other species where there is an interest in their detection and/or quantification. Such analytes may, for example, be biomarkers, where their presence, absence, or concentration in a sample may be indicative of a disease or condition.

For example, human chorionic gonadotropin (hCG) is a biomarker found in urine and blood after implantation during pregnancy. The presence of hCG in urine can indicate pregnancy in an individual, and this principle is used in pregnancy tests that use a sensor able to detect hCG in urine.

A sensor for sensing an analyte generally requires suitable sensitivity for the analyte in order to limit false negative results. For example, the EPA allowable limit for lead in drinking water is 15 ppb. Accordingly, a sensor for detecting lead in drinking water must be sensitive enough to reliably detect lead at least down to a concentration of 15 ppb.

Additionally, a sensor must have a suitable level of selectivity to the analyte in order to limit false positive results. For example, a sensor sensitive enough to detect lead in drinking water below 15 ppb will be of little use in that application if it has poor selectivity for lead. If the sensor responds similarly to sodium in drinking water, which is typically present in drinking water in much higher concentrations than lead, then such a sensor would yield many false positive results.

A sensor must also have suitable precision and accuracy for sensing the analyte depending upon the application area and sensing requirements. If a sensor is required only for detecting the presence or absence of an analyte, i.e. a qualitative sensing, the minimum precision and accuracy levels of the sensor may be less than if the sensor is to be used for quantifying the amount of the analyte.

Sensors in the prior art may have suitable sensitivity, selectivity, precision and/or accuracy for sensing an analyte, but only within a narrow analyte concentration range. For many analytes, however, it is necessary to detect and/or quantify their concentration over a wide variety of concentrations. Accordingly, there is a need to develop sensors and systems comprising sensors that have a wide dynamic range and/or a wide working range for sensing analytes at a variety of concentrations. Some sensors may meet these requirements, but may have a high equipment cost, for example they may require incorporation in a GC-MS. Further, it may be slow to determine an accurate concentration of an analyte using such sensors.

Low-cost sensors may suffer from one or more of difficult calibration yielding poor accuracy, poor repeatability yielding poor precision, poor selectivity, poor sensitivity, a narrow dynamic range and/or a narrow working range. Therefore, there is a need to develop low cost sensors and systems comprising low cost sensors having improvements in any one or more of these properties. There is also a need to develop sensors and systems comprising sensors suitable for rapid tests.

Sensors for certain biomarkers may only be effective for invasively collected samples, such as blood. For example, sensors for determining blood glucose levels for patients with diabetes are accurate and enable quantitative glucose concentration measurement, but only for invasively collected blood samples. Accordingly, there is also a need for sensors and systems comprising sensors with improved sensitivity, reliability and/or affordability that enable detection or quantitation of biomarkers in non-invasively collected samples, for example in urine, saliva, or breath.

For example, diabetes mellitus, commonly shortened to diabetes, is a serious health concern globally, and its prevalence and diabetes-related deaths have been increasing with rapid changes in lifestyle resulting in an increase in risk factors such as obesity. Recent estimates have shown the prevalence of diabetes is likely to continue to grow substantially in the coming decades. For instance, in 2000 approximately 1.0 million people in Australia aged over 25 years had diabetes. This figure is projected to reach 2.0 - 2.9 million by 2025, with the worldwide incidence of diabetes of 336 million in 2011 projected to grow to 552 million in 2030. The costs of diabetes are substantial to both individuals and governments, especially when complications occur. It is apparent that a cost-effective treatment of diabetes has tremendous potential to relieve pressure on healthcare systems.

Diabetes is caused by the body's inability to produce the hormone insulin (type 1), or the body's inability to utilise it (type 2). Diabetics can suffer from low insulin levels and the consequent elevated level of blood glucose results in an increase in the body's ketone body concentration. Ketone bodies are carbonyl containing compounds: acetoacetate, beta-hydroxybutyrate and acetone. Increased ketone body concentration in the body can cause diabetic ketoacidosis, a state of insulin deficiency that can progress rapidly and is considered a medical emergency. If left untreated it can result in coma or death due to the acidification of blood. Although this life-threatening condition primarily occurs in patients with newly diagnosed type 1 diabetes, it can also occur in patients with type 2 diabetes. Therefore, there is a need for new methods, sensors and systems suitable for monitoring levels of analytes, such as carbonyl-containing compounds, for example, ketone bodies.

A system, sensor, or method for determining the concentration of an analyte, such as a carbonyl containing compound (e.g. ketone bodies) would generally require suitable sensitivity for the carbonyl containing compound in order to limit false negative results. Additionally, such a system, sensor or method must have a suitable level of selectivity to the carbonyl containing compound in order to limit false positive results. A system, sensor or method should also have suitable precision and accuracy for determining the concentration of the carbonyl containing compound.

Devices and methods in the prior art for determining the concentration of carbonyl containing compounds (e.g. ketone bodies), have suitable sensitivity, selectivity, precision and/or accuracy, but only within a narrow analyte concentration range. For particular carbonyl containing compounds, however, it may be necessary to detect and/or quantify their concentration over a wide variety of concentrations. Accordingly, there is a need to develop systems, sensors and methods that have a wide dynamic range and/or a wide working range for determining analyte concentration, e.g., carbonyl containing compound concentration at a variety of concentrations. Some devices or methods meet these requirements, but have a high equipment cost, for example they may require incorporation in a GC-MS. Further, it may be slow to determine an accurate concentration of a carbonyl containing compound using such devices or methods.

Low-cost systems and sensors for determining various analyte concentrations, including carbonyl containing compound concentrations, commonly suffer from one or more of: difficult calibration yielding poor accuracy, poor repeatability yielding poor precision, poor selectivity, poor sensitivity, a narrow dynamic range and/or a narrow working range. Therefore, there is a need to develop low cost systems and sensors, and methods using low cost systems and sensors having improvements in one or more of these properties. There is also a need to develop sensors, systems and methods for determining analyte concentrations, such as carbonyl containing compound concentrations, that are suitable for rapid tests.

Many devices and methods for determining the concentration of diabetes biomarkers, such as ketone bodies, are only be effective for invasively collected samples, such as blood. For example, devices for determining blood glucose levels for patients with diabetes are accurate and enable quantitative glucose concentration measurement, but only for invasively collected blood samples. Accordingly, there is also a need for systems, devices, and methods with improved sensitivity, reliability and/or affordability that enable determination of the concentration of, e.g., diabetes biomarkers, such as ketone bodies, in non-invasively collected samples, for example in urine, saliva, or breath. There is also a need for rapid and accurate detection methods for analytes, such as carbonyl-containing compounds (e.g. ketone bodies), for various ketone body monitoring application, such fitness, dieting, and metabolic therapy treatments for a variety of diseases related to ketone body levels, such as diabetes and Alzheimer's disease.

It will be apparent to the skilled person that suitable detection methods for analytes such as carbonyl-containing compounds, may also be suitable for the detection of various other non-carbonyl-containing compound analytes, and further that suitable detection methods for carbonyl-containing compounds, such as ketone bodies for diabetes monitoring, may also be suitable for the detection of various other carbonyl-containing compound analytes, such as pentosidine and acetaldehyde.

It is an aim of the present invention to at least partially satisfy at least one of the above needs.

SU877425A1 relates to an indicator composition comprising 2,4-dinitrophenyl hydrazine for the determination of acetone in air.

BR8704258A relates to an indicator composition and device for the determination of acetone in breath.

WO2018/081877 relates to a device for monitoring acetone levels in breath.

## Summary of Invention

According to a first aspect there is provided a system for determining a concentration of acetone in a fluid, said system comprising:

a sensor configured to generate a differential response to a constant concentration of the acetone, wherein the sensor comprises a hydroxylammonium salt, and whereby exposure of the fluid to the hydroxylammonium salt in the presence of a liquid produces a change in a variable, said variable being dependent upon the pH of the liquid; and

an acquisition device configured to determine the concentration of the acetone from the differential response, wherein the acquisition device comprises:

a detector for measuring the change in the variable; and

a processor configured to calculate the concentration of the acetone in the fluid from the change in the variable, optionally wherein the fluid is breath;

wherein the differential response is spatially differential, whereby the sensor is configured to respond to the acetone at a plurality of detection positions on the sensor, said detection positions having different sensitivities to the acetone; and

wherein:
the sensor comprises a spatially differential local concentration or local amount of the hydroxylammonium salt at different points of the sensor so as to provide spatially differential sensitivity to the sensor; or

the sensor comprises a coating on a detection surface, and the coating has spatially differential permeability to the acetone so as to provide spatially differential sensitivity to the sensor.

The following options may be used in conjunction with the first aspect, either individually or in any suitable combination.

The differential response may be temporally differential, whereby the response varies over time.

The sensitivity to the acetone may vary along a length of the sensor. It may vary monotonically along the length of the sensor. It may vary linearly or logarithmically along the length of the sensor. The plurality of detection positions may be spaced equidistantly along the length of the sensor.

The sensor may comprise, for example, at least three detection positions having different sensitivities to the acetone.

The sensor is configured to respond to the acetone at a plurality of detection positions on the sensor; said detection positions having different sensitivities to the acetone; and the acquisition device may be configured to calculate the concentration of the acetone utilising at least the following:

when the sensor reaches a saturated response level at all of the plurality of detection positions on the sensor:
a response of the sensor to the acetone as a function of time at the detection position on the sensor having the least sensitivity to the acetone;

when the sensor does not reach a saturated response level at any of the plurality of detection positions on the sensor:
a response of the sensor to the acetone at the detection position on the sensor having the greatest sensitivity to the acetone; and

if the sensor responds to the acetone to reach a saturated response level at some, but not all, of the plurality of detection positions on the sensor:
one or more of:

a ratio of the number of detection positions on the sensor that reach a saturated response level to the number of detection positions on the sensor that do not reach a saturated response level,

a time required to reach a saturated response level for at least one of the detection positions on the sensor that reaches a saturated response level, and

a response of the sensor to the acetone for at least one of the detection positions on the sensor that does not reach a saturated response level.

The system may further comprise a flow channel or an analysis chamber, wherein the sensor is disposed within said flow channel or analysis chamber.

The analyte is a carbonyl-containing compound, namely acetone. The sensor comprises a hydroxylammonium salt, whereby exposure of the fluid to the hydroxylammonium salt in the presence of a liquid produces a change in a variable, said variable being dependent upon the pH of the liquid. The acquisition device comprises a detector for measuring the change in the variable, and a processor configured to calculate the concentration of the acetone in the fluid from the change in the variable. The detector may be an ion sensitive field effect transistor (ISFET), chemiresistive sensor, potentiometric sensor, spectroscopic sensor, colorimetric sensor, fluorometric sensor, thermal sensor, or a combination thereof.

The sensor may comprise a sorbent material, whereby the hydroxylammonium salt is sorbed in and/or on the sorbent material. The system may further comprise a plurality of electrodes configured to apply an electric field across a surface of the sorbent material so as to concentrate one or more charged species on and/or in the sorbent material. The sorbent material may further comprise a weak base sorbed thereon and/or therein. The system may comprise an indicator which is sorbed in and/or on the sorbent material. The liquid volume may be about 1 mL or less.

The variable may be an absorbance, fluorescence, resistance, ion concentration, temperature, voltage, or current. The ion concentration may be, for example, a hydronium ion concentration.

In certain embodiments, the change in the variable is not measured using a titration.

The hydroxylammonium salt may, for example, be selected from the group consisting of hydroxylammonium chloride, hydroxylammonium sulfate, hydroxylammonium azide, hydroxylamine picrate, hydroxylamine benzenesulfonate, hydroxylamine benzenesulfinate, hydroxylamine 1-sulfobutyl-3-methyl imidazole hydrosulphate salt, hydroxylammonium phosphate, hydroxylamine nitrate, and mixtures thereof.

The fluid may be breath.

In a specific embodiment there is provided a system for determining a concentration of an acetone in breath, said system comprising a sensor configured to generate a spatially differential response to a constant concentration of acetone, whereby the sensor is configured to respond to acetone at a plurality of detection positions on the sensor, said detection positions having different sensitivities to the acetone, wherein the sensor comprises a hydroxylamine, hydroxylamine salt, or combination thereof, whereby exposure of the breath to the hydroxylamine, hydroxylamine salt, or combination thereof produces a change in a variable, said variable being dependent upon the pH of the liquid; and an acquisition device configured to determine the concentration of the acetone from the differential response, said acquisition device comprising a detector for measuring the change in the variable, and a processor configured to calculate the concentration of the acetone in the breath from the change in the variable,

wherein the acquisition device is configured to calculate the concentration of the acetone utilising at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

    a response of the sensor to the acetone at the detection position on the sensor having the greatest sensitivity to the acetone; or

    a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
  one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level.

In a specific embodiment there is provided a system for determining a concentration of an acetone in breath, said system comprising a sensor configured to generate a spatially differential response to a constant concentration of acetone, whereby the sensor is configured to respond to acetone at a plurality of detection positions on the sensor, said detection positions having different sensitivities to the acetone, wherein the sensor comprises a hydroxylamine, hydroxylamine salt, or combination thereof, whereby exposure of the breath to the hydroxylamine, hydroxylamine salt, or combination thereof produces a change in a variable, said variable being dependent upon the pH of the liquid; and an acquisition device configured to determine the concentration of the acetone from the differential response, said acquisition device comprising a detector for measuring the change in the variable, and a processor configured to calculate the concentration of the acetone in the breath from the change in the variable,

wherein the acquisition device is configured to calculate the concentration of the acetone utilising at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

    a response of the sensor to the acetone at the detection position on the sensor having the greatest sensitivity to the acetone; or
a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
  one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level;

wherein the sensor comprises a sorbent material, wherein the hydroxylamine, hydroxylamine salt, or combination thereof is sorbed in and/or on the sorbent material, wherein the sensor comprises a halochromic indicator which is sorbed in and/or on the sorbent material, wherein the variable is an absorbance, and the detector is a colorimetric sensor.

In a specific embodiment there is provided a system for determining a concentration of an acetone in breath, said system comprising a sensor configured to generate a spatially differential response to a constant concentration of acetone, whereby the sensor is configured to respond to acetone at a plurality of detection positions on the sensor, said detection positions having different sensitivities to the acetone, wherein the sensor comprises a hydroxylamine, hydroxylamine salt, or combination thereof, whereby exposure of the breath to the hydroxylamine, hydroxylamine salt, or combination thereof produces a change in a variable, said variable being dependent upon the pH of the liquid; and an acquisition device configured to determine the concentration of the acetone from the differential response, said acquisition device comprising a detector for measuring the change in the variable, and a processor configured to calculate the concentration of the acetone in the breath from the change in the variable,

wherein the acquisition device is configured to calculate the concentration of the acetone utilising at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

  a response of the sensor to the acetone at the detection position on the sensor having the greatest sensitivity to the acetone; or

  a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
  one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level;

wherein the sensor comprises a sorbent material, wherein the hydroxylamine, hydroxylamine salt, or combination thereof is sorbed in and/or on the sorbent material, wherein the sensor comprises a halochromic indicator which is sorbed in and/or on the sorbent material, wherein the variable is an absorbance, and the detector is a colorimetric sensor;

wherein the liquid volume is about 1 ml or less, and wherein the change in the absorbance is not measured using a titration.

In a specific embodiment there is provided a system for determining a concentration of an acetone in breath, said system comprising a sensor configured to generate a spatially differential response to a constant concentration of acetone, whereby the sensor is configured to respond to acetone at a plurality of detection positions on the sensor, said detection positions having different sensitivities to the acetone, wherein the sensor comprises a hydroxylamine, hydroxylamine salt, or combination thereof, whereby exposure of the breath to the hydroxylamine, hydroxylamine salt, or combination thereof produces a change in a variable, said variable being dependent upon the pH of the liquid; and an acquisition device configured to determine the concentration of the acetone from the differential response, said acquisition device comprising a detector for measuring the change in the variable, and a processor configured to calculate the concentration of the acetone in the breath from the change in the variable,

wherein the acquisition device is configured to calculate the concentration of the acetone utilising at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

  a response of the sensor to the acetone at the detection position on the sensor having the greatest sensitivity to the acetone; or

  a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
  one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level;

wherein the sensor comprises a sorbent material, wherein the hydroxylamine, hydroxylamine salt, or combination thereof is sorbed in and/or on the sorbent material, wherein the variable is a hydronium ion concentration, and the change in hydronium ion concentration is measured using an ion sensitive filed effect transistor, chemiresistive sensor, or potentiometric sensor;

wherein the liquid volume is about 1 ml or less, and wherein the change in the hydronium ion concentration is not measured using a titration.

According to a second aspect there is provided a method of determining a concentration of acetone in a fluid, the method comprising:

exposing the fluid to a sensor as defined according to the first aspect, which is configured to generate a differential response to a constant concentration of acetone; and

measuring a differential response of the sensor to the acetone, and

(c) calculating the concentration of the acetone in the fluid utilising the differential response of the sensor to the acetone; wherein the method further comprises:

(i) exposing the fluid to a hydroxylammonium salt in the presence of a liquid, so as to produce a change in a variable, said variable being dependent upon the pH of the liquid;

(ii) measuring the change in the variable; and

(iii) determining the concentration of the acetone in the fluid from the change in the variable;

optionally wherein the fluid is breath;

wherein the differential response is spatially differential, whereby the sensor is configured to respond to the acetone at a plurality of detection positions on the sensor; said detection positions having different sensitivities to the acetone; and wherein said calculating utilises at least the following:

- if the sensor reaches a saturated response level at all of the plurality of detection positions on the sensor:
  a response of the sensor to the acetone as a function of time at a detection position on the sensor having the least sensitivity to the acetone;

- if the sensor does not reach a saturated response level at any of the plurality of detection positions on the sensor:
  a response of the sensor to the acetone at a detection position on the sensor having the greatest sensitivity to the acetone; or

- if the sensor responds to the acetone to reach a saturated response level at some, but not all, of the plurality of detection positions on the sensor:
  one or more of:

  a ratio of the number of detection positions on the sensor that reach a saturated response level to the number of detection positions on the sensor that do not reach a saturated response level,

  a time required to reach a saturated response level for at least one detection position on the sensor that reaches a saturated response level, and

  a response of the sensor to the acetone for at least one detection position on the sensor that does not reach a saturated response level.

The following options may be used in conjunction with the second aspect, either individually or in any suitable combination.

The differential response may be temporally differential, and the calculating may utilise at least one or more of the following:

a time required to reach a predetermined response level for at least one position on the sensor after exposure of the fluid to the sensor; and
a response level for at least one position on the sensor at a predetermined time after exposure of the fluid to the sensor.

The hydroxylammonium salt may be sorbed in and/or on a sorbent material. An indicator may be sorbed in and/or on the sorbent material.

The hydroxylammonium salt and/or the indicator may be in contact with a weak base.

The change in the variable may be substantially independent of the volume of the fluid exposed to the hydroxylammonium salt and/or the change in the variable is substantially independent of the flow rate of the fluid exposed to the hydroxylammonium salt. The change in the variable may be measured using an ion sensitive field effect transistor (ISFET), chemiresistive sensor, potentiometric sensor, spectroscopic sensor, colorimetric sensor, fluorometric sensor, thermal sensor, or a combination thereof.

The variable may be an absorbance, fluorescence, resistance, ion concentration, temperature, voltage, or current. The ion concentration may, for example, be a hydronium ion concentration.

The liquid volume may be about 1 mL or less.

In certain embodiments, the change in the variable is not measured using a titration.

The hydroxylammonium salt may, for example, be selected from the group consisting of hydroxylammonium chloride, hydroxylammonium sulfate, hydroxylammonium azide, hydroxylamine picrate, hydroxylamine benzenesulfonate, hydroxylamine benzenesulfinate, hydroxylamine 1-sulfobutyl-3-methyl imidazole hydrosulphate salt, hydroxylammonium phosphate, hydroxylamine nitrate, and mixtures thereof.

The fluid may be breath.

In a specific embodiment, there is provided a method of determining a concentration of acetone in breath, the method comprising:

exposing the breath to a sensor which is configured to generate a differential response to a constant concentration of the acetone; and
measuring a differential response of the sensor to the acetone;

whereby the method further comprises a step of calculating the concentration of the acetone utilising the differential response of the sensor to the acetone;

wherein the differential response is spatially differential, whereby the sensor is configured to respond to the acetone at a plurality of detection positions on the sensor; said detection positions having different sensitivities to the acetone; and

wherein said calculating utilises at least the following:

- if the sensor reaches a saturated response level at all of the plurality of detection positions on the sensor:
a response of the sensor to the acetone as a function of time at the detection position on the sensor having the least sensitivity to the acetone;

- if the sensor does not reach a saturated response level at any of the plurality of detection positions on the sensor:

    a response of the sensor to the acetone at the detection position on the sensor having the greatest sensitivity to the acetone; or

    a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
one or more of:

    a ratio of the number of detection positions on the sensor that reach a saturated response level to the number of detection positions on the sensor that do not reach a saturated response level,

    a time required to reach a saturated response level for at least one of the detection positions on the sensor that reaches a saturated response level, and

    a response of the sensor to the acetone for at least one of the detection positions on the sensor that does not reach a saturated response level;

wherein the method comprises:

    (a) exposing the breath to a hydroxylamine, hydroxylamine salt, or combination thereof in the presence of a liquid, so as to produce a change in a variable, said variable being dependent upon the pH of the liquid;

    (b) measuring the change in the variable; and

    (c) determining the concentration of the acetone in the breath from the change in the variable.

In a specific embodiment, there is provided a method of determining a concentration of acetone in breath, the method comprising:

exposing the breath to a sensor which is configured to generate a differential response to a constant concentration of the acetone; and
measuring a differential response of the sensor to the acetone;

whereby the method further comprises a step of calculating the concentration of the acetone utilising the differential response of the sensor to the acetone;

wherein the differential response is spatially differential, whereby the sensor is configured to respond to the acetone at a plurality of detection positions on the sensor; said detection positions having different sensitivities to the acetone; and

wherein said calculating utilises at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

    a response of the sensor to the acetone at the detection position on the sensor having the greatest sensitivity to the acetone; or

    a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
  one or more of:

    a ratio of the number of detection positions on the sensor that reach a saturated response level to the number of detection positions on the sensor that do not reach a saturated response level,

    a time required to reach a saturated response level for at least one of the detection positions on the sensor that reaches a saturated response level, and

    a response of the sensor to the acetone for at least one of the detection positions on the sensor that does not reach a saturated response level;

wherein the method comprises:

    (a) exposing the breath to a hydroxylamine, hydroxylamine salt, or combination thereof in the presence of a liquid, so as to produce a change in a variable, said variable being dependent upon the pH of the liquid;

    (b) measuring the change in the variable; and

    (c) determining the concentration of the acetone in the breath from the change in the variable

wherein the sensor comprises a sorbent material; the hydroxylamine, hydroxylamine salt, or combination thereof is sorbed in and/or on the sorbent material; the sensor comprises a halochromic indicator which is sorbed in and/or on the sorbent material; the variable is an absorbance; and the detector is a colorimetric sensor.

In a specific embodiment, there is provided a method of determining a concentration of acetone in breath, the method comprising:

exposing the breath to a sensor which is configured to generate a differential response to a constant concentration of the acetone; and
measuring a differential response of the sensor to the acetone;

whereby the method further comprises a step of calculating the concentration of the acetone utilising the differential response of the sensor to the acetone;

wherein the differential response is spatially differential, whereby the sensor is configured to respond to the acetone at a plurality of detection positions on the sensor; said detection positions having different sensitivities to the acetone; and

wherein said calculating utilises at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

  a response of the sensor to the acetone at the detection position on the sensor having the greatest sensitivity to the acetone; or

  a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
  one or more of:

  a ratio of the number of detection positions on the sensor that reach a saturated response level to the number of detection positions on the sensor that do not reach a saturated response level,

  a time required to reach a saturated response level for at least one of the detection positions on the sensor that reaches a saturated response level, and

  a response of the sensor to the acetone for at least one of the detection positions on the sensor that does not reach a saturated response level;

wherein the method comprises:

  (a) exposing the breath to a hydroxylamine, hydroxylamine salt, or combination thereof in the presence of a liquid, so as to produce a change in a variable, said variable being dependent upon the pH of the liquid;

  (b) measuring the change in the variable; and

  (c) determining the concentration of the acetone in the breath from the change in the variable;

wherein the sensor comprises a sorbent material; the hydroxylamine, hydroxylamine salt, or combination thereof is sorbed in and/or on the sorbent material; the sensor comprises a halochromic indicator which is sorbed in and/or on the sorbent material; the variable is an absorbance, the detector is a colorimetric sensor; the liquid volume is about 1 ml or less; and the method does not comprise a titration.

In a specific embodiment, there is provided a method of determining a concentration of acetone in breath, the method comprising:

exposing the breath to a sensor which is configured to generate a differential response to a constant concentration of the acetone; and
measuring a differential response of the sensor to the acetone;

whereby the method further comprises a step of calculating the concentration of the acetone utilising the differential response of the sensor to the acetone;

wherein the differential response is spatially differential, whereby the sensor is configured to respond to the acetone at a plurality of detection positions on the sensor; said detection positions having different sensitivities to the acetone; and

wherein said calculating utilises at least the following:

- if the sensor reaches a saturated response level at all of the plurality of detection positions on the sensor:
  a response of the sensor to the acetone as a function of time at the detection position on the sensor having the least sensitivity to the acetone;

- if the sensor does not reach a saturated response level at any of the plurality of detection positions on the sensor:

  a response of the sensor to the acetone at the detection position on the sensor having the greatest sensitivity to the acetone; or

a response of the sensor to the acetone at one or more predetermined temporal points at a position on the sensor having the greatest sensitivity to the acetone; and

- if the sensor responds to the acetone to reach a saturated response level at some, but not all, of the plurality of detection positions on the sensor:
one or more of:

a ratio of the number of detection positions on the sensor that reach a saturated response level to the number of detection positions on the sensor that do not reach a saturated response level,

a time required to reach a saturated response level for at least one of the detection positions on the sensor that reaches a saturated response level, and

a response of the sensor to the acetone for at least one of the detection positions on the sensor that does not reach a saturated response level;

wherein the method comprises:

(a) exposing the breath to a hydroxylamine, hydroxylamine salt, or combination thereof in the presence of a liquid, so as to produce a change in a variable, said variable being dependent upon the pH of the liquid;

(b) measuring the change in the variable; and

(c) determining the concentration of the acetone in the breath from the change in the variable

wherein the sensor comprises a sorbent material; the hydroxylamine, hydroxylamine salt, or combination thereof is sorbed in and/or on the sorbent material; the variable is a hydronium ion concentration, and the change in hydronium ion concentration is measured using an ion sensitive filed effect transistor, chemiresistive sensor, or potentiometric sensor; the liquid volume is about 1 ml or less; and the method does not comprise a titration.

In a specific embodiment, the method is for managing diabetes in a patient on a diabetes treatment plan, wherein the fluid is breath, and the method comprises determining an acetone concentration of a breath sample of the patient by the method described earlier; comparing the acetone concentration to a reference acetone concentration range; and if the acetone concentration is outside the acetone concentration reference range, adjusting the diabetes treatment plan.

In another specific embodiment, the method is to assist in diagnosing diabetes in a patient, wherein the fluid is breath, and the method comprises determining an acetone concentration of a breath sample of the patient by the method described earlier; and comparing the acetone concentration to a reference acetone concentration range to determine if the patient has diabetes.

The method of the second aspect may use the system of the first aspect. The system of the first aspect may be used in the method of the second aspect.

According to a third aspect there is provided a sensor for sensing a concentration of acetone in a fluid, the sensor being as defined according to the first or second aspect.

In a specific embodiment there is provided a sensor configured to generate a spatially differential response to a constant concentration of acetone, whereby the sensor is configured to respond to acetone at a plurality of detection positions on the sensor, said detection positions having different sensitivities to the acetone; wherein the sensor comprises a hydroxylamine, hydroxylamine salt, or combination thereof; and the sensor comprises a sorbent material, wherein the hydroxylamine, hydroxylamine salt, or combination thereof is sorbed in and/or on the sorbent material.

In a specific embodiment there is provided a sensor configured to generate a spatially differential response to a constant concentration of acetone, whereby the sensor is configured to respond to acetone at a plurality of detection positions on the sensor, said detection positions having different sensitivities to the acetone; wherein the sensor comprises a hydroxylamine, hydroxylamine salt, or combination thereof; the sensor comprises a sorbent material, wherein the hydroxylamine, hydroxylamine salt, or combination thereof is sorbed in and/or on the sorbent material; and wherein the sensor comprises a halochromic indicator which is sorbed in and/or on the sorbent material.

The sensor of the third aspect may be used in the method of the second aspect. The method of the second aspect uses the sensor of the third aspect.

The system of the first aspect uses the sensor of the third aspect. The sensor of the third aspect is a component of the system of the first aspect.

According to a fourth aspect there is provided the use of a system according to the first aspect for diagnosing and/or managing diabetes in a patient, wherein the system is capable of determining a concentration of acetone in a breath sample of the patient, and the system comprises:

a hydroxylammonium salt, whereby exposure of the breath to the hydroxylammonium salt in the presence of a liquid produces a change in a variable, said variable being dependent upon the pH of the liquid;

a detector for measuring the change in the variable; and

a processor configured to calculate the concentration of the acetone in the breath from the change in the variable.

The use of the fourth aspect is a use of the system of the first aspect. The system of the first aspect may be used for the use of the fourth aspect. The use of the fourth aspect may use the method of the second aspect. The method of the second aspect may be used in the use of the fourth aspect.

According to a fifth aspect there is provided a method of managing diabetes in a patient on a diabetes treatment plan, said method comprising:

(a) determining an acetone concentration of a breath sample of the patient using the method according to the second aspect;

(b) comparing the acetone concentration to a reference acetone concentration range; and

(c) if the acetone concentration is outside the acetone concentration reference range, adjusting the diabetes treatment plan.

The method of the fifth aspect may use the system of the first aspect. The system of the first aspect may be used for the method of the fifth aspect. The method of the fifth aspect uses the sensor of the third aspect. The sensor of the third aspect may be used for the method of the fifth aspect.

According to a sixth aspect there is provided a method to assist in diagnosing diabetes in a patient, said method comprising:

(a) determining an acetone concentration of a breath sample of the patient using the method according to the second aspect; and

(b) comparing the acetone concentration to a reference acetone concentration range to determine if the patient has diabetes.

The method of the sixth aspect may use the system of the first aspect. The system of the first aspect may be used for the method of the sixth aspect. The method of the sixth aspect uses the sensor of the third aspect. The sensor of the third aspect may be used for the method of the sixth aspect.

According to a first embodiment there is provided a system for determining a concentration of acetone in a fluid, said system comprising a sensor configured to generate a differential response to a constant concentration of the acetone; and an acquisition device configured to determine the concentration of the acetone from the differential response.

The following options may be used in conjunction with the first embodiment, either individually or in any suitable combination.

The differential response may be temporally differential, whereby the response varies over time. The differential response is spatially differential, whereby the sensor is configured to respond to the acetone at a plurality of detection positions on the sensor; said detection positions having different sensitivities to the acetone.

The plurality of detection positions may be spaced equidistantly along the length of the sensor. Sensitivity to the acetone may vary along a length of the sensor. It may vary monotonically along the length of the sensor. It may vary linearly along the length of the sensor. Alternatively, it may vary nonlinearly, e.g. logarithmically, along the length of the sensor. It may vary linearly or logarithmically along the length of the sensor. The sensor may comprise a plurality of discrete regions each having a different sensitivity to the acetone. It may comprise a coating on a detection surface, said coating having spatially differential permeability to the acetone. It may comprise at least three detection positions having different sensitivities to the acetone.

The acquisition device comprises a processor configured to calculate the concentration of the acetone utilising the differential response of the sensor to the acetone.

In an embodiment the sensor is configured to respond to the acetone at a plurality of detection positions on the sensor; said detection positions having different sensitivities to the acetone. In this embodiment the acquisition device is configured to calculate the concentration of the acetone utilising at least the following:

if the sensor reaches a saturated response level at all of the plurality of detection positions on the sensor:
a response of the sensor to the acetone as a function of time at the detection position on the sensor having the least sensitivity to the acetone;

if the sensor does not reach a saturated response level at any of the plurality of detection positions on the sensor:

a response of the sensor to the acetone at the detection position on the sensor having the greatest sensitivity to the acetone; or

a response of the sensor to the acetone at one or more predetermined temporal points at a position on the sensor having the greatest sensitivity to the acetone; and

if the sensor responds to the acetone to reach a saturated response level at some, but not all, of the plurality of detection positions on the sensor:
one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level.

The system may further comprise a flow channel or an analysis chamber. The sensor may be disposed within said flow channel or analysis chamber. In some instances, the sensor surface may form a portion of a wall of the flow channel or analysis chamber. The system may further comprise a flow controller configured to control and/or monitor a flow rate of the fluid into said analysis chamber or through said flow channel. It may further comprise a volume controller configured to control and/or monitor the volume of the fluid passing into said analysis chamber or through said flow channel.

The analyte is acetone (which is a biomarker for diabetes).

In a specific embodiment there is provided a system for determining a concentration of an acetone in a fluid, said system comprising a sensor configured to generate a spatially differential response to a constant concentration of acetone, whereby the sensor is configured to respond to acetone at a plurality of detection positions on the sensor, said detection positions having different sensitivities to the acetone, wherein the sensor comprises a compound capable of reacting with acetone, said compound being selected from the group consisting of hydroxylamines, hydroxylamine salts, and combinations thereof; and an acquisition device configured to determine the concentration of the acetone from the differential response, the acquisition device configured to calculate the concentration of the acetone utilising at least the following:

if the sensor reaches a saturated response level at all of the plurality of detection positions on the sensor:
a response of the sensor to the acetone as a function of time at the detection position on the sensor having the least sensitivity to the acetone;

if the sensor does not reach a saturated response level at any of the plurality of detection positions on the sensor:

a response of the sensor to the acetone at the detection position on the sensor having the greatest sensitivity to the acetone; or

a response of the sensor to the acetone at one or more predetermined temporal points at a position on the sensor having the greatest sensitivity to the acetone; and

if the sensor responds to the acetone to reach a saturated response level at some, but not all, of the plurality of detection positions on the sensor:
one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level.

According to a second embodiment there is provided a method of determining a concentration of acetone in a fluid, the method comprising exposing the fluid to a sensor which is configured to generate a differential response to a constant concentration of acetone; and measuring a differential response of the sensor to the acetone.

The following options may be used in conjunction with the second embodiment, either individually or in any suitable combination.

The method may further comprise the step of calculating the concentration of the acetone utilising a differential response of the sensor to the acetone. The differential response is spatially differential, and the sensor is configured to respond to the acetone at a plurality of detection positions on the sensor; said detection positions having different sensitivities to the acetone. In this case, the calculating may utilise at least the following:

if the sensor reaches a saturated response level at all of the plurality of detection positions on the sensor:
a response of the sensor to the acetone as a function of time at a detection position on the sensor having the least sensitivity to the acetone;

if the sensor does not reach a saturated response level at any of the plurality of detection positions on the sensor:

a response of the sensor to the acetone at a detection position on the sensor having the greatest sensitivity to the acetone; or
a response of the sensor to the acetone at one or more predetermined temporal points at a detection position on the sensor having the greatest sensitivity to the acetone;

or

if the sensor responds to the acetone to reach a saturated response level at some, but not all, of the plurality of detection positions on the sensor:
one or more of a ratio of the number of detection positions on the sensor that reach a saturated response level to the number of detection positions on the sensor that do not reach a saturated response level, a time required to reach a saturated response level for at least one of the detection positions on the sensor that reaches a saturated response level, and a response of the sensor to the acetone for at least one of the detection positions on the sensor that does not reach a saturated response level.

The differential response may be temporally differential, whereby the response varies over time. In this instance the calculating may utilise one or both of: a time required to reach a predetermined response level for at least one position on the sensor after exposure of the fluid to the sensor; and a response level for at least one position on the sensor at a predetermined time after exposure of the fluid to the sensor.

The method of the second embodiment may use the system of the first embodiment. The system of the first embodiment may be used in the method of the second embodiment.

According to a third embodiment there is provided a sensor for sensing a concentration of acetone in a fluid, said sensor configured to generate a differential response to a constant concentration of the acetone.

The following options may be used in conjunction with the third embodiment, either individually or in any suitable combination.

The differential response may be temporally differential, whereby the response varies over time.

The differential response is spatially differential. Thus, the sensor is configured to respond to the acetone at a plurality of detection positions on the sensor; said detection positions having different sensitivities to the acetone. The plurality of detection positions may be spaced equidistantly along the length of the sensor. Sensitivity to the acetone may vary along a length of the sensor. It may vary monotonically along a length of the sensor. It may vary linearly along a length of the sensor. Alternatively, it may vary non-linearly, e.g. logarithmically, along a length of the sensor. It may vary linearly of logarithmically along a length of the sensor. The sensor may comprise a plurality of discrete regions each having a different sensitivity to the acetone. It may comprise a coating on a detection surface, said coating having spatially differential permeability to the acetone. It may comprise at least three detection positions having different sensitivities to the acetone.

In a specific embodiment there is provided a sensor configured to generate a spatially differential response to a constant concentration of acetone, whereby the sensor is configured to respond to acetone at a plurality of detection positions on the sensor, said detection positions having different sensitivities to the acetone; wherein the sensor comprises a compound capable of reacting with acetone, said compound being selected from the group consisting of hydroxylamines, hydroxylamine salts, and combinations thereof.

The sensor of the third embodiment may be used in the system of the first embodiment. The system of the first embodiment may incorporate the sensor of the third embodiment.

The sensor of the third embodiment may be used in the method of the second embodiment. The method of the second embodiment may use the sensor of the third embodiment.

According to a fourth embodiment there is provided a method of determining a concentration of acetone in a fluid, said method comprising:

(a) exposing the fluid to a hydroxylamine salt and an indicator so as to produce a change in absorption spectrum of the indicator, wherein the hydroxylamine salt and indicator are sorbed in and/or on a sorbent material;

(b) measuring the change in the absorption spectrum of the indicator; and

(c) determining the concentration of the acetone in the fluid from the change in the absorption spectrum of the indicator.

The following options may be used in conjunction with the fourth embodiment, either individually or in any suitable combination.

The hydroxylamine salt and indicator may be sorbed in and/or on a sorbent material. The hydroxylamine salt may be selected from the group consisting of hydroxylamine hydrochloride, hydroxylammonium sulfate, hydroxylammonium phosphate, hydroxylamine nitrate, and mixtures thereof.

The hydroxylamine salt and/or indicator may be in contact with a weak base. The weak base may be selected from the group consisting of bicarbonate salts, ammonia, aniline, phenolate salts, and mixtures thereof. The fluid may be a gas and/or vapour. It may be breath.

The change in the absorption spectrum of the indicator may be a change of absorption maximum of the indicator. In this case, the change in the absorption maximum of the indicator may comprise a change in wavelength. It may comprise a change in intensity. The change in the absorption spectrum may be substantially independent of the volume of the fluid exposed to the hydroxylamine salt and halochromic indicator. It may be substantially independent of the flow rate of the fluid exposed to the hydroxylamine salt and halochromic indicator.

The indicator may be a halochromic indicator. The absorption spectrum of the halochromic indicator may vary monotonically over an operational pH range. The halochromic indicator may be selected from the group consisting of methyl orange, methyl red, methyl yellow chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline and mixtures thereof. It may, for example, be selected from the group consisting of thymol blue, bromophenol blue, and mixtures thereof.

In a specific embodiment, the method is for managing diabetes in a patient on a diabetes treatment plan, the fluid is breath, and the method comprises determining an acetone concentration of a breath sample of the patient by the method described earlier; comparing the acetone concentration to a reference acetone concentration range; and if the acetone concentration is outside the acetone concentration reference range, adjusting the diabetes treatment plan.

In another specific embodiment, the method is for diagnosing diabetes in a patient, wherein the fluid is breath, and the method comprises determining an acetone concentration of a breath sample of the patient by the method described earlier; and comparing the acetone concentration to a reference acetone concentration range to determine if the patient has diabetes.

According to a fifth embodiment there is provided a method of managing diabetes in a patient on a diabetes treatment plan, said method comprising:

(a) determining an acetone concentration of a breath sample of the patient using the method of the fourth embodiment;

(b) comparing the acetone concentration to a reference acetone concentration range; and

(c) if the acetone concentration is outside the acetone concentration reference range, adjusting the diabetes treatment plan.

According to a sixth embodiment there is provided a method of diagnosing diabetes in a patient, said method comprising:

(a) determining an acetone concentration of a breath sample of the patient using the method of the fourth embodiment; and

(b) comparing the acetone concentration to a reference acetone concentration range to determine if the patient has diabetes.

According to a seventh embodiment there is provided a method for determining a concentration of acetone in a fluid, said method comprising:

(a) exposing the fluid to an hydroxylammonium salt or an in the presence of a liquid, so as to produce a change in a variable, said variable being dependent upon the pH of the liquid;

(b) measuring the change in the variable; and

(c) determining the concentration of the acetone in the fluid from the change in the variable.

The following options may be used in conjunction with the seventh embodiment, either individually or in any suitable combination.

The variable may be an absorbance, fluorescence, resistance, ion concentration, temperature, voltage, or current. In this case, the ion concentration may be a hydronium ion concentration.

The liquid volume may be about 1 mL or less.

In some instances, the hydroxylammonium salt may be sorbed in and/or on a sorbent material.

The change in the variable may be measured using an ion sensitive field effect transistor (ISFET), chemiresistive sensor, potentiometric sensor, spectroscopic sensor, colorimetric sensor, fluorometric sensor, thermal sensor, or a combination thereof. In particular embodiments, the change in the variable is not measured using a titration.

The method may further comprise controlling and/or monitoring the volume of the fluid which is exposed to the hydroxylammonium salt.

The hydroxylammonium salt may be selected from the group consisting of hydroxylammonium chloride, hydroxylammonium sulfate, hydroxylammonium azide, hydroxylamine picrate, hydroxylamine benzenesulfonate, hydroxylamine benzenesulfinate, hydroxylamine 1-sulfobutyl-3-methyl imidazole hydrosulfate salt, hydrazinium chloride, hydrazinium sulfate, hydrazinium hydrogensulfate, hydroxylammonium phosphate, hydroxylamine nitrate, and mixtures thereof.

The fluid may be a gas and/or vapour. It may, for example, be breath.

In a specific embodiment, the method is for managing diabetes in a patient on a diabetes treatment plan, wherein the fluid is breath, and the method comprises determining an acetone concentration of a breath sample of the patient by the method described earlier; comparing the acetone concentration to a reference acetone concentration range; and if the acetone concentration is outside the acetone concentration reference range, adjusting the diabetes treatment plan.

In another specific embodiment, the method is to assist in diagnosing diabetes in a patient, wherein the fluid is breath, and the method comprises determining an acetone concentration of a breath sample of the patient by the method described earlier; and comparing the acetone concentration to a reference acetone concentration range to determine if the patient has diabetes.

According to an eighth embodiment there is provided a method of managing diabetes in a patient on a diabetes treatment plan, said method comprising:

(a) determining an acetone concentration of a breath sample of the patient using the method according to the seventh embodiment;

(b) comparing the acetone concentration to a reference acetone concentration range; and

(c) if the acetone concentration is outside the acetone concentration reference range, adjusting the diabetes treatment plan.

According to a ninth embodiment there is provided a method to assist in diagnosing diabetes in a patient, said method comprising:

(a) determining an acetone concentration of a breath sample of the patient using the method according to the seventh embodiment; and

(b) comparing the acetone concentration to a reference acetone concentration range to determine if the patient has diabetes.

**Brief Description of Drawings**

**FIGURE 1:** Schematic diagrams of example sensors according to specific embodiments of the present invention. One sensor is depicted as having a continuous spatial arrangement of sensitivities to an analyte, and another is depicted as having a discrete spatial arrangement of sensitivities to an analyte.

**FIGURE 2:** Depicts an example sensor according to a specific embodiment of the present invention having an internal treatment that affects the local concentration of an analyte at different sensor positions.

**FIGURE 3:** Depicts an example sensor according to a specific embodiment of the present invention having an internal treatment that affects the local exposure of an analyte at different sensor positions.

**FIGURE 4:** Example sensor responses as a function of time at a position of the sensor after exposure of the sensor to the analyte. Figure 4(a) depicts a reversible non-saturated response, Figure 4(b) depicts a reversible saturated response, Figures 4(c) and 4(d) depict irreversible non-saturated responses, and Figure 4(e) depicts an irreversible saturated response.

**FIGURE 5(a):** An example sensor according to a specific embodiment of the present invention having increased sensitivity to an analyte from left to right along its length, displaying a sensor response divided into segment A, where the sensor response does not reach saturation; and segment B, where the sensor response reaches saturation.

**FIGURE 5(b):** A graph showing sensor response (signal) as a function of sensor sensitivity and distance across the sensor depicted in Figure 5(a).

**FIGURE 5(c):** A graph showing the time for the sensor response to reach saturation (100%) as a function of sensor sensitivity and distance across the sensor depicted in Figure 5(a).

**FIGURE 6:** Graphs showing sensor response (signal) as a function of sensor sensitivity and distance across the sensor depicted in Figure 5(a), and the time for the sensor response to reach saturation as a function of sensor sensitivity and distance across the sensor depicted in Figure 5(a). These graphs are for each of three cases of sensor response: where the sensor response does not reach saturation at any position (Case 1); where the sensor response reaches saturation at some, but not all positions (Case 2); and where the sensor response reaches saturation at all positions (Case 3).

**FIGURE 7:** A schematic depiction of a device according to a specific embodiment of the present invention. The device is a component of a system which analyses and stores data from the device. The device has a sorbent material strip on/in which a hydroxylammonium salt is sorbed.

**FIGURE 8:** A schematic depiction of a device according to a specific embodiment of the present invention. The device is a component of a system which analyses and stores data from the device. The device has a sorbent material strip on/in which a hydroxylammonium salt is sorbed, and also comprises an ISFET detector.

**FIGURE 9:** A schematic depiction of a device according to a specific embodiment of the present invention. The device is a component of a system which analyses and stores data from the device. The device has a sorbent material strip and a neutralisation chamber comprising a neutralisation agent which is capable of neutralising a compound sorbed on and/or in the sorbent material.

**FIGURE 10(a):** A diagram of a sorbent material component of a device according to a specific embodiment of the present invention.

**FIGURE 10(b):** A diagram of a sorbent material component of a device according to a specific embodiment of the present invention. The sorbent material has a plurality of exposure regions.

**FIGURE 11:** Depicts a sorbent material according to a specific embodiment of the present invention, the sorbent material having an internal treatment that affects the local concentration of a carbonyl containing compound at different sorbent material positions.

**FIGURE 12:** Depicts a sorbent material according to a specific embodiment of the present invention, the sorbent material having an internal treatment that affects the local exposure of a carbonyl containing compound at different sorbent material positions.

**FIGURE 13(a):** Depicts example sensor responses as a function of length across the sensor for a continuous spatially differential sensor such as depicted in Figure 5(a), at a high, medium and low analyte concentration.

**FIGURE 13(b):** Depicts example sensor responses as a function of exposure time for a sensor such as depicted in Figure 3, at a high, medium and low analyte concentration.

**FIGURE 14:** Depicts example sensor responses as a function of time exhibiting delays in their initial response.

**FIGURE 15(a):** A schematic depiction of a device according to a specific embodiment of the present invention having an ISFET detector system. The ISFET detector system comprises an ISFET and a traditional reference electrode.

**FIGURE 15(b):** A schematic depiction of a device according to a specific embodiment of the present invention having an ISFET detector system. The ISFET detector system comprises an ISFET and a reference field effect transistor (REFET).

**FIGURE 15(c):** A schematic depiction of a device according to a specific embodiment of the present invention having an ISFET detector system. The ISFET detector system comprises an ISFET and a disposable pseudo reference electrode.

**FIGURE 16(a):** A schematic depiction of a device according to a specific embodiment of the present invention having a chemiresistive detector system. The chemiresistive detector system utilises a direct resistivity measurement.

**FIGURE 16(b):** A schematic depiction of a device according to a specific embodiment of the present invention having a chemiresistive detector system. The chemiresistive detector system utilises an indirect resistivity measurement.

**FIGURE 16(c):** A schematic depiction of a device according to a specific embodiment of the present invention having a chemiresistive detector system. The chemiresistive detector comprises a Capacitively-Coupled Contactless Conductivity Detector ($C_4D$).

**FIGURE 17:** A device according to a specific embodiment of the present invention comprising a smartphone.

**FIGURE 18:** Example sensors according to a specific embodiment of the present invention, each with two discrete triangular shaped regions with higher (top right triangle) and lower (bottom left triangle) sensitivity to acetone, shown after exposure to: (a) 10 ppm acetone, and (b) 50 ppm acetone.

**FIGURE 19:** The sensor response plotted as a function of time for a position of an example sensor after exposure to 50 ppm acetone.

**FIGURE 20:** An exploded view of a device according to a specific embodiment of the present invention.

**FIGURE 21:** An exploded view of a device according to a specific embodiment of the present invention. The sorbent material has a plurality of exposure regions.

**FIGURE 22:** Depicts sensor responses (i.e. change in absorption spectrum of an indicator) at two different wavelengths for different acetone concentrations in gas samples using a method according to a specific embodiment of the present invention. The hydroxylamine salt and halochromic indicator are in solution.

**FIGURE 23:** Depicts sensor responses (i.e. change in absorption spectrum of an indicator) at two different wavelengths for different acetone concentrations in gas samples using a method according to a specific embodiment of the present invention. The hydroxylamine salt and halochromic indicator are sorbed in and/or on a sorbent material test strip.

**FIGURE 24:** Depicts sensor responses (i.e. change in absorption spectrum of an indicator) for different acetone concentrations in gas samples of two different gas volumes (0.5 L or 0.75 L) using a method according to a specific embodiment of the present invention.

**FIGURE 25:** Depicts sensor responses (i.e. change in absorption spectrum of an indicator) at two different wavelengths for different acetone concentrations in gas samples using a method according to a specific embodiment of the present invention. The method comprises exposing the gas samples to sodium bicarbonate.

**FIGURE 26:** The change in absorption spectrum of an indicator (i.e. response) plotted as a function of time for a position of an example sensor after exposure to 50 ppm acetone.

**FIGURE 27:** Depicts sensor response (i.e. change in voltage) or different acetone concentrations in gas samples using a method according to a specific embodiment of the present invention.

**Definitions**

As used herein, the term "about", is relative to the actual value stated, as will be appreciated by those of skill in the art, and allows for approximations, inaccuracies and limits of measurement under the relevant circumstances. Depending on context, it may allow a variation from the stated value of $\pm$ 10%, $\pm$ 5%, $\pm$ 2%, $\pm$ 1%, $\pm$ 0.5%, $\pm$ 0.2%, $\pm$ 0.1%, $\pm$ 0.05%, $\pm$ 0.02%, or $\pm$ 0.01%.

As used herein, the term "comprising" indicates the presence of the specified integer(s), but allows for the possibility of other integers, unspecified. This term does not imply any particular proportion of the specified integers. Variations of the word "comprising", such as "comprise" and "comprises", have correspondingly similar meanings.

As used herein, the term "small organic molecule" indicates an organic molecule having a molecular weight below about 900 Daltons.

As used herein, the term "accuracy" in reference to a sensor is a measure of how closely the analyte concentration quantified from the sensor response agrees with the "true" or expected analyte concentration. Accuracy may be expressed as an absolute error (absolute error = | obtained result-expected result | ). It may be expressed as a percentage relative error (percentage relative error = [( | obtained result-expected result | ) x 100]/expected result). For example, the term "accuracy" in reference to a device, system, sensor or method for determining the concentration of a carbonyl containing compound may be a measure of how closely the carbonyl containing compound concentration quantified using the device, system, sensor, or method respectively agrees with the "true" or expected carbonyl containing compound concentration. Accuracy may be expressed as an absolute error (absolute error = | obtained result-expected result | ). It may be expressed as a percentage relative error (percentage relative error = [( | obtained result-expected result | ) x 100]/expected result).

As used herein, the term "precision" in reference to the sensor is a measure of the reproducibility and repeatability of the sensor response at a given analyte concentration. This may be expressed as a percentage relative standard deviation. For example, the term "precision" in reference to a device, sensor, system or method for determining the concentration of a carbonyl-containing compound may be a measure of the reproducibility and repeatability of the change in the variable at a given carbonyl-containing compound concentration. This may be expressed as a percentage relative standard deviation. In certain embodiments the term "precision" in reference to a device, sensor, system, or method for determining the concentration of a carbonyl containing compound may be a measure of the reproducibility and repeatability of the change in the absorption spectrum of the indicator at a given carbonyl containing compound concentration. This may be expressed as a percentage relative standard deviation.

As used herein, the term "selectivity" in reference to the sensor for an analyte in a fluid, is a measure of the sensor's response to the analyte compared with the sensor's response to a non-analyte component of the fluid. For example, as used herein, the term "selectivity" in reference to a device, sensor, system, or method for determining the concentration of a carbonyl-containing compound, may be a measure of the change in the variable for a carbonyl-containing compound concentration in the fluid compared with another component of the fluid. In certain embodiments the term "selectivity" in reference to a device, system, sensor or method for determining the concentration of a carbonyl containing compound, may be a measure of the change in the absorption spectrum of the indicator for a carbonyl containing compound concentration in the fluid compared with another component of the fluid.

As used herein, the term "sensitivity" in reference to a position of the sensor is a measure of the sensor's response to a given concentration of an analyte at the position of the sensor. For example, a low sensitivity position of the sensor may produce a low response level or no response to a low concentration of an analyte, whereas a high sensitivity position of the sensor may produce a higher response level to the same analyte concentration.

As used herein, the term "dynamic range" in reference to the sensor is a measure of the concentration range of an analyte in which the sensor is able to sense or detect the analyte and produce a response that is distinguishable from any background response. For example, the term "dynamic range" in reference to a device, system, sensor or method for determining the concentration of a carbonyl-containing compound may be a measure of the concentration range of a carbonyl-containing compound in which the device, system, sensor or method is able to produce a change in the variable that is distinguishable from any background change in the variable. In certain embodiments the term "dynamic range" in reference to a device, system, sensor or method for determining the concentration of a carbonyl containing compound may be a measure of the concentration range of a carbonyl containing compound in which the device, system, sensor or method is able to produce a change in the absorption spectrum of the indicator that is distinguishable from any background change in the absorption spectrum of the indicator.

As used herein, the term "working range" in reference to the sensor is a measure of the concentration range of an analyte in which the analyte concentration can be quantified from the sensor response. For example, the term "working range" in reference to a device, system, sensor or method for determining the concentration of a carbonyl-containing compound may be a measure of the concentration range of a carbonyl-containing compound in which the carbonyl-containing compound concentration can be quantified from the change in the variable. For example, the term "working range" in reference to a device, system, sensor or method for determining the concentration of a carbonyl-containing compound may be a measure of the concentration range of a carbonyl-containing compound in which the carbonyl-containing compound concentration can be quantified from the change in the variable. In certain embodiments, the term "working range" in reference to the device, system, sensor, or method for determining the concentration of a carbonyl containing compound may be a measure of the concentration range of a carbonyl containing compound in which the carbonyl containing compound concentration can be quantified from the change in the absorption spectrum of the indicator.

As used herein, the term "differential response" in reference to the sensor means that the sensor can generate more than one response level when exposed to a constant concentration of the analyte. It may mean, for example, that the sensor can generate different responses at different times after exposure to a constant concentration of the analyte (i.e. temporally differential). It may mean that the sensor can generate different responses at different positions on the sensor after exposure to a constant concentration of the analyte (i.e. spatially differential). It may mean that the sensor can generate different responses at different positions on the sensor and at different times after exposure to a constant concentration of the analyte (i.e. both temporally and spatially differential). Thus, a differential response is a response which varies with a variation in one or more parameters other than analyte concentration, e.g. a position on the sensor, time from initial exposure to the analyte, or both of these.

As used herein, the term "detection position" or "detection positions" in reference to the sensor mean a position or positions of the sensor where the response may be measured.

As used herein, the term "configured" means to set up for operation, especially in a particular way.

As used herein, the term "exposure region" or "exposure regions" in reference to the device or system mean a region or regions of the sorbent material or sensor which is exposed to the fluid.

As used herein, the term "small organic molecule" indicates an organic molecule having a molecular weight below about 900 Daltons.

As used herein, the term "sorbent" means a material capable of adsorbing and/or absorbing a liquid, solid, and/or gaseous compound therein and/or thereon.

As used herein, the term "halochromic" means a material which changes colour when pH changes occur.

As used herein, the term "absorption maximum" means a maximum wavelength and/or intensity of electromagnetic absorption.

As used herein, the term "peak response level" means the highest response level at a position of the sensor reached upon exposure of the sensor to the analyte-containing fluid. For example, the term "peak response level" in relation to a device, system, sensor, or method for determining the concentration of a carbonyl-containing compound may mean the highest response level (i.e. greatest change in the variable) reached upon exposure of the hydroxylammonium salt to a concentration of the analyte (i.e. the carbonyl-containing compound) in a fluid. In certain embodiments, the term "peak response level" in relation to a device, system, sensor, or method for determining the concentration of a carbonyl-containing compound may mean the highest response level (i.e. greatest change in absorption spectrum of the indicator) reached at a position of the sorbent material upon exposure to a concentration of the analyte (i.e. the carbonyl containing compound) in a fluid.

As used herein, the term "steady state response level" means the response level upon exposure of a position of the sensor to a concentration of the analyte in a fluid, when the response has reached a steady state, or local equilibrium, i.e. no longer changes over time. For example, the term "steady state response level" in relation to a device, system, sensor, or method for determining the concentration of a carbonyl-containing compound may mean the response level (i.e. change in variable) upon exposure of the hydroxylammonium salt to a concentration of the analyte (i.e. the carbonyl-containing compound) in a fluid, when the response has reached a steady state, or local equilibrium, i.e. no longer changes over time. In certain embodiments, the term "steady state response level" in relation to a device, system, sensor, or method for determining the concentration of a carbonyl-containing compound may mean the response level (i.e. change in absorption spectrum of the indicator) reached at a position of the sorbent material upon exposure to a concentration of the analyte (i.e. the carbonyl containing compound) in a fluid, when the response has reached a steady state, or local equilibrium, i.e. no longer changes over time.

As used herein, the term "baseline response level" means the sensor response level at a position of the sensor prior to exposure of the sensor to a concentration of the analyte in a fluid. For example, the term "baseline response level" in relation to a device, system, sensor, or method for determining the concentration of a carbonyl-containing compound may mean the response level (i.e. change in variable) prior to exposure of the hydroxylammonium salt to a concentration of the analyte (i.e. the carbonyl-containing compound) in a fluid. In certain embodiments, the term "baseline response level" in relation to a device, system, sensor, or method for determining the concentration of a carbonyl-containing compound may mean the response level (i.e. change in absorption spectrum of the indicator) at a position of the sorbent material prior to exposure of the sorbent material to a concentration of the analyte (i.e. the carbonyl containing compound) in a fluid.

A "saturated response level" may mean that the sensor response level reaches about 100% of the maximum possible response level to the analyte at a position of the sensor, or it may mean, for example, that the sensor response level reaches from about 80% to about 100%, from about 85% to about 100%, from about 90% to about 100%, from about 95% to about 100%, from about 97% to about 100%, from about 98% to about 100%, from about 99% to about 100%, from about 99.5% to about 100%, from about 99.7% to about 100%, or from about 99.9% to about 100% of the maximum possible response level to the analyte at a position of the sensor. For example, the term "saturated response level" in relation to a device, system, sensor, or method for determining the concentration of a carbonyl-containing compound may mean that the response level (i.e. change in variable) reaches about 100%, from about 80% to about 100%, from about 85% to about 100%, from about 90% to about 100%, from about 95% to about 100%, from about 97% to about 100%, from about 98% to about 100%, from about 99% to about 100%, from about 99.5% to about 100%, from about 99.7% to about 100%, or from about 99.9% to about 100% of the maximum possible response level to the analyte (i.e. the carbonyl-containing compound) at a position of the sensor. In certain embodiments, the term "saturated response level" in relation to a device, system, sensor, or method for determining the concentration of a carbonyl-containing compound may mean that the response level (i.e. change in absorption spectrum of the indicator) reaches about 100%, from about 80% to about 100%, from about 85% to about 100%, from about 90% to about 100%, from about 95% to about 100%, from about 97% to about 100%, from about 98% to about 100%, from about 99% to about 100%, from about 99.5% to about 100%, from about 99.7% to about 100%, or from about 99.9% to about 100% of the maximum possible response level to the analyte (i.e. the carbonyl containing compound) at a position of the sorbent material.

As used herein, the term "non-saturated response level" means that the response level reaches less than the saturated response level to the analyte at a position of the sensor. For example, the term "non-saturated response level" in relation to a device, system, sensor, or method for determining the concentration of a carbonyl-containing compound may mean that the response level (i.e. change in variable) reaches less than the saturated response level to the analyte (i.e. the carbonyl-containing compound) at a position of the sorbent material. In certain embodiments, the term "non-saturated response level" in relation to a device, system, sensor, or method for determining the concentration of a carbonyl-containing compound may mean that the response level (i.e. change in absorption spectrum of the indicator) reaches less than the saturated response level to the analyte (i.e. the carbonyl containing compound) at a position of the sorbent material.

As used herein, the term "maximum possible response level" means the highest possible response level to the analyte at a position of the sensor. It is the response level to the analyte if a position of the sensor was exposed to pure undiluted analyte for sufficient time for the response to reach a steady state. For example, the term "maximum possible response level" in relation to a device, system, sensor, or method for determining the concentration of a carbonyl-containing compound may mean the highest possible response level (i.e. change in variable) to the analyte (i.e. the carbonyl-containing compound). It may be the response level to the analyte if the hydroxylammonium salt were exposed to pure undiluted analyte for sufficient time for the response to reach a steady state. In certain embodiments, the term "maximum possible response level" in relation to a device, system, sensor, or method for determining the concentration of a carbonyl-containing compound may mean the highest possible response level (i.e. change in absorption spectrum of the indicator) to the analyte (i.e. the carbonyl containing compound) at a position of the sorbent material. It may mean the response level to the analyte if the position of the sorbent material were exposed to pure undiluted analyte for sufficient time for the response to reach a steady state.

## Description of Embodiments

Disclosed herein is a system for determining a concentration of acetone in a fluid in accordance with the claims. The system comprises a sensor configured to generate a differential response to a constant concentration of the acetone, and an acquisition device configured to determine the concentration of the acetone from the differential response.

With respect to certain embodiments of the present invention the inventors have surprisingly discovered that a sorbent material having a hydroxylamine salt and an indicator sorbed therein and/or thereon in the presence of a fluid comprising acetone produces a change in the absorption spectrum of the indicator that is substantially dependent upon the concentration of the acetone in the fluid, but is substantially independent of the fluid volume exposed to the sorbent material. This enables the concentration of the acetone to be determined without the need for strict control or accurate measurement of the fluid volume.

## System

The system comprises the sensor and an acquisition device configured to measure a response of the sensor to the acetone. The system may be portable. It may be non-portable. It may be a detector. It may be a handheld system, for example a handheld detector. The sensor may be removably disposed within the system.

The system may further comprise a flow channel or an analysis chamber. The sensor may be disposed within the flow channel or analysis chamber. It may be located so as to be exposed to fluid in the flow channel or analysis chamber. It may, for example, form part of an inner surface or wall of the flow channel or analysis chamber. The system may further comprise a flow controller configured to control and/or monitor a flow rate of the fluid into the analysis chamber or through the flow channel. It may comprise a volume controller configured to control and/or monitor the volume of the fluid passing into the analysis chamber or through the flow channel.

## Sensor

The sensor is for sensing a concentration of acetone in a fluid. It may be a chemical sensor. It may be a biosensor. It may be a single-use sensor. It may be reusable. It may be suitable for sensing a concentration of acetone in a fluid, for example, a liquid, vapour, gas or mixture thereof. The sensor may comprise a strip, or it may be a part of a strip. The skilled person will understand that the sensor may be any suitable shape or size. It may be flat, or essentially two-dimensional, or it may be three-dimensional in shape. The sensor may comprise a sorbent material.

The sensor is configured to generate a differential response to a constant concentration of the acetone. The differential response is spatially differential, whereby the sensor is configured to respond to the acetone at a plurality of detection positions on the sensor, the detection positions having different sensitivities to the acetone. The differential response may be a combination of temporally differential and spatially differential. In other words, the response may be different at different positions, for example due to a difference in sensitivity at different positions of the sensor, and the response at a position may vary over time, after exposure of the sensor to the acetone.

The sensor responds to the acetone by producing a sensor response at a position on the sensor. This response may vary over time. It may vary in intensity or response level over time. For example, the response level at a position on the sensor may be at 0% of the maximum possible response level, or it may produce no response, or a baseline response level prior to exposure of the sensor to the acetone. Upon exposure of the sensor to the acetone the response level at a position of the sensor may increase over a period of time. The response level at a position of the sensor upon exposure to the acetone may increase to a peak response level, a steady state response level, or a saturated response level over a period of time. If the concentration of the acetone is below a threshold concentration for a position of the sensor, the response level at the position of the sensor may not increase above its baseline response level. The sensor response may be measured at a defined, or predetermined time after exposure, or commencement of exposure of the sensor to the acetone.

The sensor response is a change in a variable as defined in the claims. It may, for example, be a change in an absorbance, fluorescence, resistance, ion concentration, temperature, voltage, or current. The sensor response may be a change in colour, or a change in absorbance of electromagnetic radiation. The electromagnetic radiation may be, for example, infrared, far infrared, near infrared, visible, ultraviolet, or some other electromagnetic radiation. The sensor response may be a change in resistance. It may be a change in temperature. It may be a change in voltage. It may be a change in electric current. It may be production of electromagnetic radiation, for example x-ray, fluorescent, phosphorescent, or chemiluminescent radiation.

The sensor comprises a hydroxylammonium salt which selectively interacts or reacts with acetone. The hydroxylammonium salt may be adsorbed, absorbed, and/or bound in the sensor. It may be adsorbed, absorbed, or bound in and/or on an absorbent, adsorbent, and/or functionalised media in the sensor. The hydroxylammonium salt may react with or bind to the acetone. The interaction of the hydroxylammonium salt with the acetone produces a change, for example it may produce a colour change, a change in resistance, or a change in temperature. The sensor has spatially differential sensitivity to the acetone. The spatially differential sensitivity may result from a spatially differential local concentration or local amount of the hydroxylammonium salt at different points of the sensor. The sensitivity to the acetone may increase at a position of the sensor having increased local concentration or amount of the hydroxylammonium salt when compared to a position of the sensor having decreased local concentration or amount of the hydroxylammonium salt.

The spatially differential sensitivity results in a differential sensor response at different positions of the sensor when the sensor is exposed to a concentration of the acetone in a fluid.

The response of the sensor upon exposure to a concentration of the acetone in a fluid may vary from about 0% (no response) to a saturated response. It may vary from about 0% (no response) to a saturated response at different positions of the sensor. It may vary from about 0% (no response) to a saturated response at different times after exposure to the fluid. The sensor response to the acetone is spatially dependent. In other words, the sensor response may be dependent upon the position on the sensor. The sensor response to the acetone may additionally be time dependent. In other words, the sensor response may be dependent upon the time after exposure to the fluid. In some instances, the time dependence may vary at different positions on the sensor.

The spatially differential sensitivity to the acetone at any particular position of the sensor may be from about 0% to about 100% of the sensitivity of a sensor position having the greatest sensitivity to the acetone, or it may be from about 0.1% to about 100%, about 0.5% to about 100%, about 1% to about 100%, about 5% to about 100%, about 10% to about 100%, about 20% to about 100%, about 30% to about 100%, about 40% to about 100%, about 50% to about 100%, about 60% to about 100%, about 70% to about 100%, about 80% to about 100%, about 90% to about 100%, or about 95% to about 100% of the sensitivity of a sensor position having the greatest sensitivity to the acetone.

The sensor comprises a plurality of positions having different sensitivity to the acetone. The sensor may comprise at least two positions having different sensitivities to the acetone, or it may comprise at least 3, 4, 5, 6, 7, 8, 9, 10, 12, 14, 16, 18, 20, 30, 40, or 50, or more than 50 positions having different sensitivities to the acetone. It may comprise from about 2 to about 200 positions having different sensitivities to the acetone, or it may comprise from about 2 to about 100, about 2 to about 50, about 2 to about 20, about 3 to about 10, about 5 to about 50, or about 10 to about 50 positions having different sensitivities to the acetone.

The skilled person will understand that the detection positions of the sensor may be in any arrangement. They may, for example, be spaced equidistantly along a length of the sensor. They may be non-equidistantly spaced along a length of the sensor. They may be arranged in a regular pattern on the sensor. They may be arranged in a non-regular pattern on the sensor. They may be arranged on a single face of the sensor, or on more than one face of the sensor. They may be arranged in a linear or radial arrangement on the sensor. They may be arranged in an array. They may be arranged through, or within the sensor. They may be in an orthogonal arrangement to each other. They may be arranged, for example, to have a large region comprising a larger amount of positions with suitable sensitivity for detecting concentrations of the acetone within a specified concentration range, and smaller regions comprising a smaller amount of positions with suitable sensitivity for detecting concentrations of the acetone outside of the specified concentration range.

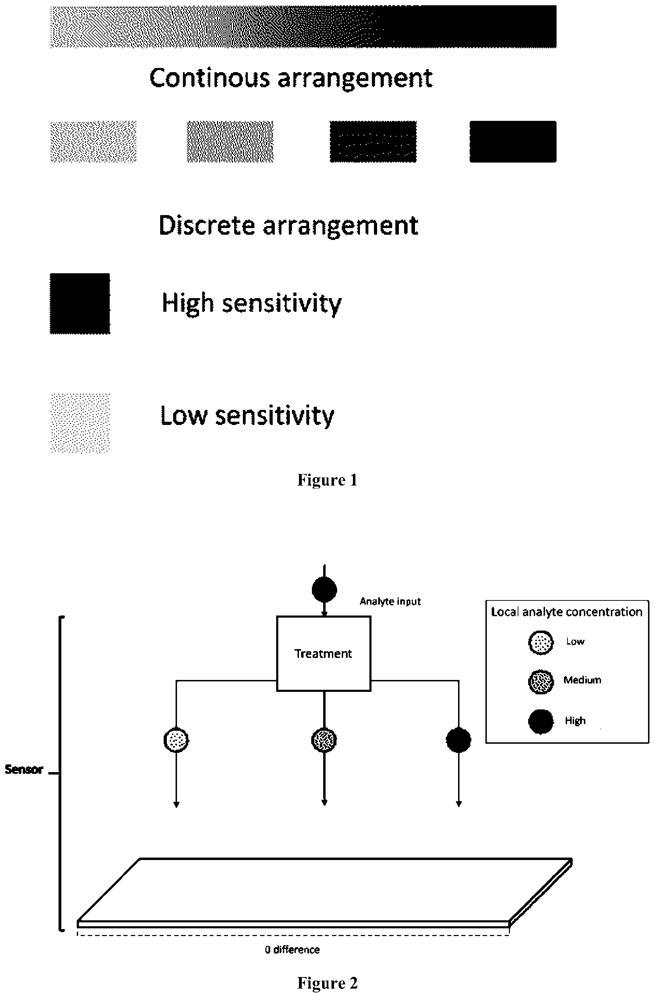

The sensitivity to the acetone may vary along a length of the sensor. It may vary monotonically along a length of the sensor. It may increase continuously along a length of the sensor. It may vary regularly along a length of the sensor. It may increase regularly along a length of the sensor. It may increase non-regularly along a length of the sensor. It may vary linearly or logarithmically along a length of the sensor. It may increase linearly or logarithmically along a length of the sensor. It may vary continuously along a length of the sensor. It may vary radially outward from a position of the sensor. It may increase radially outward from a position of the sensor. It may decrease radially outwards from a position of the sensor. It may vary in a pattern arrangement on the sensor. The difference in sensitivity across the sensor may be discrete or continuous as depicted in Figure 1, or it may be arranged in a different configuration. The skilled person will understand that the detection positions having different sensitivity to the acetone may be arranged in any manner. Their arrangement may depend, for example, on the kinetics of the interaction between the sensor and the acetone. It may depend, for example, on the kinetics of a reaction between the acetone and a compound in the sensor that reacts with the acetone to produce a sensor response at a position of the sensor.

In certain embodiments where the sensitivity to the acetone varies continuously along a length of the sensor, the sensor may comprise a sensitivity gradient (ds/dl), that is the rate of change of the sensitivity to the acetone (in response level per acetone concentration (in $mol.L^{-1}$), for example $\Delta\Omega.L.mol^{-1}$ in the case where the response is a change in resistance) as a function of the distance along the length of the sensor (in mm). The sensitivity gradient along the sensor may be adjusted to suit a variety of acetone concentration ranges. The sensitivity gradient may be greater than about 0, or it may be greater than about 0.01, 0.02, 0.05, 0.1, 0.2, 0.5, 1, 1.5, 2, 2.5, 5, 7.5, or 10. The sensitivity gradient may be constant along a length of the sensor, or it may vary along a length of the sensor.

The sensor comprises a plurality of discrete regions each having a different sensitivity to the acetone. They may be adjacent to each other, or they may be separated by areas having no sensitivity to the acetone. The discrete regions may be equally sized, or they may have different sizes. The sensor may comprise at least two discrete regions each having different sensitivity to the acetone, or it may comprise at least 3, 4, 5, 6, 7, 8, 9, or 10, or more than 10 discrete regions each having different sensitivity to the acetone. It may comprise from about 2 to about 50 discrete regions each having different sensitivity to the acetone, or it may comprise from about 2 to about 20, about 2 to about 10, about 5 to about 20, or about 10 to about 30 discrete regions each having different sensitivity to the acetone.

In certain embodiments, the sensor may comprise a coating on a detection surface. The coating has spatially differential permeability to the acetone so as to provide spatially differential sensitivity to the sensor. The coating may increase or decrease the concentration of the acetone concentration at the detecting surface when compared with the acetone concentration exposed to the sensor. The coating may have variable thickness, such that, for example, the sensor has higher sensitivity to the acetone at a position where the coating is thinner, and lower sensitivity to the acetone as a position where the coating is thicker. The coating may comprise apertures, e.g. pores, allowing a fraction of acetone to pass therethrough. It may comprise channels, for example microfluidic channels allowing a fraction of acetone to pass therethrough. The channels may, for example, have varying flow rates and/or permeability to the acetone. The spatially differential sensitivity to the acetone may result from the varying flow rate of the channels. For example, the sensor portion below the coating may have uniform sensitivity, but the varying flow rates of the channels of the coating may provide the sensor spatially differential sensitivity. The spatially differential sensitivity to the acetone may result from the spatially differential permeability of the coating. For example, the sensor portion below the coating may have uniform sensitivity, but the spatially differential permeability of the coating may provide the sensor spatially differential sensitivity. The spatially differential sensitivity to the acetone may result from a differential in time of exposure at a sensor portion below the coating because of the differential permeability of the coating. For example, the sensor portion below the coating may have uniform sensitivity, but the varying exposure time at a sensor portion below the coating as a result of the coating may provide the sensor spatially differential sensitivity. The sensitivity to the acetone may increase at a position of the sensor having increased permeability of the coating when compared to a position of the sensor having decreased permeability of the coating. The coating may comprise a blocking agent that interacts with the acetone. The blocking agent may react with, bind to, and/or adsorb the acetone.

The blocking agent may affect the dynamic range of the sensor by adjusting the local concentration of the acetone at a position of the sensor. The blocking agent may be used in a different concentration or amount at a plurality of positions of the sensor. The spatially differential sensitivity to the acetone may result from the different concentration or amount of the blocking agent at different positions of the sensor. For example, the sensor portion below the coating may have uniform sensitivity, but the differential concentration or amount of the blocking agent may provide the sensor spatially differential sensitivity as depicted in Figure 2. The sensitivity to the acetone may increase at a position of the sensor having decreased local concentration or amount of the blocking agent when compared to a position of the sensor having increased local concentration or amount of the blocking agent.

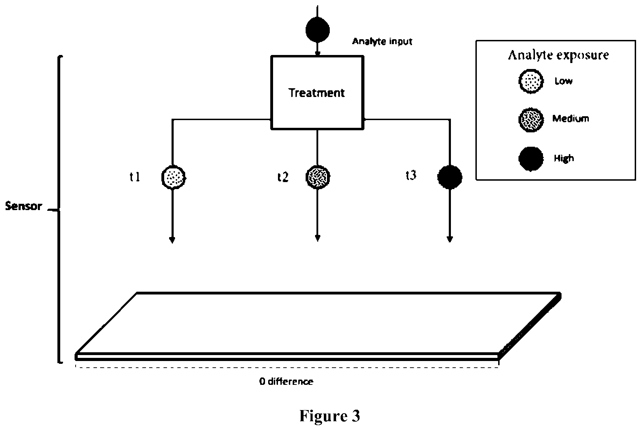

The sensor may comprise channels, for example microfluidic channels allowing a fraction of acetone to pass therethrough. The channels may, for example, have varying flow rates and/or permeability to the acetone. The fluid may pass through a microfluidic channel prior to contacting a detection position. The spatially differential sensitivity may result from the varying flow rate of the channels, providing a variable amount or volume of the fluid to the plurality of detection positions. For example, the detection positions of the sensor may have uniform sensitivity, but the varying flow rates of the channels of the coating may provide the sensor spatially differential sensitivity. The spatially differential sensitivity may result from varying exposure time at the plurality of detection positions. For example, the sensor positions may have uniform sensitivity, but the varying exposure time at different detection positions may provide the sensor spatially differential sensitivity. This example is depicted in Figure 3, where a concentration of the analyte is incident upon the sensor, where it is split into three regions which provide low (t1), medium (t2), and high (t3) exposure times at different detection positions.

The sensor may be disposed within a flow channel, or an analysis chamber. It may be disposed on or in a test strip. It may be a component of a detector. It may a component of a sensor array. It may be a component of a system. It may be a component of a device.

The response of the sensor upon exposure to a concentration of the acetone in a fluid may vary from about 0% (no response) to a saturated response. The response of the sensor upon exposure to a concentration of the acetone in a fluid may vary from about 0% (no response) to a saturated response at different positions of the sensor. The response of the sensor upon exposure to a concentration of the acetone in a fluid may change as a function of time at a position of the sensor. The response at a position of the sensor may initially be 0% prior to exposure to the acetone. The response may increase at the position of the sensor upon exposure to the acetone until a peak, or steady state response level is reached, which may be from about 0% (no response) to a saturated response. After all positions of the sensor have reached their peak, or steady state response level, there may be between about 0% and about 100% of positions of the sensor at a saturated response level, and between about 0% and about 100% of positions of the sensor at a non-saturated response level.

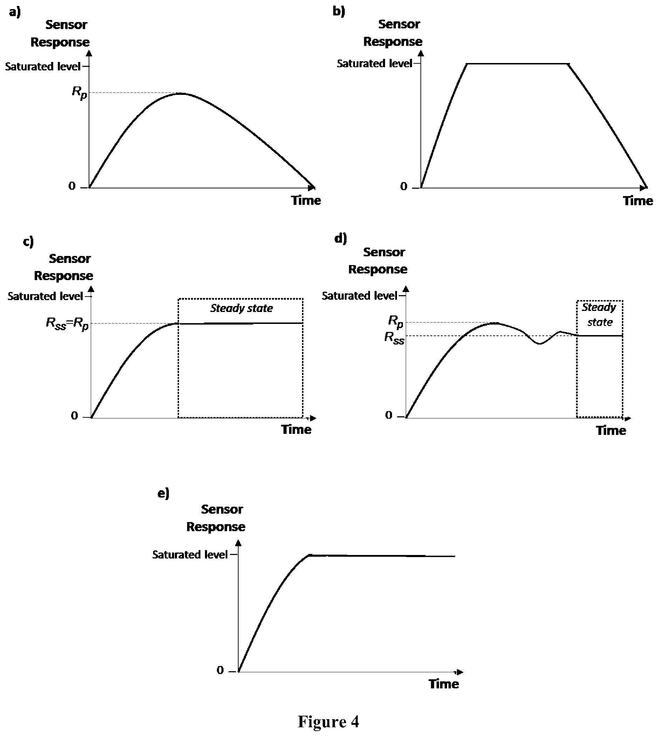

The response of the sensor upon exposure to a concentration of the acetone in a fluid may be reversible, that is it may return to zero over time after cessation of exposure to the acetone. In this case the sensor may be reusable. Figures 4(a) and 4(b) depict example reversible sensor responses as a function of time at a position of the sensor. Figure 4(a) depicts a non-saturated response with a peak response level $R_p$. Figure 4(b) depicts a saturated response.

Alternatively, the sensor response may be irreversible, that is it may not return to zero over time after exposure to the acetone. In this case the sensor may be non-reusable. Figures 4(c-e) depict example irreversible sensor responses as a function of time at a position of the sensor. Figures 4(c-d) depict a non-saturated response with a peak response level $R_p$ and a steady state response level $R_{ss}$. Figure 4(e) depicts a saturated response. In Figure 4(c) the sensor response increases over time after exposure to the analyte, until it reaches a steady state response level. In this case the peak response level and steady state response levels are equivalent. In Figure 4(d) the sensor response increases over time after exposure to the analyte, until it reaches a peak response level. The response level subsequently varies until it reaches a steady state response level as the sensor and analyte reach a local equilibrium.

The time for a position of the sensor to reach its steady state response level upon exposure to a concentration of the acetone in the fluid may be from about 0.1 seconds to about 120 minutes, or it may be from about 0.1 seconds to about 60 minutes, about 0.1 seconds to about 30 minutes, about 0.1 seconds to about 20 minutes, about 0.1 seconds to about 10 minutes, about 0.1 seconds to about 5 minutes, about 0.1 seconds to about 2 minutes, about 0.1 seconds to about 1 minute, about 0.1 seconds to about 30 seconds, about 1 second to about 10 minutes, about 30 seconds to about 60 minutes, about 1 minute to about 10 minutes, about 1 minute to about 60 minutes, about 5 minutes to about 60 minutes, about 10 minutes to about 60 minutes, about 5 minutes to about 30 minutes, or about 10 minutes to about 30 minutes. It may be less that about 60 minutes, or less than about 30, 20, 10, 5, 2, 1, or 0.5 minutes.

The time for a position of the sensor to reach its peak response level upon exposure to a concentration of the acetone in the fluid may be from about 0.01 seconds to about 120 minutes, or it may be from about 0.01 seconds to about 60 minutes, about 0.01 seconds to about 30 minutes, about 0.01 seconds to about 20 minutes, about 0.01 seconds to about 10 minutes, about 0.01 seconds to about 5 minutes, about 0.01 seconds to about 2 minutes, about 0.01 seconds to about 1 minute, about 0.01 seconds to about 30 seconds, about 1 second to about 10 minutes, about 30 seconds to about 60 minutes, about 1 minute to about 60 minutes, about 5 minutes to about 60 minutes, about 10 minutes to about 60 minutes, about 5 minutes to about 30 minutes, or about 10 minutes to about 30 minutes. It may be less that about 60 minutes, or less than about 30, 20, 10, 5, 2, 1, 0.5, or 0.1 minutes.

The sensor may be selective for the acetone. It may respond to the acetone but not substantially respond to any other component of the fluid at a position of the sensor. It may produce a higher response to the acetone when compared with its response to a non-acetone component of the fluid at the same concentration at a position of the sensor. It may produce a faster response to the acetone when compared with its response to a non-acetone component of the fluid at the same concentration at a position of the sensor. The ratio of the sensor response to the acetone, to the sensor response to any particular non-acetone component of the fluid at the same concentration at a position of the sensor may be from about 1.5 to about $1 \times 10^6$, or it may be from about 2 to about $1 \times 10^6$, about 5 to about $1 \times 10^6$, about 10 to about $1 \times 10^6$, about 50 to about $1 \times 10^6$, about 100 to about $1 \times 10^6$, or about $1 \times 10^3$ to about $1 \times 10^6$. It may be greater than about 1.5, 2, 5, 10, 20, 50, 100, 200, 500, 1000, or $1 \times 10^4$.

The sensor may be sensitive to the acetone. It may have a limit of detection (LOD) for the acetone of less than about 1000 ppm, or less than about 500 ppm, 200 ppm, 100 ppm, 50 ppm, 20 ppm, 10 ppm, 5 ppm, 2 ppm, 1 ppm, 500 ppb, 200 ppb, 100 ppb, 50 ppb, 10 ppb, 5 ppb, 2 ppb, or 1 ppb. It may have a LOD for the acetone of from about 0.1 ppb to about 1000 ppm, or from about 5 ppb to about 500 ppm, about 10 ppb to about 500 ppm, about 10 ppb to about 1000 ppm, about 100 ppb to about 500 ppm, about 500 ppb to about 500 ppm, or about 1 ppm to about 500 ppm. It may have a limit of quantitation (LOQ) for the acetone of less than about 1000 ppm, or less than about 500 ppm, 200 ppm, 100 ppm, 50 ppm, 20 ppm, 10 ppm, 5 ppm, 2 ppm, 1 ppm, 500 ppb, 200 ppb, 100 ppb, 50 ppb, 10 ppb, 5 ppb, 2 ppb, or 1 ppb. It may have a LOQ for the acetone of from about 0.1 ppb to about 1000 ppm, or from about 5 ppb to about 500 ppm, about 10 ppb to about 500 ppm, about 100 ppb to about 500 ppm, about 500 ppb to about 500 ppm, or about 1 ppm to about 500 ppm.

The dynamic range of the sensor may be from about 0.1 ppb to about 1000 g/L, or from about 5 ppb to about 1000 g/L, about 10 ppb to about 1000 g/L, about 10 ppb to about 1000 ppm, about 100 ppb to about 1000 g/L, about 500 ppb to about 1000 g/L, about 1 ppm to about 1000 g/L, about 10 ppm to about 1000 g/L, about 100 ppm to about 1000 g/L, or about 1000 ppm to about 1000 g/L.

The working range of the sensor may be from about 0.1 ppb to about 1000 g/L, or from about 5 ppb to about 1000 g/L, about 10 ppb to about 1000 g/L, about 100 ppb to about 1000 g/L, about 500 ppb to about 1000 g/L, about 1 ppm to about 1000 g/L, about 10 ppm to about 1000 g/L, about 100 ppm to about 1000 g/L, or about 1000 ppm to about 1000 g/L.

The accuracy of the sensor may be such that its percentage relative error for the acetone may be less than about 20%, or less than about 10%, 5%, 2%, 1%, 0.5%, 0.2%, 0.1%, 0.05%, 0.02%, or 0.01%.

The precision of the sensor may be such that its percentage relative standard deviation for an acetone concentration may be less than about 20%, or less than about 10%, 5%, 2%, 1%, 0.5%, 0.2%, 0.1%, 0.05%, 0.02%, or 0.01%.

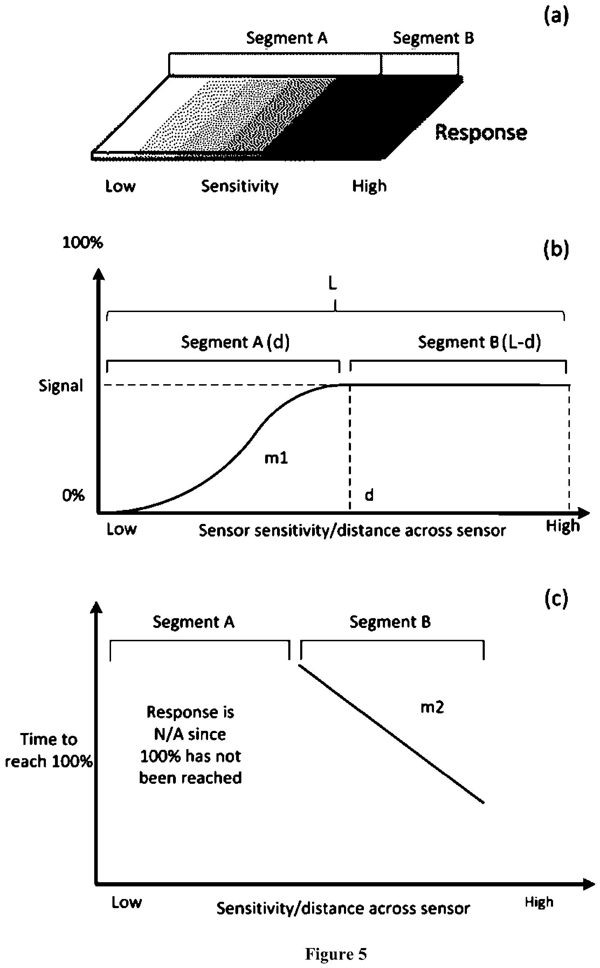

The sensor may be a component of a system for determining a concentration of acetone in a fluid. It may be a component of a device for determining a concentration of acetone in a fluid. When the sensor is exposed to a concentration of the acetone in the fluid, the response of the sensor may vary from about 0% (no response) to a saturated response at different detection positions of the sensor. After all detection positions of the sensor have reached their peak, or steady state response level, there may be between about 0% and about 100% of the positions of the sensor at a saturated response level, and between about 0% and about 100% of the positions of the sensor at a non-saturated response level. In the example spatially differential response sensor shown in Figure 5(a), Segment A depicts positions of the sensor at a non-saturated response level, and Segment B depicts positions of the sensor at a saturated response level.

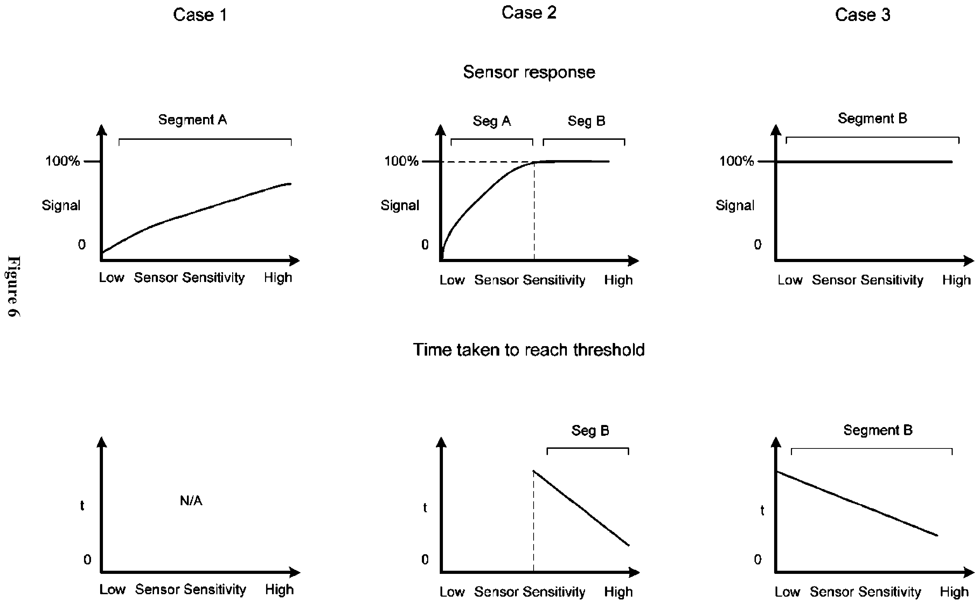

Figure 5(b) exhibits results that may be indicative of segments A and B as shown on the sensor after reaction of the sensing material with the analyte. For segment A and B, these are the gradient of response, and the time taken to reach 100% threshold respectively. As shown in Figures 5(b) and (c), the response of the sensor may provide three key data points: the slope of segment A (**m1**), the time response gradient of segment B (m2), and the length of segment A (d) or B (L-d) where L is the length of the sensor. High concentrations of the analyte may result in a steeper slope of **m1**, a shorter length of d, and a shallower slope of m2. In contrast, low concentrations of the analyte may result in a shallower slope of **m1,** a longer length of d and a steeper slope of m2. Depending on the concentration of analyte, the sensor response may be composed entirely of segment A (Case 1), B (Case 3), or a combination of both (Case 2). The three different cases of sensor response for an example spatially differential response sensor are depicted in Figure 6.

Device

The device may be the system as hereinbefore described. It may be a component of the system as hereinbefore described. The device may be a single-use device. It may be reusable. It may be portable. It may be non-portable. It may be a handheld device. It may be integrated within a portable health monitoring device. It may be suitable for determining a concentration of acetone in a fluid, for example, a liquid, vapour, gas or mixture thereof. The device may comprise a strip. It may be a lab-on chip. The skilled person will understand that the device may be any suitable shape or size. It may comprise an inlet for passing the fluid through. In the case where the fluid is breath, the device may comprise a mouthpiece for breathing through. The device may comprise a sorbent material removably disposed therein. It may comprise a sensor removably disposed therein. The device may be connected to a larger system that utilises analytical methods such as machine learning and neural networking to provide both historical and predictive analysis of a user's acetone levels. The device may be attachable, or connectable to a smartphone. It may comprise a smartphone. The device may be suitable for an unskilled operator to use. That is, it may be simple to use, such that a person without any formal training may be able to use the device by, for example, following a set of oral, written, and/or pictorial instructions.

The device may comprise a flow channel or an analysis chamber. The hydroxylammonium salt may be disposed within the flow channel or analysis chamber. It may be located so as to be exposed to fluid in the flow channel or analysis chamber. It may, for example, form part of an inner surface or wall of the flow channel or analysis chamber. The device may further comprise a flow controller configured to control and/or monitor a flow rate of the fluid into the analysis chamber or through the flow channel. It may comprise a flow controller configured to control and/or monitor the volume of the fluid which is exposed to the hydroxylammonium salt. It may comprise a volume controller configured to control and/or monitor the volume of the fluid passing into the analysis chamber or through the flow channel. In some instances, the device does not comprise a flow channel or analysis chamber. The device may comprise a flow monitor system, comprising a regulator valve, flow meter, timer, or any combination of the three. In this instance the regulator valve may be capable of limiting the minimum and/or maximum flow rate of the fluid when the device is in use. The timer may be used to monitor or control the length of time that the fluid is exposed to the hydroxylammonium salt. The flow meter may be able to measure the flow rate of the fluid during use of the device. The flow meter system may be, for example, an ultrasonic flow meter, turbine flow meter, or reflective light flowmeter. Alternatively, an intermediate storage device such as an air bag may be utilised to contain a fixed volume of the fluid. In some instances, the device does not comprise a flow controller and/or a volume controller.

The change in the variable (i.e. the response) upon exposure of the fluid to the hydroxylammonium salt may vary from about 0% (no response) to a saturated response. In the case where the device comprises a sorbent material, the change in the variable upon exposure of the fluid to the hydroxylammonium salt may vary from about 0% (no response) to a saturated response at different positions of the sorbent material. The change in the variable upon exposure to a concentration of the acetone in a fluid may change as a function of time at a position of the sorbent material. The response at a position of the sorbent material may initially be 0% prior to exposure to the acetone. The response may increase at the position of the sorbent material upon exposure to the acetone until a peak, or steady state response level is reached, which may be from about 0% (no response) to a saturated response. After all positions of the sorbent material have reached their peak, or steady state response level, there may be between about 0% and about 100% of positions of the sorbent material at a saturated response level, and between about 0% and about 100% of positions of the sorbent material at a non-saturated response level.

In the case where the device comprises a sorbent material, the time for a position of the sorbent material to reach its steady state response level upon exposure to a concentration of the acetone in the fluid may be from about 0.1 seconds to about 120 minutes, or it may be from about 0.1 seconds to about 60 minutes, about 0.1 seconds to about 30 minutes, about 0.1 seconds to about 20 minutes, about 0.1 seconds to about 10 minutes, about 0.1 seconds to about 5 minutes, about 0.1 seconds to about 2 minutes, about 0.1 seconds to about 1 minute, about 0.1 seconds to about 30 seconds, about 1 second to about 10 minutes, about 30 seconds to about 60 minutes, about 1 minute to about 10 minutes, about 1 minute to about 60 minutes, about 5 minutes to about 60 minutes, about 10 minutes to about 60 minutes, about 5 minutes to about 30 minutes, or about 10 minutes to about 30 minutes. It may be less that about 60 minutes, or less than about 30, 20, 10, 5, 2, 1, or 0.5 minutes.

In the case where the device comprises a sorbent material, the time for a position of the sorbent material to reach its peak response level upon exposure to a concentration of the acetone in the fluid may be from about 0.01 seconds to about 120 minutes, or it may be from about 0.01 seconds to about 60 minutes, about 0.01 seconds to about 30 minutes, about 0.01 seconds to about 20 minutes, about 0.01 seconds to about 15 minutes, about 0.01 seconds to about 10 minutes, about 0.01 seconds to about 5 minutes, about 0.01 seconds to about 2 minutes, about 0.01 seconds to about 1 minute, about 0.01 seconds to about 30 seconds, about 1 second to about 10 minutes, about 1 second to about 15 minutes, about 30 seconds to about 15 minutes, about 1 minute to about 15 minutes, about 5 minutes to about 15 minutes, about 10 minutes to about 15 minutes, about 5 minutes to about 30 minutes, or about 10 minutes to about 30 minutes. It may be less that about 60 minutes, or less than about 30, 20, 15, 10, 5, 2, 1, 0.5, or 0.1 minutes.

The device may be selective for the acetone. That is, it may respond to the acetone (i.e. produce a change in the variable), but not substantially respond (i.e. may not produce a substantial change in the variable) to any other component of the fluid. It may respond to the acetone (i.e. may produce a change in the variable), but not substantially respond (i.e. may not produce a substantial change in the variable) to any other non-carbonyl component of the fluid. It may produce a higher response to the acetone when compared with its response to any other component of the fluid at the same concentration. It may produce a higher response to the acetone when compared with its response to any other non-carbonyl component of the fluid at the same concentration. It may produce a faster response to the acetone when compared with its response to any other component of the fluid. It may produce a faster response to the acetone when compared with its response to any other non-carbonyl component of the fluid. The ratio of the device response to the acetone, to the device response to any other component of the fluid at the same concentration, optionally to any other non-carbonyl component of the fluid at the same concentration, may be from about 1.5 to about $1 \times 10^6$, or it may be from about 2 to about $1 \times 10^6$, about 5 to about $1 \times 10^6$, about 10 to about $1 \times 10^6$, about 50 to about $1 \times 10^6$, about 100 to about $1 \times 10^6$, or about $1 \times 10^3$ to about $1 \times 10^6$. It may be greater than about 1.5, 2, 5, 10, 20, 50, 100, 200, 500, 1000, or $1 \times 10^4$.

The device may be sensitive to the acetone. It may have a limit of detection (LOD) for the acetone of less than about 1000 ppm (v/v), or less than about 500 ppm (v/v), 200 ppm (v/v), 100 ppm (v/v), 50 ppm (v/v), 20 ppm (v/v), 10 ppm (v/v), 5 ppm (v/v), 2 ppm (v/v), 1 ppm (v/v), 500 ppb (v/v), 200 ppb (v/v), 100 ppb (v/v), 50 ppb (v/v), 10 ppb (v/v), 5 ppb (v/v), 2 ppb (v/v), or 1 ppb (v/v). It may have a LOD for the acetone of from about 0.1 ppb (v/v) to about 1000 ppm (v/v), or from about 5 ppb (v/v) to about 500 ppm (v/v), about 10 ppb (v/v) to about 500 ppm (v/v), about 10 ppb (v/v) to about 1000 ppm (v/v), about 100 ppb (v/v) to about 500 ppm (v/v), about 500 ppb (v/v) to about 500 ppm (v/v), or about 1 ppm (v/v) to about 500 ppm (v/v). It may have a limit of quantitation (LOQ) for the acetone of less than about 1000 ppm (v/v), or less than about 500 ppm (v/v), 200 ppm (v/v), 100 ppm (v/v), 50 ppm (v/v), 20 ppm (v/v), 10 ppm (v/v), 5 ppm (v/v), 2 ppm (v/v), 1 ppm (v/v), 500 ppb (v/v), 200 ppb (v/v), 100 ppb (v/v), 50 ppb (v/v), 10 ppb (v/v), 5 ppb (v/v), 2 ppb (v/v), or 1 ppb (v/v). It may have a LOQ for the acetone of from about 0.1 ppb (v/v) to about 1000 ppm (v/v), or from about 5 ppb (v/v) to about 500 ppm (v/v), about 10 ppb (v/v) to about 500 ppm (v/v), about 100 ppb (v/v) to about 500 ppm (v/v), about 500 ppb (v/v) to about 500 ppm (v/v), or about 1 ppm (v/v) to about 500 ppm (v/v).

The dynamic range of the device may be from about 0.1 ppb (v/v) to about 100,000 ppm (v/v), or from about 5 ppb (v/v) to about 10,000 ppm (v/v), about 500 ppb (v/v) to about 100 ppm (v/v), about 100 ppb (v/v) to about 10,000 ppm (v/v), about 10 ppb (v/v) to about 10,000 ppm (v/v), about 10 ppb (v/v) to about 1000 ppm (v/v), about 100 ppb (v/v) to about 10,000 ppm (v/v), about 500 ppb (v/v) to about 10,000 ppm, about 1 ppm (v/v) to about 10,000 ppm (v/v), about 10 ppm (v/v) to about 10,000 ppm (v/v), about 100 ppm (v/v) to about 10,000 ppm (v/v), or about 1000 ppm (v/v) to about 10,000 ppm (v/v).

The working range of the device may be from about 0.1 ppb (v/v) to about 100,000 ppm (v/v), or from about 5 ppb (v/v) to about 10,000 ppm (v/v), about 500 ppb (v/v) to about 100 ppm (v/v), about 100 ppb (v/v) to about 10,000 ppm (v/v), about 10 ppb (v/v) to about 10,000 ppm (v/v), about 100 ppb (v/v) to about 10,000 ppm (v/v), about 500 ppb (v/v) to about 10,000 ppm (v/v), about 1 ppm (v/v) to about 10,000 ppm (v/v), about 10 ppm (v/v) to about 10,000 ppm (v/v), about 100 ppm (v/v) to about 10,000 ppm (v/v), or about 1000 ppm (v/v) to about 10,000 ppm (v/v).

The accuracy of the device may be such that its percentage relative error for a concentration of the acetone may be less than about 20%, or less than about 10%, 5%, 2%, 1%, 0.5%, 0.2%, 0.1%, 0.05%, 0.02%, or 0.01%.

The precision of the device may be such that its percentage relative standard deviation for a concentration of the acetone may be less than about 20%, or less than about 10%, 5%, 2%, 1%, 0.5%, 0.2%, 0.1%, 0.05%, 0.02%, or 0.01%.

The device may further comprise a neutralisation chamber, said neutralisation chamber comprising a neutralisation reagent capable of neutralising a compound, such as the hydroxylammonium salt, or a product of the reaction between the acetone and the hydroxylammonium salt. In this context, "neutralisation" indicates that the hydroxylammonium salt is converted to a less hazardous and/or more environmentally friendly product or products. The neutralisation reagent may comprise a biological and/or a chemical material. It may comprise a bacterium. It may, for example, comprise a bacterium selected from the *Nitrosomonas* genus. It may, for example, comprise *Nitrosomonas europaea.* It may comprise an enzyme. It may comprise an oxidant. It may comprise, for example, one or more compound selected from the group consisting of hydrogen peroxide, chlorite salts, chlorate salts, perchlorate salts, hypochlorite salts, and mixtures thereof. The neutralisation reagent may be able to react with the hydroxylammonium salt. It may be able, for example, to oxidise the hydroxylammonium salt. This may serve to convert the hydroxylammonium salt into a less hazardous and/or more environmentally friendly product or products, such as ammonia, water and dilute nitric acid.

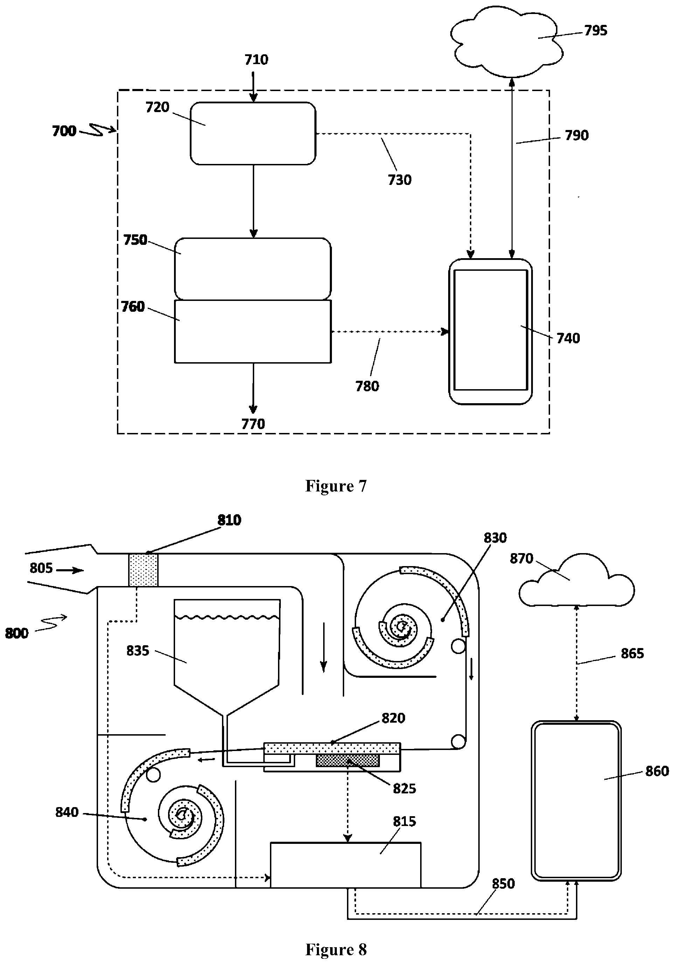

The device may be a component of a system. The system may be for personal acetone measurement and monitoring over a period. Figure 7 displays an example system. In this system, the device **700** comprises a flow monitor **720,** hydroxylammonium salt sorbed on a sorbent material **750,** a detector **760,** and a smart device (i.e. processor) **740.** The user breathes into the device, thereby supplying breath **710,** which passes through the flow monitor **720.** The sample flow rate and/or volume information for the sample are sent via path **730** from flow monitor **720** to smart device **740.** The breath sample is exposed to the hydroxylammonium salt sorbed on the sorbent material **750,** producing a change in a variable which is measured by the detector **760.** The change in the variable measurement is sent from detector **760** to smart device **740** via path **780.** The smart device calculates the concentration of the acetone from the sample flow rate and/or volume information and the change in the variable.

Further, in the example system depicted in Figure 7, the sensor data can be transmitted to the smart device such as a phone via wired or wireless connections at paths **730** and **780.** This allows various methods of local or cloud hosted computation and analytics to be performed to provide an accurate measurement of breath ketone bodies. Over time the device software can be upgraded which will enable more reliable results, and auxiliary data, such as activity and location data, obtained from the user's smartphone or paired devices such as a smart watch can be recorded at the time of ketone analysis. Data from the total user population can then be aggregated within a cloud service **795.** This data may be for used for machine learning, enabling predictions in user ketone body breath concentration trends based on regularly reported auxiliary data.

Figure 8 depicts another example system. In this system, the device **800** comprises disposable sorbent material **820** in a tape format. The tape is impregnated with the hydroxylammonium salt reagent in multiple discrete regions, and is coiled within a storage cartridge **830.** The device comprises a mouth piece for introducing a breath sample **805,** and a flow regulator **810** capable of measuring and/or regulating the flow of the breath sample. The liquid storage and delivery may be integrated into the device as shown at vessel **835** or may be supplied by external means (not shown). The device has a detector comprising an ISFET **825.** The ISFET **825** is capable of measuring a change in voltage when the carbonyl-containing compound reacts with the hydroxylammonium salt. The device also comprises a processor **815** configured to calculate the concentration of the carbonyl-containing compound in the fluid from the change in the voltage. The system comprises a connection component **850** which is capable of sending information from processor **815** to smartphone **860.**

Before testing, the user mechanically aligns a new section of the sorbent material **820** into the region above the ISFET **825** by moving a section of the sorbent material out of the storage cartridge **830.** Liquid is absorbed into the sorbent material through capillary channels connected to a reservoir **835,** that are positioned beneath the sorbent material **820** and adjacent to the ISFET **825.** Liquid absorption is limited by the saturation level of the sorbent material **820** and the liquid dissolves the hydroxylammonium salt reagent into a solution that is contained within the reaction.

The dried alkoxyamine/hydrazine reagents are impregnated within the absorbent reaction media that is segmented into multiple discreet regions and aligned in an array along a flexible tape. The tape stores numerous discreet regions that are suitable for a single use and is coiled within a storage container.

The user breathes through a mouthpiece and into the device. Within the mouthpiece inlet there is a flow regulator **810,** comprising a flow meter and timer, that is used to control, measure and provide flow information on the user's breath to the processor **815.** The breath contacts the wet sorbent material and imine formation takes place, leading to the associated formation of $H_3O^+$ ions and a reduction in pH.

The ISFET detector **825** measures the resultant change in voltage at the sorbent material and transmits this information to the processor **815.** The processor **815** uses the ISFET data and flow information to calculate the concentration of the user's breath ketone level. The device can be connected to a smart phone **860** as shown, either through a wired or wireless connection **850,** where the user's breath ketone level is displayed. Data from the total user population can then be aggregated within a cloud service **870** via wireless connection **865.**

The used sorbent material **820** portion is then moved away from the ISFET and into waste cartridge **840,** moving a new portion of the sorbent material **820** into contact with the ISFET **825** ready for the next breath sample analysis.

The device may further comprise a sorbent material, whereby the hydroxylammonium salt may be sorbed in and/or on the sorbent material.

In certain embodiments the device may be a single-use device. It may be reusable. It may be portable. It may be non-portable. It may be a handheld device. It may be integrated within a portable health monitoring device. It is suitable for determining a concentration of acetone in a fluid, for example, a liquid, vapour, gas or mixture thereof. The device may comprise a strip. It may be a lab-on chip. The skilled person will understand that the device may be any suitable shape or size. It may comprise an inlet for passing the fluid through. In the case where the fluid is breath, the device may comprise a mouthpiece for breathing through. The sorbent material may be removably disposed within the device. This device may be connected to a larger system that utilises analytical methods such as machine learning and neural networking to provide both historical and predictive analysis of a user's acetone levels. The device may be attachable, or connectable to a smartphone. It may comprise a smartphone.

The device may comprise a flow channel or an analysis chamber. The sorbent material may be disposed within the flow channel or analysis chamber. It may be located so as to be exposed to fluid in the flow channel or analysis chamber. It may, for example, form part of an inner surface or wall of the flow channel or analysis chamber. The device may further comprise a flow controller configured to control and/or monitor a flow rate of the fluid into the analysis chamber or through the flow channel. It may comprise a volume controller configured to control and/or monitor the volume of the fluid passing into the analysis chamber or through the flow channel. In some instances, the device does not comprise a flow channel or analysis chamber. In some instances, the device does not comprise a flow controller and/or a volume controller.

The device may comprise a plurality of electrodes configured to apply an electric field across a surface of the sorbent material so as to concentrate one or more charged species on and/or in the sorbent material. The electrodes may be in pairs, each electrode pair capable of applying a potential difference of from about 0.01 volts to about 20 volts, or about 0.01 volts to about 10 volts, about 0.01 volts to about 5 volts, about 0.01 volts to about 2 volts, about 0.01 volts to about 1 volt, about 0.01 volts to about 0.5 volts, about 0.01 volts to about 0.2 volts, about 0.01 volts to about 0.1 volts, about 0.1 volts to about 20 volts, about 0.1 volts to about 10 volts, about 1 volt to about 20 volts, about 1 volt to about 15 volts, about 1 volt to about 10 volts, or about 1 volt to about 5 volts. Each electrode pair may be capable of applying a potential difference of about 0.01, 0.02, 0.05, 0.1, 0.2, 0.5, 1, 2, 3, 4, 5, 6, 7, 8, 9, 10, 11, 12, 15 or 20 volts. When a potential difference is applied between a pair of electrodes, a positively charged species may migrate to, and be concentrated at, a position proximate to a negatively charged electrode, whereas a negatively charged species may migrate to, and be concentrated at, a position proximate to a positively charged electrode. This concentrating effect may be utilised to improve the sensitivity of the device by, for example, concentrating charged indicator species so that their local concentration is increased and produces a greater change in absorption spectrum at a position of increased local concentration.

The change in absorption spectrum of the indicator (i.e. the response) upon exposure of the fluid to the sorbent material may vary from about 0% (no response) to a saturated response. The change in absorption spectrum of the indicator upon exposure of the fluid to the sorbent material may vary from about 0% (no response) to a saturated response at different positions of the sorbent material. The change in absorption spectrum of the indicator upon exposure to a concentration of the acetone in a fluid may change as a function of time at a position of the sorbent material. The response at a position of the sorbent material may initially be 0% prior to exposure to the acetone. The response may increase at the position of the sorbent material upon exposure to the acetone until a peak, or steady state response level is reached, which may be from about 0% (no response) to a saturated response. After all positions of the sorbent material have reached their peak, or steady state response level, there may be between about 0% and about 100% of positions of the sorbent material at a saturated response level, and between about 0% and about 100% of positions of the sorbent material at a non-saturated response level.

The time for a position of the sorbent material to reach its steady state response level upon exposure to a concentration of the acetone in the fluid may be from about 0.1 seconds to about 120 minutes, or it may be from about 0.1 seconds to about 60 minutes, about 0.1 seconds to about 30 minutes, about 0.1 seconds to about 20 minutes, about 0.1 seconds to about 10 minutes, about 0.1 seconds to about 5 minutes, about 0.1 seconds to about 2 minutes, about 0.1 seconds to about 1 minute, about 0.1 seconds to about 30 seconds, about 1 second to about 10 minutes, about 30 seconds to about 60 minutes, about 1 minute to about 10 minutes, about 1 minute to about 60 minutes, about 5 minutes to about 60 minutes, about 10 minutes to about 60 minutes, about 5 minutes to about 30 minutes, or about 10 minutes to about 30 minutes. It may be less that about 60 minutes, or less than about 30, 20, 10, 5, 2, 1, or 0.5 minutes.

The time for a position of the sorbent material to reach its peak response level upon exposure to a concentration of the acetone in the fluid may be from about 0.01 seconds to about 120 minutes, or it may be from about 0.01 seconds to about 60 minutes, about 0.01 seconds to about 30 minutes, about 0.01 seconds to about 20 minutes, about 0.01 seconds to about 15 minutes, about 0.01 seconds to about 10 minutes, about 0.01 seconds to about 5 minutes, about 0.01 seconds to about 2 minutes, about 0.01 seconds to about 1 minute, about 0.01 seconds to about 30 seconds, about 1 second to about 10 minutes, about 1 second to about 15 minutes, about 30 seconds to about 15 minutes, about 1 minute to about 15 minutes, about 5 minutes to about 15 minutes, about 10 minutes to about 15 minutes, about 5 minutes to about 30 minutes, or about 10 minutes to about 30 minutes. It may be less that about 60 minutes, or less than about 30, 20, 15, 10, 5, 2, 1, 0.5, or 0.1 minutes.

The device may be selective for the acetone. That is, it may respond to the acetone (i.e. produce a change in absorption spectrum of the indicator), but not substantially respond (i.e. may not produce a substantial change in absorption spectrum of the indicator) to any other component of the fluid. It may respond to the acetone (i.e. may produce a change in absorption spectrum of the indicator), but not substantially respond (i.e. may not produce a substantial change in absorption spectrum of the indicator) to any other non-carbonyl component of the fluid. It may produce a higher response to the acetone when compared with its response to any other component of the fluid at the same concentration. It may produce a higher response to the acetone when compared with its response to any other non-carbonyl component of the fluid at the same concentration. It may produce a faster response to the acetone when compared with its response to any other component of the fluid. It may produce a faster response to the acetone when compared with its response to any other non-carbonyl component of the fluid. The ratio of the device response to the acetone, to the device response to any other component of the fluid at the same concentration, optionally to any other non-carbonyl component of the fluid at the same concentration, may be from about 1.5 to about $1 \times 10^6$, or it may be from about 2 to about $1 \times 10^6$, about 5 to about $1 \times 10^6$, about 10 to about $1 \times 10^6$, about 50 to about $1 \times 10^6$, about 100 to about $1 \times 10^6$, or about $1 \times 10^3$ to about $1 \times 10^6$. It may be greater than about 1.5, 2, 5, 10, 20, 50, 100, 200, 500, 1000, or $1 \times 10^4$.

The device may be sensitive to the acetone. It may have a limit of detection (LOD) for the acetone of less than about 1000 ppm (v/v), or less than about 500 ppm (v/v), 200 ppm (v/v), 100 ppm (v/v), 50 ppm (v/v), 20 ppm (v/v), 10 ppm (v/v), 5 ppm (v/v), 2 ppm (v/v), 1 ppm (v/v), 500 ppb (v/v), 200 ppb (v/v), 100 ppb (v/v), 50 ppb (v/v), 10 ppb (v/v), 5 ppb (v/v), 2 ppb (v/v), or 1 ppb (v/v). It may have a LOD for the acetone of from about 0.1 ppb (v/v) to about 1000 ppm (v/v), or from about 5 ppb (v/v) to about 500 ppm (v/v), about 10 ppb (v/v) to about 500 ppm (v/v), about 10 ppb (v/v) to about 1000 ppm (v/v), about 100 ppb (v/v) to about 500 ppm (v/v), about 500 ppb (v/v) to about 500 ppm (v/v), or about 1 ppm (v/v) to about 500 ppm (v/v). It may have a limit of quantitation (LOQ) for the acetone of less than about 1000 ppm (v/v), or less than about 500 ppm (v/v), 200 ppm (v/v), 100 ppm (v/v), 50 ppm (v/v), 20 ppm (v/v), 10 ppm (v/v), 5 ppm (v/v), 2 ppm (v/v), 1 ppm (v/v), 500 ppb (v/v), 200 ppb (v/v), 100 ppb (v/v), 50 ppb (v/v), 10 ppb (v/v), 5 ppb (v/v), 2 ppb (v/v), or 1 ppb (v/v). It may have a LOQ for the acetone of from about 0.1 ppb (v/v) to about 1000 ppm (v/v), or from about 5 ppb (v/v) to about 500 ppm (v/v), about 10 ppb (v/v) to about 500 ppm (v/v), about 100 ppb (v/v) to about 500 ppm (v/v), about 500 ppb (v/v) to about 500 ppm (v/v), or about 1 ppm (v/v) to about 500 ppm (v/v).

The dynamic range of the device may be from about 0.1 ppb (v/v) to about 100,000 ppm (v/v), or from about 5 ppb (v/v) to about 10,000 ppm (v/v), about 500 ppb (v/v) to about 100 ppm (v/v), about 100 ppb (v/v) to about 10,000 ppm (v/v), about 10 ppb (v/v) to about 10,000 ppm (v/v), about 10 ppb (v/v) to about 1000 ppm (v/v), about 100 ppb (v/v) to about 10,000 ppm (v/v), about 500 ppb (v/v) to about 10,000 ppm, about 1 ppm (v/v) to about 10,000 ppm (v/v), about 10 ppm (v/v) to about 10,000 ppm (v/v), about 100 ppm (v/v) to about 10,000 ppm (v/v), or about 1000 ppm (v/v) to about 10,000 ppm (v/v).

The working range of the device may be from about 0.1 ppb (v/v) to about 100,000 ppm (v/v), or from about 5 ppb (v/v) to about 10,000 ppm (v/v), about 500 ppb (v/v) to about 100 ppm (v/v), about 100 ppb (v/v) to about 10,000 ppm (v/v), about 10 ppb (v/v) to about 10,000 ppm (v/v), about 100 ppb (v/v) to about 10,000 ppm (v/v), about 500 ppb (v/v) to about 10,000 ppm (v/v), about 1 ppm (v/v) to about 10,000 ppm (v/v), about 10 ppm (v/v) to about 10,000 ppm (v/v), about 100 ppm (v/v) to about 10,000 ppm (v/v), or about 1000 ppm (v/v) to about 10,000 ppm (v/v).

The accuracy of the device may be such that its percentage relative error for a concentration of the acetone may be less than about 20%, or less than about 10%, 5%, 2%, 1%, 0.5%, 0.2%, 0.1%, 0.05%, 0.02%, or 0.01%.

The precision of the device may be such that its percentage relative standard deviation for a concentration of the acetone may be less than about 20%, or less than about 10%, 5%, 2%, 1%, 0.5%, 0.2%, 0.1%, 0.05%, 0.02%, or 0.01%.

The device may further comprise a detection region which is in fluid connection with the sorbent material and is optically coupled to the detector. The detection region may be distal to the sorbent material. In the case where the fluid comprises a liquid, or if a liquid is added to the fluid, the detection region may be configured to reduce meniscus formation at the edges of the gas-liquid interface within the detection region. This may reduce interference in the detector measurement of the change in absorption spectrum of the indicator.

The device may further comprise a neutralisation chamber, said neutralisation chamber comprising a neutralisation reagent capable of neutralising a compound sorbed on and/or in the sorbent material. In this context, "neutralisation" indicates that the hydroxylammonium salt is converted to a less hazardous and/or more environmentally friendly product or products. The neutralisation reagent may comprise a biological and/or a chemical material. It may comprise a bacterium. It may, for example, comprise a bacterium selected from the *Nitrosomonas* genus. It may, for example, comprise *Nitrosomonas europaea.* It may comprise an enzyme. It may comprise an oxidant. It may comprise, for example, one or more compound selected from the group consisting of hydrogen peroxide, chlorite salts, chlorate salts, perchlorate salts, hypochlorite salts, and mixtures thereof. The neutralisation reagent may be able to react with the hydroxylamine. It may be able, for example, to oxidise the hydroxylamine. This may serve to convert the hydroxylamine into a less hazardous and/or more environmentally friendly product or products, such as ammonia, water and dilute nitric acid.

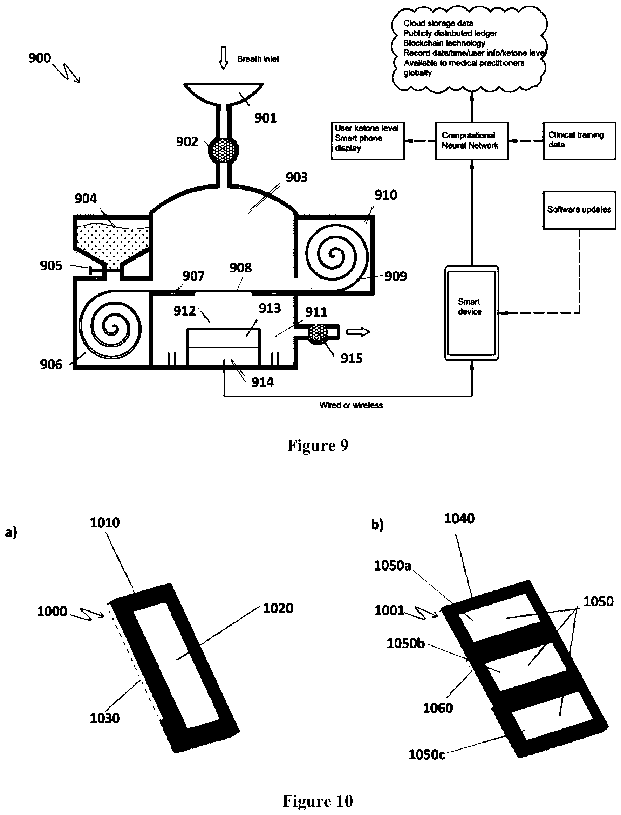

The device may be a component of a system. The system may be for personal acetone measurement and monitoring over a period. Figure 9 displays an example system. In this system, the device **900** comprises disposable sorbent material **909** in a tape format. The tape is impregnated with the halochromic indicator and hydroxylamine salt reagents, and is coiled within a storage cartridge **910.** The device comprises a mouth piece **901** for introducing a breath sample, and reaction chamber **903** where a breath sample can be exposed to the sorbent material. Solvent storage and delivery may be integrated into the device as shown at the pre-treatment chamber **902** or may be supplied by external means (not shown). The device has a detector comprising a spectrometric sensor **913** with a lens **912,** and a light source **911.** The sensor **913** is capable of measuring a change in absorption at sensing region **908**. The detector comprises a connection component **914** which is capable of sending information from detector to the smartphone. The device also comprises a waste collection cartridge **906,** where used sorbent material can be treated with neutralisation reagent **904** which can be dispensed by opening valve **905.** Any waste gas generated may be released through the gas outlet **915.**

Before testing, the user mechanically aligns a new section of the sorbent material **909** into the sensing region **908** above the detector by moving a section of the sorbent material out of the storage cartridge **910.** The light source **911** irradiates the sensing region with electromagnetic radiation, in this case visible light. Any light which is not absorbed by the indicator sorbed in and/or on the sorbent material is reflected by the sorbent material to produce an absorption spectrum which passes through the lens **912** where it is concentrated and measured at the sensor **913.** After the user breathes into the device through the mouth piece **901,** the fluid passes through the pretreatment area **902** and enters the reaction chamber **903** where it is exposed to the sorbent material at the sensing region **908.** The sensor **913** measures the absorption spectrum of the indicator and sends this information via the connection component **914** to a smart device (i.e. the processor). The smart device calculates the concentration of the acetone, which is recorded and analysed. In this example, the used sorbent material is passed into the waste collection cartridge **906,** where it is treated with neutralisation reagent **904** which can be dispensed by opening a valve **905.** Any waste gas generated may be released through the gas outlet **915.** This system may enable environmentally friendly storage, safe handling and disposal of multiple ketone sensing chips.

Further, in the example system depicted in Figure 9, the sensor data can be transmitted to the smart device such as a phone via wired or wireless connections. This allows various methods of local or cloud hosted computation and analytics to be performed to provide an accurate measurement of breath ketone bodies. Over time the device software can be upgraded which will enable more reliable results, and auxiliary data, such as activity and location data, obtained from the user's smartphone or paired devices such as a smart watch can be recorded at the time of ketone analysis. Data from the total user population can then be aggregated within a cloud service. This data may be for used for machine learning, enabling predictions in user ketone body breath concentration trends based on regularly reported auxiliary data.

**Sorbent material**

The sorbent material may be a component of the sensor as hereinbefore described. The sensor as hereinbefore described may comprise the sorbent material. The sorbent material may be transparent or at least partially transparent. It may be opaque. The sorbent material may be disposed within a flow channel, or an analysis chamber. It may be disposed on or in a test strip.

The sorbent material may be a surface. It may be a glass surface. It may be a polymer surface. It may be a surface-modified material, for example a surface modified glass. It may be a liquid vessel, sponge, hydrogel, paper, or an absorbent polymer. The sorbent material may comprise a polymer fibre. It may be a porous material, such as paper. It may comprise a wettable polymer. It may comprise cellulose, nitrocellulose, polypropylene or a combination thereof. It may comprise a hydrophilic region and/or a hydrophobic region. The hydrophilic region may comprise a wettable material. It may be a porous material. The hydrophobic region may comprise a hydrophobic polymer, a wax, or a combination thereof. The sorbent material may be disposed on an at least partially optically transparent substrate. It may be disposed on an optically opaque substrate. The sorbent material may be positioned in direct contact with the detector. In some instances, the sorbent material is not in direct contact with the detector.

The variable may be an absorbance, fluorescence, resistance, ion concentration, temperature, voltage, or current. In the case where the variable is an ion concentration, it may be a hydronium ion concentration. In particular embodiments, the change in the variable is not measured using a titration. In the case where the change in variable is not measured using a titration, the device may require only a small liquid volume, such as less than 1 mL. This may enable the device to have a simplified device design when compared with devices requiring a titration step, and/or may enable the device to be portable. In such a case, the device may be simple to use. That is, it may not require a highly skilled operator. It may, for example, be suitable for an unskilled operator to use.

In the case where the variable is an absorbance, it may be the absorbance of an indicator. In this instance, a change in the absorption spectrum of the indicator may comprise a change in wavelength. It may comprise a change in intensity. The change in the absorption spectrum may be substantially independent of the volume of the fluid exposed to the hydroxylammonium salt and halochromic indicator. It may be substantially independent of the flow rate of the fluid exposed to the hydroxylammonium salt and halochromic indicator.

The change in the absorption spectrum of the indicator may be a change of absorption maximum of the indicator. In this case, the change in the absorption maximum of the indicator may comprise a change in wavelength of the absorption maximum of the indicator. It may comprise a change in intensity of the absorption maximum of the indicator. It may comprise a change in wavelength and intensity. The change in the absorption spectrum of the indicator may comprise a change in intensity of the absorption of the indicator at a particular wavelength or at more than one wavelengths. It may be a change in colour, or a change in absorption e.g. absorbance of electromagnetic radiation. The electromagnetic radiation may be, for example, infrared, far infrared, near infrared, visible, ultraviolet, or some other electromagnetic radiation.

In the case where the indicator is a halochromic indicator, the absorption spectrum of the halochromic indicator may vary monotonically over an operational pH range. The halochromic indicator may be selected from the group consisting of methyl orange, methyl red, methyl yellow chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline, phenol blue, and mixtures thereof. It may, for example, be selected from the group consisting of thymol blue, bromophenol blue, and mixtures thereof.

In the case where the change in absorption spectrum of the indicator is a change in intensity at a particular wavelength or wavelengths, each particular wavelength or wavelengths may be within the range of from about 10 nm to about 300 $\mu$m, or from about 390 nm to about 700 nm, about 10 nm to about 400 nm, about 700 nm to about 300 $\mu$m, about 390 nm to about 500 nm, about 500 nm to about 600 nm, or about 600 nm to about 700 nm. It may be, for example, at about 10nm, 50 nm, 100 nm, 200 nm, 300 nm, 400 nm, 425 nm, 450 nm, 475 nm, 500 nm, 525 nm, 550 nm, 575 nm, 600 nm, 625 nm, 650 nm, 675 nm, 700 nm, 800 nm, 900 nm, 1 $\mu$m, 2 $\mu$m, 5 $\mu$m, 10 $\mu$m, 20 $\mu$m, 50 $\mu$m, 100 $\mu$m, 200 $\mu$m, or 300 $\mu$m.

In the case where the change in the absorption spectrum of the indicator is a change in wavelength of the absorption maximum of the indicator, the absorption maximum may mean the maximum absorption in a particular region of the electromagnetic spectrum. It may mean, for example, the maximum absorption in a region of from about 10 nm to about 300 $\mu$m, or from about 390 nm to about 700 nm, about 10 nm to about 400 nm, about 700 nm to about 300 $\mu$m, about 390 nm to about 500 nm, about 500 nm to about 600 nm, or about 600 nm to about 700 nm.

The indicator may be a material that changes due to the concentration of hydroxylammonium salts. It may be a halochromic indicator. In the case where the indicator is a halochromic indicator, it may be an indicator which changes absorption spectrum as a result of a change in pH. The indicator may comprise more than one such halochromic indicator. It may comprise a plurality of indicators, each indicator having a transition pH range, whereby the transition pH ranges of the plurality of indicators together span the operational pH range. It may, for example, be selected from the group consisting of methyl orange, methyl red, methyl yellow chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline, phenol blue, and mixtures thereof. It may, for example, be selected from the group consisting of thymol blue, bromophenol blue, and mixtures thereof. The absorption spectrum of the halochromic indicator may vary monotonically over an operational pH range. The term "operational pH range" may mean the pH range at a position of the sorbent material from the pH if the position was not exposed to any analyte, to the pH if the position was exposed to pure undiluted analyte for sufficient time for the pH to reach a steady state. The term "transition pH range" means the pH range in which small changes in pH produce a change in the indicator maximum absorption. The operational pH range may be from about 1 to about 9, about 1 to about 8, about 1 to about 7, about 1 to about 6, about 1 to about 5, about 2 to about 7, about 3 to about 7, about 4 to about 7, about 2 to about 5, or about 2 to about 4. The halochromic indicator may be selected to have an operational pH range that includes a pH range below the pH at a position of the sorbent material prior to exposure of the sorbent material to a concentration of the acetone in a fluid. It may be selected to have an operational pH range that includes a pH range below the pH of the fluid prior to exposure of the hydroxylammonium salt to a concentration of the acetone in a fluid. The halochromic indicator may change from a neutral species at a higher pH to a charged species at a lower pH.

In the case where the variable is a fluorescence, the device may comprise one or more fluorophores that are sensitive to one or more products from the reaction of a acetone with a hydroxylammonium salt. In this case, the detector may be, for example, a fluorometer.

In the case where the variable is a fluorescence, the change in fluorescence may be a change in intensity of fluorescence at a particular wavelength or wavelengths, each particular wavelength or wavelengths may be within the range of from about 10 nm to about 300 $\mu$m, or from about 390 nm to about 700 nm, about 10 nm to about 400 nm, about 700 nm to about 300 $\mu$m, about 390 nm to about 500 nm, about 500 nm to about 600 nm, or about 600 nm to about 700 nm. It may be, for example, at about 10nm, 50 nm, 100 nm, 200 nm, 300 nm, 400 nm, 425 nm, 450 nm, 475 nm, 500 nm, 525 nm, 550 nm, 575 nm, 600 nm, 625 nm, 650 nm, 675 nm, 700 nm, 800 nm, 900 nm, 1 $\mu$m, 2 $\mu$m, 5 $\mu$m, 10 $\mu$m, 20 $\mu$m, 50 $\mu$m, 100 $\mu$m, 200 $\mu$m, or 300 $\mu$m.

In the case where the variable is an ion concentration, it may be, for example, a hydronium concentration. It may be a hydroxylammonium concentration. It may be a concentration of the counter ion (i.e. anion) of a hydroxylammonium salt. It may be a concentration of a product ion from the reaction of a acetone and a hydroxylammonium salt.

In the case where the variable is an ion concentration, the change in ion concentration may be a change in ion concentration in the liquid. The change in ion concentration may be within the range of from about 0.001 mM to about 100 mM, or from about 0.01 mM to about 50 mM, about 0.01 mM to about 20 mM, about 0.01 mM to about 10 mM, about 0.01 mM to about 1 mM, about 0.01 mM to about 0.5 mM, or about 0.01 mM to about 0.1 mM.

In the case where the variable is a voltage and/or current, the detector may be, for example, a potentiostat, or an ISFET. In the case where the detector is a potentiostat, it may comprise three electrodes in contact with the liquid and/or sorbent material, where the voltage and/or current flow between a reference electrode and working electrode varies as a function of ion concentration (e.g. pH) of the liquid.

In the case where the variable is a voltage, the change in voltage may be a change in voltage between two electrodes at different positions of the liquid and/or sorbent material. The change in voltage may be within the range of from about 0.01 V to about 10 V, or from about 0.01 V to about 5 V, about 0.01 V to about 2 V, about 0.01 V to about 1 V, about 0.01 V to about 0.5 V, about 0.01 V to about 0.2 V, or about 0.01 V to about 0.1 V.

In the case where the variable is a current, the change in current may be a change in current between two electrodes at different positions of the liquid and/or sorbent material. The change in current may be within the range of from about 0.001 A to about 1 A, or from about 0.001 A to about 0.5 A, about 0.001 A to about 0.2 A, about 0.001 A to about 0.1 A, about 0.001 A to about 0.05 A, about 0.001 A to about 0.02 A, or about 0.001 A to about 0.01 A.

In the case where the variable is a resistance, the detector may be, for example, a chemiresistor sensor. In this case the liquid and/or sorbent material may be placed in direct contact with the chemiresistor sensor. The chemiresistor may comprise two opposing electrodes separated by the liquid and/or sorbent material, through which a current is passed at a stable voltage. The chemiresistor may measure the resistance across the liquid and/or sorbent material. A higher number of ions (e.g. hydronium ions) in the liquid and/or sorbent material may result in a lower resistance measurement.

In the case where the variable is a resistance, the change in resistance may be a change in resistance between two electrodes at different positions of the liquid and/or sorbent material. The change in resistance may be within the range of from about 0.1 Ω to about 10,000 Ω, or from about 0.1 Ω to about 5000 Ω, about 0.1 Ω to about 2000 Ω, about 0.1 Ω to about 1000 Ω, about 0.1 Ω to about 500 Ω, about 0.1 Ω to about 200 Ω, about 0.1 Ω to about 100 Ω, about 0.1 Ω to about 50 Ω, about 0.1 Ω to about 20 Ω, about 0.1 Ω to about 10 Ω, about 0.1 Ω to about 5 Ω, about 0. 1 Ω to about 2 Ω, or about 0.1 Ω to about 1 Ω.

In the case where the variable is a temperature, the detector may be, for example, a thermal sensor, such as a thermometer. In this case it may measure the temperature change caused by the enthalpy of reaction of a acetone with a hydroxylammonium salt.

In the case where the variable is a temperature, the change in temperature may be a change in temperature at a position of the liquid and/or sorbent material. The change in temperature may be within the range of from about 0.001 °C to about 10 °C, or from about 0.001 °C to about 5 °C, about 0.001 °C to about 2 °C, about 0.001 °C to about 1 °C, about 0.001 °C to about 0.5 °C, about 0.001 °C to about 0.2 °C, or about 0.001 °C to about 0.1 °C.

In certain embodiments the sorbent material has a hydroxylamine salt and an indicator sorbed therein and/or thereon, such that exposure of the fluid to the sorbent material produces a change in absorption spectrum of the indicator. The sorbent material may be transparent or at least partially transparent. It may be opaque. The sorbent material may be disposed within a flow channel, or an analysis chamber. It may be disposed on or in a test strip.

The sorbent material may be a surface. It may be a glass surface. It may be a polymer surface. It may be a surface-modified material, for example a surface modified glass. The sorbent material may comprise a polymer fibre. It may be a porous material, such as paper. It may comprise a wettable polymer. It may comprise cellulose, nitrocellulose, polypropylene or a combination thereof. It may comprise a hydrophilic region and/or a hydrophobic region. The hydrophilic region may comprise a wettable material. It may be a porous material. The hydrophobic region may comprise a hydrophobic polymer, a wax, or a combination thereof. The sorbent material may be disposed on an at least partially optically transparent substrate.

As shown in the example sorbent material depicted in Figure 10(a), sorbent material **1000** has a hydrophilic sensing region **1020,** bordered by a hydrophobic containment region **1010**. The sorbent material is disposed on an at least partially optically transparent substrate **1030**. In this case hydroxylamine hydrochloride and an indicator may be sorbed in and/or on the hydrophilic region. Upon exposure of the sorbent material to the fluid, the hydrophobic containment region restricts the fluid and/or any reagents from spreading. In this example, the at least partially transparent substrate enables spectroscopic measurement therethrough. The example sorbent material depicted in Figure 10(b) has three exposure areas, and is described elsewhere herein.

The change in the absorption spectrum of the indicator may be a change of absorption maximum of the indicator. In this case, the change in the absorption maximum of the indicator may comprise a change in wavelength of the absorption maximum of the indicator. It may comprise a change in intensity of the absorption maximum of the indicator. It may comprise a change in wavelength and intensity. The change in the absorption spectrum of the indicator may comprise a change in intensity of the absorption of the indicator at a particular wavelength or at more than one wavelengths. It may be a change in colour, or a change in absorption e.g. absorbance of electromagnetic radiation. The electromagnetic radiation may be, for example, infrared, far infrared, near infrared, visible, ultraviolet, or some other electromagnetic radiation.

In the case where the change in absorption spectrum of the indicator is a change in intensity at a particular wavelength or wavelengths, each particular wavelength or wavelengths may be within the range of from about 10 nm to about 300 μm, or from about 390 nm to about 700 nm, about 10 nm to about 400 nm, about 700 nm to about 300 μm, about 390 nm to about 500 nm, about 500 nm to about 600 nm, or about 600 nm to about 700 nm. It may be, for example, at about 10nm, 50 nm, 100 nm, 200 nm, 300 nm, 400 nm, 425 nm, 450 nm, 475 nm, 500 nm, 525 nm, 550 nm, 575 nm, 600 nm, 625 nm, 650 nm, 675 nm, 700 nm, 800 nm, 900 nm, 1 μm, 2 μm, 5 μm, 10 μm, 20 μm, 50 μm, 100 μm, 200 μm, or 300 μm.

In the case where the change in the absorption spectrum of the indicator is a change in wavelength of the absorption maximum of the indicator, the absorption maximum may mean the maximum absorption in a particular region of the electromagnetic spectrum. It may mean, for example, the maximum absorption in a region of from about 10 nm to about 300 μm, or from about 390 nm to about 700 nm, about 10 nm to about 400 nm, about 700 nm to about 300 μm, about 390 nm to about 500 nm, about 500 nm to about 600 nm, or about 600 nm to about 700 nm.

The hydroxylamine salt may be, for example, selected from the group consisting of hydroxylamine hydrochloride, hydroxylammonium sulfate, hydroxylammonium phosphate, hydroxylamine nitrate, and mixtures thereof. It may be hydroxylamine hydrochloride. The hydroxylamine salt may be used in a molar excess when compared with the indicator. It may be used in a molar excess when compared with the weak base.

The indicator may be a material that changes due to the concentration of hydroxylamine salts. It may be a halochromic indicator. In the case where the indicator is a halochromic indicator, it may be an indicator which changes absorption spectrum as a result of a change in pH. The indicator may comprise more than one such halochromic indicator. It may comprise a plurality of indicators, each indicator having a transition pH range, whereby the transition pH ranges of the plurality of indicators together span the operational pH range. It may, for example, be selected from the group consisting of methyl orange, methyl red, methyl yellow chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline, and mixtures thereof. It may, for example, be selected from the group consisting of thymol blue, bromophenol blue, and mixtures thereof. The absorption spectrum of the halochromic indicator may vary monotonically over an operational pH range. The term "operational pH range" may mean the pH range at a position of the sorbent material from the pH if the position was not exposed to any analyte, to the pH if the position was exposed to pure undiluted analyte for sufficient time for the pH to reach a steady state. The term "transition pH range" means the pH range in which small changes in pH produce a change in the indicator maximum absorption. The operational pH range may be from about 1 to about 9, about 1 to about 8, about 1 to about 7, about 1 to about 6, about 1 to about 5, about 2 to about 7, about 3 to about 7, about 4 to about 7, about 2 to about 5, or about 2 to about 4. The halochromic indicator may be selected to have an operational pH range that includes a pH range below the pH at a position of the sorbent material prior to exposure of the sorbent material to a concentration of the acetone in a fluid. The halochromic indicator may change from a neutral species at a higher pH to a charged species at a lower pH.

The sorbent material may comprise a plurality of exposure regions, said plurality of exposure regions having different sensitivities to the acetone. In this case, the sensitivity to the acetone at any particular exposure region of the sorbent material may be from about 0% to about 100% of the sensitivity of an exposure region having the greatest sensitivity to the acetone, or it may be from about 0.1% to about 100%, about 0.5% to about 100%, about 1% to about 100%, about 5% to about 100%, about 10% to about 100%, about 20% to about 100%, about 30% to about 100%, about 40% to about 100%, about 50% to about 100%, about 60% to about 100%, about 70% to about 100%, about 80% to about 100%, about 90% to about 100%, or about 95% to about 100% of the sensitivity of an exposure region having the greatest sensitivity to the acetone.

In the case where the sorbent material comprises a plurality of exposure regions, the sorbent material may comprise at least two exposure regions having different sensitivities to the acetone, or it may comprise at least 3, 4, 5, 6, 7, 8, 9, 10, 12, 14, 16, 18, 20, 30, 40, or 50, or more than 50 exposure regions having different sensitivities to the acetone. It may comprise from about 2 to about 200 exposure regions having different sensitivities to the acetone, or it may comprise from about 2 to about 100, about 2 to about 50, about 2 to about 20, about 3 to about 10, about 5 to about 50, or about 10 to about 50 exposure regions having different sensitivities to the acetone.

The skilled person will understand that the exposure regions of the sorbent material may be in any arrangement. They may, for example, be spaced equidistantly along a length of the sorbent material. They may be non-equidistantly spaced along a length of the sorbent material. They may be arranged in a regular pattern on the sorbent material. They may be arranged in a non-regular pattern on the sorbent material. They may be arranged on a single face of the sorbent material, or on more than one face of the sorbent material. They may be arranged in a linear or radial arrangement on the sorbent material. They may be arranged in an array. They may be arranged through, or within the sorbent material. They may be in an orthogonal arrangement to each other. They may be arranged, for example, to have a large exposure region comprising a larger amount of positions with suitable sensitivity for detecting concentrations of the acetone within a specified concentration range, and smaller regions comprising a smaller amount of positions with suitable sensitivity for detecting concentrations of the acetone outside of the specified concentration range.

Figure 10(b) shows an example sorbent material **1001** having three exposure regions **1050a, 1050b, 1050c** each having different sensitivity to the carbonyl containing compound. In this example each exposure region comprises a hydrophilic material and is surrounded by a hydrophobic containment border **1040** which separates the exposure regions and prevents the fluid to pass from one exposure region to another. In this example, the sorbent material is disposed on an at least partially transparent optical substrate **1060.**

In the case where the sorbent material comprises a plurality of exposure regions, said plurality of exposure regions having different sensitivities to the acetone, the different sensitivities may result from a spatially differential local concentration or local amount of the hydroxylamine salt and/or the indicator at different exposure regions of the sorbent material. In this case, the sensitivity to the acetone may increase at an exposure region of the sorbent material having increased local concentration or amount of the hydroxylamine salt and/or the indicator when compared to an exposure region of the sorbent material having decreased local concentration or amount of the hydroxylamine salt and/or the indicator.

In certain embodiments, the sorbent material may have an exposure surface for exposure to the fluid. The exposure surface may comprise a coating having spatially differential permeability to the acetone, so as to provide spatially differential sensitivity to the sorbent material. The coating may increase or decrease the concentration of the acetone concentration at the detecting surface when compared with the acetone concentration exposed to the sorbent material. The coating may have variable thickness, such that, for example, the sorbent material has higher sensitivity to the acetone at a position where the coating is thinner, and lower sensitivity to the acetone at a position where the coating is thicker. The coating may comprise apertures, e.g. pores, allowing a fraction of the acetone to pass therethrough. It may comprise channels, for example microfluidic channels allowing a fraction of the acetone to pass therethrough. The channels may, for example, have varying flow rates and/or permeability to the acetone. The spatially differential sensitivity may result from the varying flow rate of the channels. For example, the sorbent material portion below the coating may have uniform sensitivity, but the varying flow rates of the channels of the coating may provide the sorbent material spatially differential sensitivity. The spatially differential sensitivity to the acetone may result from the spatially differential permeability of the coating. For example, the sorbent material portion below the coating may have uniform sensitivity, but the spatially differential permeability of the coating may provide the sorbent material spatially differential sensitivity. In the case where the sorbent material has spatially differential sensitivity to the acetone, the spatially differential sensitivity may result from a differential in time of exposure at a sorbent material portion below the coating because of the differential permeability of the coating. For example, the sorbent material portion below the coating may have uniform sensitivity, but the varying exposure time at a sorbent material portion below the coating as a result of the coating may provide the sorbent material spatially differential sensitivity. The sensitivity to the acetone may increase at a position of the sorbent material having increased permeability of the coating when compared to a position of the sorbent material having decreased permeability of the coating. The coating may comprise a blocking agent that interacts with the acetone. The blocking agent may react with, bind to, and/or adsorb the acetone.

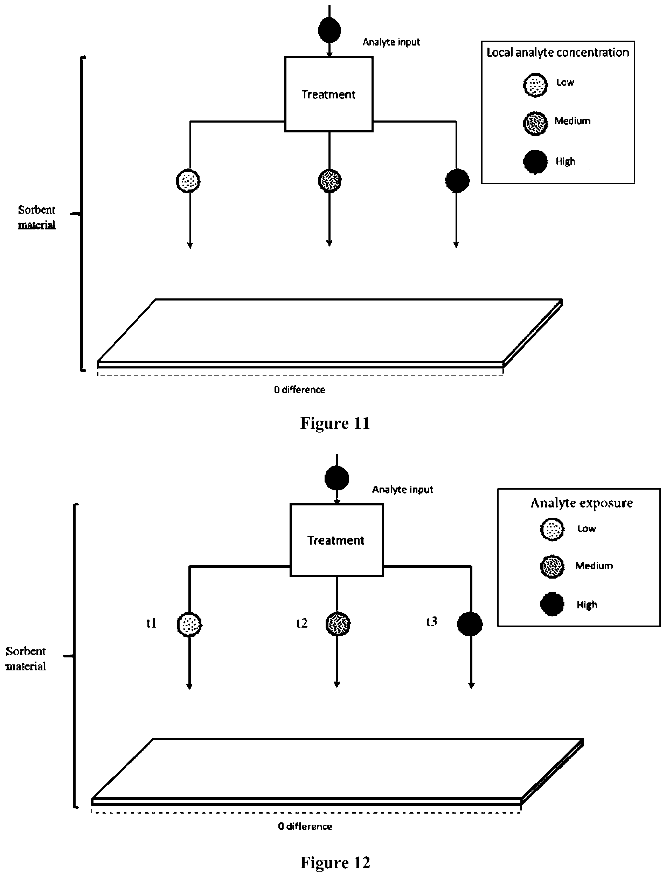

The blocking agent may affect the dynamic range of the device by adjusting the local concentration of the acetone at a position of the sorbent material. The blocking agent may be used in a different concentration or amount at a plurality of positions of the sorbent material. The spatially differential sensitivity to the acetone may result from the different concentration or amount of the blocking agent at different positions of the sorbent material. For example, the sorbent material portion below the coating may have uniform sensitivity, but the differential concentration or amount of the blocking agent may provide the sorbent material spatially differential sensitivity as depicted in Figure 11. In this example, a fluid incident upon the device is subjected to a differential treatment such that different positions of the sorbent material have different local analyte concentrations, shown as low, medium and high local concentrations in Figure 11. The sensitivity to the carbonyl containing compound may increase at a position of the sorbent material having decreased local concentration or amount of the blocking agent when compared to a position of the sorbent material having increased local concentration or amount of the blocking agent.

The sorbent material may comprise channels, for example microfluidic channels allowing a fraction of the acetone to pass therethrough. The channels may, for example, have varying flow rates and/or permeability to the acetone. The fluid may pass through a microfluidic channel prior to contacting an exposure region. The spatially differential sensitivity may result from varying exposure time at the plurality of exposure regions. For example, the exposure regions may have inherent uniform sensitivity, but the varying exposure time at different exposure regions may provide the sorbent material spatially differential sensitivity. This example is depicted in Figure 12, where a concentration of the carbonyl containing compound is incident upon the sorbent layer, where it is split into three sections which provide low (t1), medium (t2), and high (t3) exposure times at different exposure regions.

The sorbent material may further comprise a weak base sorbed thereon and/or therein. The weak base may be, for example, selected from the group consisting of bicarbonate salts, ammonia, aniline, phenolate salts, and mixtures thereof. It may be, for example, a bicarbonate salt. It may be sodium bicarbonate. The weak base may be used to adjust the working range of the device by increasing the pH at a surface of the sorbent material. The weak base may have a pKb of greater than about 3, or greater than about 4, 5, 6, 7, 8, 9, or 10. The weak base may have a pKb of from about 3 to about 12, or from about 3 to about 10, about 3 to about 8, about 5 to about 12, about 5 to about 10, about 6 to about 9, or about 7 to about 8. It may be, for example, about 3, 3.5, 4, 4.5, 5, 5.5, 6, 6.5, 7, 7.2, 7.4, 7.6, 7.8, 8, 8.2, 8.4, 8.6, 8.8, 9, 9.5, 10, 10.5, 11, 11.5, or 12.

**Hydroxylammonium salt**

The hydroxylammonium salt may be, for example, selected from the group consisting of hydroxylammonium chloride, hydroxylammonium sulfate, hydroxylammonium azide, hydroxylamine picrate, hydroxylamine benzenesulfonate, hydroxylamine benzenesulfinate, hydroxylamine 1-sulfobutyl-3-methyl imidazole hydrosulfate salt, hydroxylammonium phosphate, hydroxylamine nitrate, and mixtures thereof. It may be hydroxylamine hydrochloride.

In the case where the device comprises a sorbent material, the sorbent material may comprise a plurality of exposure regions, said plurality of exposure regions having different sensitivities to the acetone. In this case, the sensitivity to the acetone at any particular exposure region of the sorbent material may be from about 0% to about 100% of the sensitivity of an exposure region having the greatest sensitivity to the acetone, or it may be from about 0.1% to about 100%, about 0.5% to about 100%, about 1% to about 100%, about 5% to about 100%, about 10% to about 100%, about 20% to about 100%, about 30% to about 100%, about 40% to about 100%, about 50% to about 100%, about 60% to about 100%, about 70% to about 100%, about 80% to about 100%, about 90% to about 100%, or about 95% to about 100% of the sensitivity of an exposure region having the greatest sensitivity to the acetone.

In the case where the sorbent material comprises a plurality of exposure regions, the sorbent material may comprise at least two exposure regions having different sensitivities to the acetone, or it may comprise at least 3, 4, 5, 6, 7, 8, 9, 10, 12, 14, 16, 18, 20, 30, 40, or 50, or more than 50 exposure regions having different sensitivities to the acetone. It may comprise from about 2 to about 200 exposure regions having different sensitivities to the acetone, or it may comprise from about 2 to about 100, about 2 to about 50, about 2 to about 20, about 3 to about 10, about 5 to about 50, or about 10 to about 50 exposure regions having different sensitivities to the acetone.

The skilled person will understand that the exposure regions of the sorbent material may be in any arrangement. They may, for example, be spaced equidistantly along a length of the sorbent material. They may be non-equidistantly spaced along a length of the sorbent material. They may be arranged in a regular pattern on the sorbent material. They may be arranged in a non-regular pattern on the sorbent material. They may be arranged on a single face of the sorbent material, or on more than one face of the sorbent material. They may be arranged in a linear or radial arrangement on the sorbent material. They may be arranged in an array. They may be arranged through, or within the sorbent material. They may be in an orthogonal arrangement to each other. They may be arranged, for example, to have a large exposure region comprising a larger amount of positions with suitable sensitivity for detecting concentrations of the acetone within a specified concentration range, and smaller regions comprising a smaller amount of positions with suitable sensitivity for detecting concentrations of the acetone outside of the specified concentration range.

In the case where the sorbent material comprises a plurality of exposure regions, said plurality of exposure regions having different sensitivities to the acetone, the different sensitivities may result from a spatially differential local concentration or local amount of the hydroxylammonium salt at different exposure regions of the sorbent material. In this case, the sensitivity to the acetone may increase at an exposure region of the sorbent material having increased local concentration or amount of the hydroxylammonium salt when compared to an exposure region of the sorbent material having decreased local concentration or amount of the hydroxylammonium salt.

In certain embodiments, the sorbent material may have an exposure surface for exposure to the fluid. The exposure surface may comprise a coating having spatially differential permeability to the acetone, so as to provide spatially differential sensitivity to the sorbent material. The coating may increase or decrease the concentration of the acetone concentration at the detecting surface when compared with the acetone concentration exposed to the sorbent material. The coating may have variable thickness, such that, for example, the sorbent material has higher sensitivity to the acetone at a position where the coating is thinner, and lower sensitivity to the acetone at a position where the coating is thicker. The coating may comprise apertures, e.g. pores, allowing a fraction of the acetone to pass therethrough. It may comprise channels, for example microfluidic channels allowing a fraction of the acetone to pass therethrough. The channels may, for example, have varying flow rates and/or permeability to the acetone. The spatially differential sensitivity may result from the varying flow rate of the channels. For example, the sorbent material portion below the coating may have uniform sensitivity, but the varying flow rates of the channels of the coating may provide the sorbent material spatially differential sensitivity. In the case where the sorbent material has spatially differential sensitivity to the acetone, the spatially differential sensitivity may result from the spatially differential permeability of the coating. For example, the sorbent material portion below the coating may have uniform sensitivity, but the spatially differential permeability of the coating may provide the sorbent material spatially differential sensitivity. In the case where the sorbent material has spatially differential sensitivity to the acetone, the spatially differential sensitivity may result from a differential in time of exposure at a sorbent material portion below the coating because of the differential permeability of the coating. For example, the sorbent material portion below the coating may have uniform sensitivity, but the varying exposure time at a sorbent material portion below the coating as a result of the coating may provide the sorbent material spatially differential sensitivity. The sensitivity to the acetone may increase at a position of the sorbent material having increased permeability of the coating when compared to a position of the sorbent material having decreased permeability of the coating. The coating may comprise a blocking agent that interacts with the acetone. The blocking agent may react with, bind to, and/or adsorb the acetone.

The blocking agent may affect the dynamic range of the device by adjusting the local concentration of the acetone at a position of the sorbent material. The blocking agent may be used in a different concentration or amount at a plurality of positions of the sorbent material. In the case where the sorbent has spatially differential sensitivity to the acetone, the spatially differential sensitivity may result from the different concentration or amount of the blocking agent at different positions of the sorbent material. The sensitivity to the acetone may increase at a position of the sorbent material having decreased local concentration or amount of the blocking agent when compared to a position of the sorbent material having increased local concentration or amount of the blocking agent.

The sorbent material may comprise channels, for example microfluidic channels allowing a fraction of the acetone to pass therethrough. The channels may, for example, have varying flow rates and/or permeability to the acetone. The fluid may pass through a microfluidic channel prior to contacting an exposure region. In the case where the sorbent material has spatially differential sensitivity to the acetone, the spatially differential sensitivity may result from varying exposure time at the plurality of exposure regions. For example, the exposure regions may have inherent uniform sensitivity, but the varying exposure time at different exposure regions may provide the sorbent material spatially differential sensitivity.

The sorbent material may further comprise a weak base sorbed thereon and/or therein. The weak base may be, for example, selected from the group consisting of bicarbonate salts, ammonia, aniline, phenolate salts, and mixtures thereof. It may be, for example, a bicarbonate salt. It may be sodium bicarbonate. The weak base may be used to adjust the working range of the device by increasing the pH at a surface of the sorbent material. The weak base may have a pKb of greater than about 3, or greater than about 4, 5, 6, 7, 8, 9, or 10. The weak base may have a pKb of from about 3 to about 12, or from about 3 to about 10, about 3 to about 8, about 5 to about 12, about 5 to about 10, about 6 to about 9, or about 7 to about 8. It may be, for example, about 3, 3.5, 4, 4.5, 5, 5.5, 6, 6.5, 7, 7.2, 7.4, 7.6, 7.8, 8, 8.2, 8.4, 8.6, 8.8, 9, 9.5, 10, 10.5, 11, 11.5, or 12.

### Analyte

In all aspects of the invention, the analyte is a carbonyl-containing compound, namely acetone.

### Fluid

The fluid may be a gas, liquid, vapour or combination thereof. It may be a dispersion. It may be an emulsion. It may be a microemulsion. It may be a sol. It may be a solution, for example an aqueous or organic solution. It may be a suspension. It may be a biological fluid. It may be, for example, whole blood, breath, sweat, sputum, mucus, urine, serum, semen, plasma, or saliva. The fluid may be derived from a solid, for example by dissolution of the solid in a solvent.

### Liquid

The liquid may be water, or some other suitable solvent. It may be an organic solvent, an aqueous solution, or a combination thereof. It may be suitable for dissolving the hydroxylammonium salt. It may be suitable for dissolving the acetone. The liquid, or a portion thereof, may be a component of the fluid. Alternatively, or additionally, the liquid, or a portion thereof, may be separate to the fluid. In the case where the liquid is separate to the fluid, the liquid may be added to the hydroxylammonium salt prior to or during exposure of the fluid to the hydroxylammonium salt. The liquid may, for example, be pre-sorbed onto the sorbent material. The hydroxylammonium salt may be in a solution with the liquid prior to exposure to the fluid.

The liquid volume may be about 1 mL or less. It may be less than about 0.5 mL, 0.2 mL, 0.1 mL, 0.05 mL, 0.02 mL, or 0.01 mL. The liquid volume may be from about 0.001 mL to about 1 mL, or from about 0.001 mL to about 0.5 mL, about 0.001 mL to about 0.2 mL, about 0.005 mL to about 0.5 mL, about 0.005 mL to about 0.05 mL, or about 0.005 mL to about 1 mL.

### Acquisition Device

The acquisition device is configured to determine the concentration of the acetone from the differential response. It may be configured to measure a response of the sensor to the acetone as a function of time. It may be configured to determine the concentration of the acetone utilising at least one detection position of the sensor. It may be configured to measure the sensor response at a defined or predetermined time after exposure of the sensor to the acetone. The time may be, for example, from about 0.01 seconds to about 120 minutes, or it may be from about 0.01 seconds to about 60 minutes, about 0.01 seconds to about 30 minutes, about 0.01 seconds to about 20 minutes, about 0.01 seconds to about 10 minutes, about 0.01 seconds to about 5 minutes, about 0.01 seconds to about 2 minutes, about 0.01 seconds to about 1 minute, about 0.01 seconds to about 30 seconds, about 30 seconds to about 60 minutes, about 1 minute to about 60 minutes, about 5 minutes to about 60 minutes, about 10 minutes to about 60 minutes, about 5 minutes to about 30 minutes, or about 10 minutes to about 30 minutes. It may be less than about 60 minutes, or less than about 30, 20, 10, 5, 2, 1, 0.5, or 0.1 minutes. It may be, for example, about 0.01, 0.02, 0.05, 0.1, 0.2, 0.5, 1, 2, 5, 10, 15, 20, 25, 30, 35, 40, 45, 50, or 55 seconds. It may be, for example, about 1, 2, 5, 10, 15, 20, 30, 40, 50, 60, 70, 80, 90, 100, 110, or 120 minutes.

The acquisition device may be configured to measure the time taken for at least one of the sensor detection positions to reach a defined response level after exposure of the sensor to the acetone. The defined response level may be, for example, from about 0% to about 100% of the maximum possible response level, or it may be from about 0% to about 90%, about 0% to about 80%, about 0% to about 70%, about 0% to about 60%, about 0% to about 50%, about 0% to about 40%, about 0% to about 30%, about 0% to about 20%, about 0% to about 10%, about 0% to about 5%, about 0.5% to about 100%, about 1% to about 100%, about 5% to about 100%, about 10% to about 100%, about 20% to about 100%, about 20% to about 60%, about 40% to about 60%, or about 20% to about 50% of the maximum possible response level. It may be, for example, about 0, 0.01, 0.02, 0.05, 0.1, 0.2. 0.5, 1, 2, 5, 10, 20, 25, 30, 40, 50, 60, 70, 75, 80, 90, or 100% of the maximum possible response level.

The acquisition device may be configured to measure the time taken for at least one of the sensor detection positions to reach a saturated response level after exposure of the sensor to the acetone. It may be configured to measure the time taken for at least one of the sensor detection positions to reach a peak response level after exposure of the sensor to the acetone. It may be configured to measure the time taken for at least one of the sensor positions to reach a steady state response level after exposure of the sensor to the acetone.

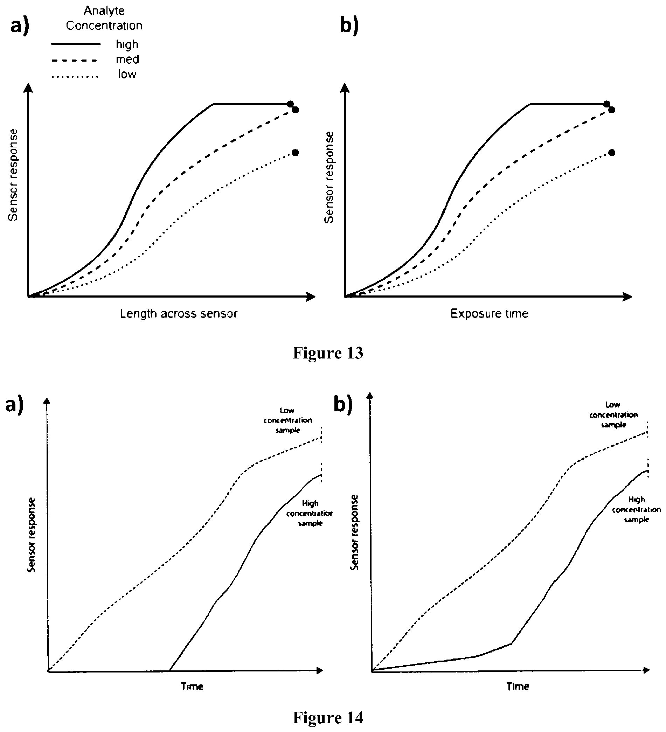

The acquisition device comprises a processor configured to calculate the concentration of the acetone utilising the differential response of the sensor to the acetone. The processor may be configured to calculate the concentration of the acetone based on a response of the sensor to the acetone as a function of time. The processor may be configured to calculate the concentration of the acetone based on a response of the sensor to the acetone at a defined period after exposure of the sensor to the acetone. The processor may be configured to calculate the concentration of the acetone based on the time taken for at least one of the sensor detection positions to reach a defined response level after exposure of the sensor to the acetone. The processor may be configured to calculate the concentration of the acetone based on the time taken for at least one of the sensor detection positions to reach a saturated response level after exposure of the sensor to the acetone. The processor may be configured to calculate the concentration of the acetone based on the time taken for at least one of the sensor detection positions to reach a peak response level after exposure of the sensor to the acetone. The processor may be configured to calculate the concentration of the acetone based on the time taken for at least one of the sensor detection positions to reach a steady state response level after exposure of the sensor to the acetone. The processor may be configured to calculate the concentration of the acetone based on the area under the curve of a plot of the sensor response as a function of time for at least one of the sensor detection positions. It may be configured to calculate the concentration of the acetone based on the integral the sensor response as a function of time for at least one of the sensor detection positions. It may be configured to calculate the concentration of the acetone based on the gradient or slope of the sensor response as a function of time for at least one of the sensor detection positions. Figure 13(a) shows sensor responses as a function of length across the sensor for a continuous spatially differential sensor such as depicted in Figure 5(a), at a high, medium and low analyte concentration. Figure 13(b) shows sensor responses as a function of exposure time for a sensor such as depicted in Figure 3, at a high, medium and low analyte concentration. The differences in curve shape, integration and gradient at each concentration can be utilised by the processor to determine the analyte concentration. Figures 14(a) and 14(b) depict example sensor responses as a function of time, where the responses exhibiting initial delays. In this example case, the slope or gradient of the sensor response as a function of time may be used to determine the concentration of the analyte.

As discussed earlier, the sensor is configured to respond to the acetone at a plurality of detection positions on the sensor having different sensitivities to the acetone. The acquisition device may be configured to calculate the concentration of the acetone utilising at least the following:

if the sensor reaches a saturated response level at all of the plurality of detection positions on the sensor:
a response of the sensor to the acetone as a function of time at the detection position on the sensor having the least sensitivity to the acetone;
if the sensor does not reach a saturated response level at any of the plurality of detection positions on the sensor:
a response of the sensor to the acetone at the detection position on the sensor having the greatest sensitivity to the acetone; and
if the sensor responds to the acetone to reach a saturated response level at some, but not all, of the plurality of detection positions on the sensor:
one or more of:

the ratio of the number of detection positions on the sensor that reach a saturated response level to the number of detection positions on the sensor that do not reach a saturated response level,
the time required to reach a saturated response level for at least one of the detection positions on the sensor that reaches a saturated response level, and
a response of the sensor to the acetone for at least one of the detection positions on the sensor that does not reach a saturated response level.

In the case where the differential response of the sensor is temporally differential, the acquisition device may be configured to calculate the concentration of the acetone utilising at least one or more of a time required to reach a predetermined response level for at least one position on the sensor after exposure of the fluid to the sensor; and a response level for at least one position on the sensor at a predetermined time after exposure of the fluid to the sensor.

In the case where the differential response of the sensor is temporally differential and spatially differential, the acquisition device may be configured to calculate the concentration of the acetone using a sensor response at two or more temporal points at one or more positions of the sensor.

The processor is configured to calculate the concentration of the acetone using the sensor response at one or more of the detection positions of the sensor, optionally 2, 3, 4, 5, 10, 50, 100 or more than 100 detection positions thereof.

At a detection position of the sensor where the sensor reaches a saturated response level upon exposure to the concentration of the acetone, the processor may be configured to calculate the concentration of the acetone based on the time taken for the sensor to reach saturation. At a detection position of the sensor where the sensor reaches a non-saturated response level upon exposure to the concentration of the acetone, the processor may be configured to calculate the concentration of the acetone based on any one or more of: the peak response level, the steady state response level, the time taken for the sensor to reach the peak response level, and the time taken for the sensor to reach the peak response level.

In the case where the sensor response to the acetone produces a colour change, the acquisition device may comprise a camera and a light source. The acquisition device may measure data from the sensor over a period of time, such that the final colour (which may be, for example, the steady state response, or peak response) of a detection position of the sensor is measured. It may measure the time taken to reach the steady state response at a detection position of the sensor. It may measure the time taken to reach the peak response at a detection position of the sensor. It may measure the time taken to reach a saturated response for at least one detection position of the sensor that reaches a saturation level.

In the case where the sensor response to the acetone produces a change in resistance, the acquisition device may measure changes in local conductivity across the sensor. The change in resistance may be caused by changes in the ionic concentration of the sensor following the interaction with the acetone. The acquisition device may for example comprise multiple electrode pairs that may be connected at either side of the sensor, perpendicular to a sensitivity gradient, and the conductivity of the sensor material may be measured by the acquisition device for at least one detection position of the sensor.

The acquisition device comprises a processor and a detector.

**Detector**

The detector may be an ion sensitive field effect transistor (ISFET), chemiresistive sensor, potentiometric sensor, spectroscopic sensor, colorimetric sensor, fluorometric sensor, thermal sensor, or a combination thereof. It may be a combined sensor, for example a combined ISFET and colorimetric sensor. In the case where the detector is a spectroscopic or colorimetric sensor, it may be sensitive to a defined range of electromagnetic radiation, for example, ultraviolet-A (UVA), ultraviolet-B (UVB), ultraviolet-C (UVC), visible light, near-infrared (NIR), far-infrared (FIR), or some combination thereof. It may be a change-coupled device (CCD), an active pixel sensor, or a hybrid thereof. It may, for example, be a complementary metal-oxide-semiconductor (CMOS) sensor, or a scientific complementary metal-oxide-semiconductor (sCMOS) sensor. The skilled person will understand that various sensing apparatuses can be used as the detector such as, for example, a red-green-blue (RGB) colour sensor, a photodiode, a photoresistor, a phototransistor, a light emitting diode (LED), a photovoltaic cell, or an image sensor used, for example, in a smart phone. The detector may comprise a camera and a light source. It may comprise a smart phone. The device may comprise one or more filters, optionally optical filters, which selectively allow particular wavelengths or combinations of wavelengths of electromagnetic radiation to reach the spectroscopic sensor.

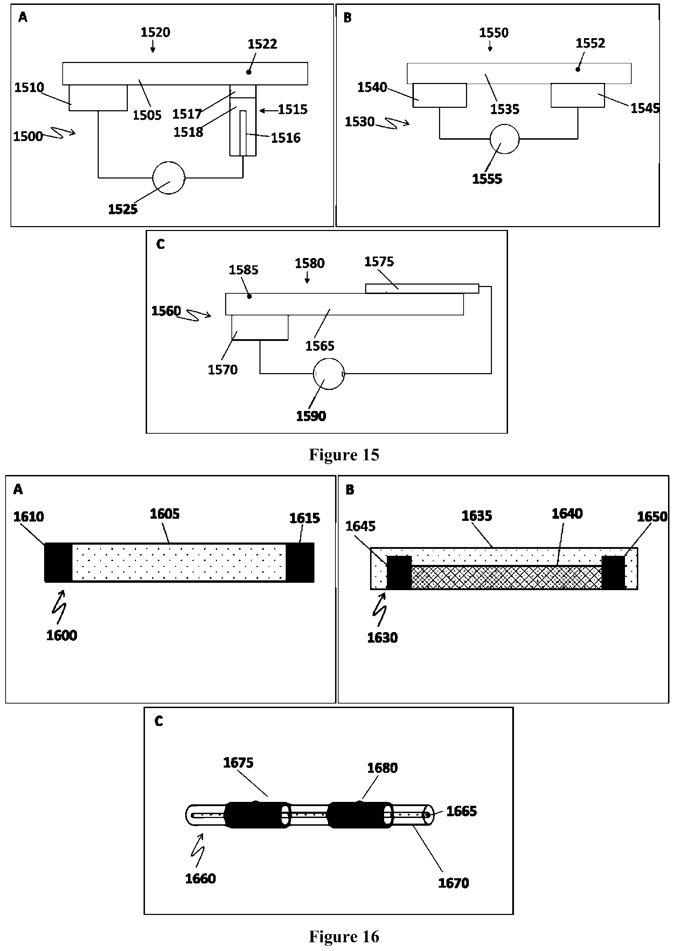

In the case where the detector is an ISFET, the hydroxylammonium salt sorbent material may be utilised as the gate electrode for the transistor and an oxide surface may be used as a gate material to separate the reaction media from the transistor channel. Typical gate materials may include $SiO_2$, $Si_3N_4$, $Al_2O_3$ or $Ta_2O_5$. Hydrolysis of the gate material may occur when it is exposed to ions in the sorbent material, causing the threshold voltage of the ISFET to vary as a function of ion concentration. Figure 15(a-c) show different example layouts of the ISFET detector within the device. In certain embodiments where the detector is an ISFET, the sorbent material may be disposable, that is suitable for single use, and the ISFET may be reusable. In particular embodiments, a reference electrode may be positioned adjacent to the ISFET and may be reusable, i.e. non-disposable. The ISFET and the reference electrode may both be in contact with the acetone. In another embodiment, the reference electrode may be an Ag/AgCl 'pseudo reference electrode', which may be disposable and may be positioned within, or on the surface of the sorbent material. The disposable pseudo reference electrode may be brought into conductive contact with conductive pads when the disposable sorbent material is inserted into the device, thereby forming a sensing circuit.

Example device **1500** shown in Figure 15(a) comprises ISFET **1510**, sorbent material **1505** (having hydroxylammonium salt sorbed therein and/or thereon), a standard reference electrode **1515** (consisting of electrode wire **1516**, electrolyte **1518**, and membrane **1517**) and processor **1525**. When a user supplies a breath sample **1520** to the sorbent material **1505**, reaction of a carbonyl-containing compound in the breath sample **1520** with the hydroxylammonium salt sorbed in and/or sorbed on sorbent material 1505, produces an increase in hydronium ion concentration **1522**, resulting in a change in the threshold voltage of the ISFET **1510** relative to the standard reference electrode **1515**. The processor **1525** calculates the concentration of the carbonyl-containing compound in the breath sample from the change in voltage.

Example device **1530** shown in Figure 15(b) comprises ISFET **1540**, sorbent material **1535** (having hydroxylammonium salt sorbed therein and/or thereon), a Reference Electrode Field Effect Transistor (REFET) **1545** and processor **1555**. When a user supplies a breath sample **1550** to the sorbent material **1535**, reaction of a carbonyl-containing compound in the breath sample **1550** with the hydroxylammonium salt sorbed in and/or sorbed on sorbent material **1535,** produces an increase in hydronium ion concentration **1552,** resulting in a change in the threshold voltage of the ISFET **1540** relative to the REFET **1545.** The processor **1555** calculates the concentration of the carbonyl-containing compound in the breath sample from the change in voltage.

Example device **1560** shown in Figure 15(c) comprises ISFET **1570,** sorbent material **1585** (having hydroxylammonium salt sorbed therein and/or thereon), a disposable pseudo reference electrode **1575** and processor **1590.** When a user supplies a breath sample **1580** to the sorbent material **1585,** reaction of a carbonyl-containing compound in the breath sample **1580** with the hydroxylammonium salt sorbed in and/or sorbed on sorbent material **1565,** produces an increase in hydronium ion concentration **1585,** resulting in a change in the threshold voltage of the ISFET **1570** relative to the disposable pseudo reference electrode **1575.** The processor **1590** calculates the concentration of the carbonyl-containing compound in the breath sample from the change in voltage.

In the case where the detector is a potentiometric sensor, it may be a potentiostat. In this case, it may comprise three electrodes in contact with the liquid and/or sorbent material, where the potential between a reference electrode and working electrode varies as a function of ion concentration (e.g. pH) of the liquid.

In the case where the detector is a fluorometric sensor, it may be, for example, a fluorometer. In this case, the device may comprise one or more fluorophores that are sensitive to one or more products from the reaction of acetone with a hydroxylammonium salt.

In the case where the detector is a thermal sensor, it may be, for example, a thermometer. In this case the detector may measure the temperature change caused by the enthalpy of reaction of acetone with a hydroxylammonium salt.

In the case where the detector is a chemiresistor sensor, the liquid and/or sorbent material may be placed in direct contact with the chemiresistor sensor. The chemiresistor may comprise two opposing electrodes separated by the liquid and/or sorbent material, through which a current is passed at a stable voltage. The chemiresistor measures the resistance across the liquid and/or sorbent material. A higher number of ions (e.g. hydronium ions) in the liquid and/or sorbent material may result in a lower resistance measurement.

Alternatively, ion sensitive intermediate substances may be utilised, such as conductive polymers or carbon materials that alter their resistivity depending on the concentration of available ions in their surrounding environment. Furthermore, another method of chemiresistive sensing, a $C_4D$ sensor (Capacitively-Coupled Contactless Conductivity Detector) may be utilised to determine the resistivity of the liquid and/or sorbent material. An example of device arrangements for a direct resistivity measurement, an intermediate resistivity measurement and $C_4D$ sensor measurement are shown in Figures 16(a), 16(b) and 16(c) respectively.

The example device **1600** depicted in Figure 16(a) comprises sorbent material **1605** (having hydroxylammonium salt sorbed therein and/or thereon) between a cathode **1610** and anode **1615.** When a breath sample is exposed to the device, the reaction of a carbonyl-containing compound in the breath sample with the hydroxylammonium salt sorbed in and/or sorbed on sorbent material **1605,** produces an increase in hydronium ion concentration, resulting in a change in the resistance between cathode **1610** and anode **1615.** A processor (not shown) calculates the concentration of the carbonyl-containing compound in the breath sample from the change in resistance.

The example device **1630** depicted in Figure 16(b) comprises sorbent material 1635 (having hydroxylammonium salt sorbed therein and/or thereon) in close contact with an ion-sensitive material **1640** which is located between cathode **1645** and anode **1650.** The resistivity of the ion-sensitive material changes depending on the concentration of available ions in the surrounding environment. When a breath sample is exposed to the device, the reaction of a carbonyl-containing compound in the breath sample with the hydroxylammonium salt sorbed in and/or sorbed on sorbent material **1635,** produces an increase in hydronium ion concentration in the sorbent material, resulting in a change in the resistance in the ion-sensitive material **1640** between cathode **1610** and anode **1615.** A processor (not shown) calculates the concentration of the carbonyl-containing compound in the breath sample from the change in resistance.

The example device **1660** depicted in Figure 16(c) comprises liquid **1665** (comprising hydroxylammonium salt) circumferentially surrounded by channel wall **1670.** Device **1660** also comprises actuator electrode **1675** and pickup electrode **1680.** When a breath sample is exposed to the liquid, the reaction of a carbonyl-containing compound in the breath sample with the hydroxylammonium salt in liquid **1665,** produces an increase in hydronium ion concentration in the liquid, resulting in a change in the resistance between actuator electrode **1675** and pickup electrode **1680.** A processor (not shown) calculates the concentration of the carbonyl-containing compound in the breath sample from the change in resistance.

The detector may measure the change in the variable over a period of time, such that the final change in the variable (which may be, for example, the steady state response, or peak response) is measured. It may measure the time taken to reach the steady state change in the variable. It may measure the time taken to reach the peak response (i.e. change in variable maximum). It may measure the time taken to reach a saturated response (i.e. change in variable).

The detector may be configured to measure a response (i.e. the change in the variable) of the device as a function of time. It may be configured to measure the change in the variable at a defined or predetermined time after exposure of the hydroxylammonium salt to the acetone. The time may be, for example, from about 0.01 seconds to about 120 minutes, or it may be from about 0.01 seconds to about 60 minutes, about 0.01 seconds to about 30 minutes, about 0.01 seconds to about 20 minutes, about 0.01 seconds to about 10 minutes, about 0.01 seconds to about 5 minutes, about 0.01 seconds to about 2 minutes, about 0.01 seconds to about 1 minute, about 0.01 seconds to about 30 seconds, about 30 seconds to about 60 minutes, about 1 minute to about 60 minutes, about 5 minutes to about 60 minutes, about 10 minutes to about 60 minutes, about 5 minutes to about 30 minutes, or about 10 minutes to about 30 minutes. It may be less than about 60 minutes, or less than about 30, 20, 10, 5, 2, 1, 0.5, or 0.1 minutes. It may be, for example, about 0.01, 0.02, 0.05, 0.1, 0.2, 0.5, 1, 2, 5, 10, 15, 20, 25, 30, 35, 40, 45, 50, or 55 seconds. It may be, for example, about 1, 2, 5, 10, 15, 20, 30, 40, 50, 60, 70, 80, 90, 100, 110, or 120 minutes.

The detector may be configured to measure the time taken for the device to reach a defined response level (i.e. change in variable) after exposure of the hydroxylammonium salt to the acetone. The defined response level may be, for example, from about 0% to about 100% of the maximum possible response level, or it may be from about 0% to about 90%, about 0% to about 80%, about 0% to about 70%, about 0% to about 60%, about 0% to about 50%, about 0% to about 40%, about 0% to about 30%, about 0% to about 20%, about 0% to about 10%, about 0% to about 5%, about 0.5% to about 100%, about 1% to about 100%, about 5% to about 100%, about 10% to about 100%, about 20% to about 100%, about 20% to about 60%, about 40% to about 60%, or about 20% to about 50% of the maximum possible response level. It may be, for example, about 0, 0.01, 0.02, 0.05, 0.1, 0.2. 0.5, 1, 2, 5, 10, 20, 25, 30, 40, 50, 60, 70, 75, 80, 90, or 100% of the maximum possible response level.

The detector may be configured to measure the time taken for the device to reach a saturated response level (i.e. change in variable) after exposure of the hydroxylammonium salt to the acetone. It may be configured to measure the time taken for the device to reach a peak response level (i.e. change in variable) after exposure of the hydroxylammonium salt to the acetone. It may be configured to measure the time taken for the device to reach a steady state response level (i.e. change in variable) after exposure of the hydroxylammonium salt to the acetone.

In certain embodiments the detector may be sensitive of a defined range of electromagnetic radiation, for example, UVA, UVB, UVC, visible light, NIR, FIR, or some combination thereof. It may be a CCD, an active pixel sensor, or a hybrid thereof. It may, for example, be a CMOS sensor, or a sCMOS sensor. The skilled person will understand that various sensing apparatuses can be used as the detector such as, for example, an RGB colour sensor, a photodiode, a photoresistor, a phototransistor, an LED, a photovoltaic cell, or an image sensor used, for example, in a smart phone.

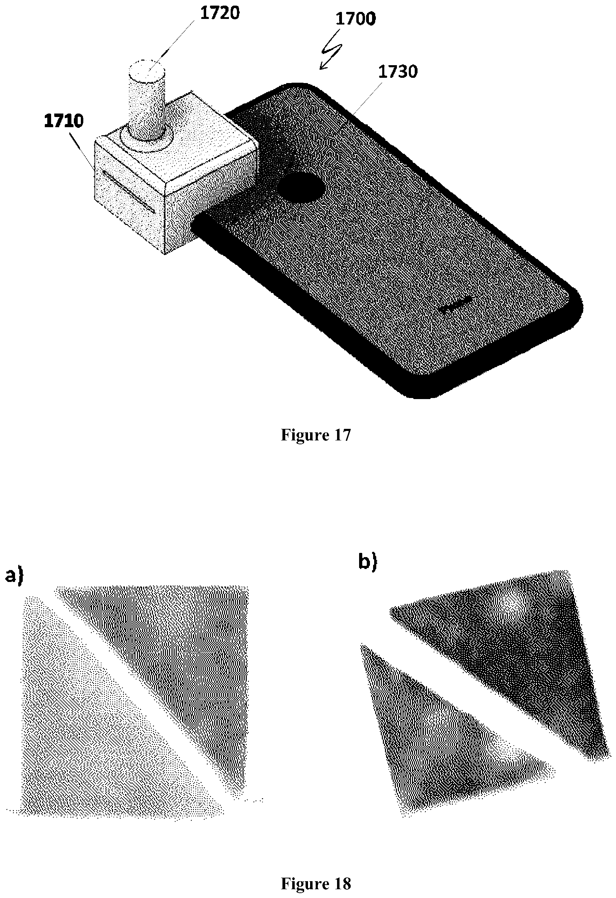

The detector may comprise a camera and a light source. It may comprise a smart phone. For example, as depicted in Figure 17, the device **1700** comprises a smart phone **1730** which performs both the detector and processor functions. That is, it is able to both measure the change in the absorption spectrum of the indicator and calculate the concentration of the carbonyl containing compound in the fluid from the change in the absorption spectrum of the indicator. In this example, the device comprises a chip **1710** that comprises the sorbent material (not shown), and a breath input **1720** for a user to breath into, thereby exposing the sorbent material to the fluid (e.g. the user's breath). In this case, the smart phone is a removable component of the device. The digital nature of the device data captured in, for example, a smart phone component of the device may allow easy transferal to external sources for data analysis. In this example a smartphone could be utilised to perform the spectrometric measurement of the change in absorption spectrum, in addition to analysis and computation. Here, a spectrometric assessment of the chip may be performed both before and after contact with user's breath, a device housing is utilised such that the camera of the smartphone is positioned beneath the acetone sensing chip, and the change in absorption spectrum is measured via the phone camera to quantify breath acetone levels.

External sources include portable computing units, such as those found in smart phones and cloud-based computing technologies. This enables the analysis of data and improvement of the detection of the acetone in the breath through methods such as machine learning. The data processing and analytics of the device response can be processed via hardware and/or software approaches via various platforms such as microcontroller, cloud computing and a smart phone.

The detector may measure the change in the absorption maximum over a period of time, such that the final change in absorption maximum (which may be, for example, the steady state response, or peak response) is measured. It may measure the time taken to reach the steady state change in absorption maximum. It may measure the time taken to reach the peak response (i.e. change in absorption maximum). It may measure the time taken to reach a saturated response (i.e. change in absorption maximum).

The detector may be configured to measure a response (e.g. the change in the absorption spectrum of the indicator) of the device as a function of time. It may be configured to measure the change in the absorption spectrum of the indicator at a defined or predetermined time after exposure of the sorbent material to the acetone. The time may be, for example, from about 0.01 seconds to about 120 minutes, or it may be from about 0.01 seconds to about 60 minutes, about 0.01 seconds to about 30 minutes, about 0.01 seconds to about 20 minutes, about 0.01 seconds to about 10 minutes, about 0.01 seconds to about 5 minutes, about 0.01 seconds to about 2 minutes, about 0.01 seconds to about 1 minute, about 0.01 seconds to about 30 seconds, about 30 seconds to about 60 minutes, about 1 minute to about 60 minutes, about 5 minutes to about 60 minutes, about 10 minutes to about 60 minutes, about 5 minutes to about 30 minutes, or about 10 minutes to about 30 minutes. It may be less than about 60 minutes, or less than about 30, 20, 10, 5, 2, 1, 0.5, or 0.1 minutes. It may be, for example, about 0.01, 0.02, 0.05, 0.1, 0.2, 0.5, 1, 2, 5, 10, 15, 20, 25, 30, 35, 40, 45, 50, or 55 seconds. It may be, for example, about 1, 2, 5, 10, 15, 20, 30, 40, 50, 60, 70, 80, 90, 100, 110, or 120 minutes.

The detector may be configured to measure the time taken for the device to reach a defined response level (i.e. change in absorption spectrum of the indicator) after exposure of the sorbent material to the acetone. The defined response level may be, for example, from about 0% to about 100% of the maximum possible response level, or it may be from about 0% to about 90%, about 0% to about 80%, about 0% to about 70%, about 0% to about 60%, about 0% to about 50%, about 0% to about 40%, about 0% to about 30%, about 0% to about 20%, about 0% to about 10%, about 0% to about 5%, about 0.5% to about 100%, about 1% to about 100%, about 5% to about 100%, about 10% to about 100%, about 20% to about 100%, about 20% to about 60%, about 40% to about 60%, or about 20% to about 50% of the maximum possible response level. It may be, for example, about 0, 0.01, 0.02, 0.05, 0.1, 0.2. 0.5, 1, 2, 5, 10, 20, 25, 30, 40, 50, 60, 70, 75, 80, 90, or 100% of the maximum possible response level.

The detector may be configured to measure the time taken for the device to reach a saturated response level (i.e. change in absorption spectrum of the indicator) after exposure of the sorbent material to the acetone. It may be configured to measure the time taken for the device to reach a peak response level (i.e. change in absorption spectrum of the indicator) after exposure of the sorbent material to the acetone. It may be configured to measure the time taken for the device to reach a steady state response level (i.e. change in absorption spectrum of the indicator) after exposure of the sorbent material to the acetone.

**Processor**

The processor is configured to calculate the concentration of the acetone in the fluid from the change in the variable. It may be configured to determine the concentration of the acetone utilising at least one exposure region of the sensor. It may, for example, determine the concentration of the acetone by comparing the change in variable to a standard curve. In this example, the standard curve may be a plot of the change in variable for a variety of known acetone concentrations. The standard curve may, for example, be expressed as a mathematical function. It may, for example, express the concentration of the acetone as a function of the change in variable. In this case the processor may use the function to calculate the concentration of the acetone from the change in variable measured by the detector.

The processor may be configured to calculate the concentration of the acetone using the response at one or more of the exposure regions of the sorbent layer, optionally 2, 3, 4, 5, 10, 50, 100 or more than 100 exposure regions thereof.

In certain embodiments the processor may be configured to calculate the concentration of the acetone in the fluid from the change in the absorption spectrum of the indicator. It may be configured to determine the concentration of the acetone utilising at least one exposure region of the sensor. It may, for example, determine the concentration of the acetone by comparing the change in absorption spectrum of the indicator to a standard curve. In this example, the standard curve may be a plot of the change in absorption spectrum of the indicator for a variety of known acetone concentrations. The standard curve may, for example, be expressed as a mathematical function. It may, for example, express the concentration of the acetone as a function of the change in absorption spectrum of the indicator. In this case the processor may use the function to calculate the concentration of the acetone from the change in absorption spectrum measured by the detector.

The processor may be configured to calculate the concentration of the acetone using the response at one or more of the exposure regions of the sorbent layer, optionally 2, 3, 4, 5, 10, 50, 100 or more than 100 exposure regions thereof.

**Method of determining a concentration of acetone**

Disclosed herein is a method of determining a concentration of acetone in a fluid in accordance with the second aspect.

The method comprises the step of calculating the concentration of the acetone utilising the differential response of the sensor to the acetone. The differential response is spatially differential, and the sensor is configured to respond to the acetone at a plurality of detection positions on the sensor having different sensitivities to the acetone.

The differential response may be temporally differential, and said calculating may utilise at least one or more of a time required to reach a predetermined response level for at least one position on the sensor after exposure of the fluid to the sensor; and a response level for at least one position on the sensor at a predetermined time after exposure of the fluid to the sensor.

The method may further comprise passing the fluid through a flow channel, or into an analysis chamber, in which the sensor is disposed.

The method may use the system disclosed herein. The method uses the sensor disclosed herein. The system disclosed herein may be used in the method.

The method may further comprise the step of calculating the concentration of the acetone based on a response of the sensor to the acetone as a function of time. The calculating may be performed by the processor.

The method as hereinbefore described comprises exposing the fluid to a hydroxylammonium salt in the presence of a liquid, so as to produce a change in a variable, the variable being dependent upon the pH of the liquid; measuring the change in the variable; and determining the concentration of the acetone in the fluid from the change in the variable.

The acetone, fluid, and hydroxylammonium salt may be as described hereinbefore in relation to the device. The hydroxylammonium salt may be sorbed in and/or on a sorbent material. The sorbent material may be as described herein in relation to the device. The liquid may be as described herein in relation to the device.

The variable may be an absorbance, fluorescence, resistance, ion concentration, temperature, voltage, or current. In the case where the variable is an ion concentration, it may be a hydronium ion concentration.

In the case where the variable is an absorbance, it may be the absorbance of an indicator. In this instance, a change in the absorption spectrum of the indicator may comprise a change in wavelength. It may comprise a change in intensity. The change in the absorption spectrum may be substantially independent of the volume of the fluid exposed to the hydroxylammonium salt and halochromic indicator. It may be substantially independent of the flow rate of the fluid exposed to the hydroxylammonium salt and halochromic indicator. In the case where the indicator is a halochromic indicator, the absorption spectrum of the halochromic indicator may vary monotonically over an operational pH range. The halochromic indicator may be selected from the group consisting of methyl orange, methyl red, methyl yellow chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline, phenol blue, and mixtures thereof. It may, for example, be selected from the group consisting of thymol blue, bromophenol blue, and mixtures thereof.

The method may further comprise adding the liquid to the hydroxylammonium salt, prior to or during exposure of the fluid to the hydroxylammonium salt.

The method may be capable of rapidly determining the concentration of the acetone in the fluid. It may, for example, be capable of determining the concentration of the acetone in the fluid in less than about 10 minutes, or less than about 5 minutes, 2 minutes, 1 minute, 45 seconds or 30 seconds after exposure of the fluid to the hydroxylammonium salt.

In some instances, the method does not comprise a titration step. In the case where the method does not comprise a titration step, the method may require only a small liquid volume, such as less than 1 mL. This may enable the method to be integrated into a simplified device design when compared with devices requiring a titration step, and/or may enable the device to be portable. In such a case, the method may be simple to use. That is, it may not require a highly skilled operator. It may, for example, be suitable for an unskilled operator to use.

The method may use the device described herein. The device described herein may be used in the method.

The method may comprise exposing the fluid to an hydroxylamine salt and an indicator so as to produce a change in absorption spectrum of the indicator, wherein the hydroxylamine salt and indicator are sorbed in and/or on a sorbent material; measuring the change in the absorption spectrum of the indicator; and determining the concentration of the acetone in the fluid from the change in the absorption spectrum of the indicator.

The acetone, fluid, indicator, and hydroxylamine salt may be as described hereinbefore in relation to the device. The hydroxylamine salt and/or indicator may be in contact with a weak base. The weak base may be as described herein in relation to the device.

The change in the absorption spectrum of the indicator may comprise a change in wavelength. It may comprise a change in intensity. The change in the absorption spectrum may be substantially independent of the volume of the fluid exposed to the hydroxylamine salt and halochromic indicator. It may be substantially independent of the flow rate of the fluid exposed to the hydroxylamine salt and halochromic indicator. In the case where the indicator is a halochromic indicator, the absorption spectrum of the halochromic indicator may vary monotonically over an operational pH range. The halochromic indicator may be selected from the group consisting of methyl orange, methyl red, methyl yellow chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline and mixtures thereof. It may, for example, be selected from the group consisting of thymol blue, bromophenol blue, and mixtures thereof.

The sorbent material may be as described hereinabove with respect to the device. The method may further comprise adding a liquid carrier, optionally to the sorbent material, prior to or during exposure of the fluid to the hydroxylamine salt and indicator. The liquid carrier may be water, or some other suitable solvent.

The method may be capable of rapidly determining the concentration of the acetone in the fluid. It may, for example, be capable of determining the concentration of the acetone in the fluid in less than about 10 minutes, or less than about 5 minutes, 2 minutes, 1 minute, 45 seconds or 30 seconds after exposure of the fluid to the hydroxylamine salt and indicator.

In some instances, the method does not comprise a titration step.

The method may use the device described herein. The device described herein may be used in the method.

**Use of a device for diagnosing and/or managing diabetes in a patient**

Disclosed herein is the use of a device as hereinbefore described for diagnosing and/or managing diabetes in a patient, wherein the device is capable of determining a concentration of acetone in a breath sample of the patient, and the device comprises a sorbent material having a hydroxylammonium salt, whereby exposure of the breath to the hydroxylammonium salt in the presence of a liquid produces a change in a variable, said variable being dependent upon the pH of the liquid; a detector for measuring the change in the variable; and a processor configured to calculate the concentration of acetone in the breath sample from the change in the variable.

The hydroxylammonium salt, sorbent material, detector, and processor may be as hereinbefore described.

The use of the device may use the method as hereinbefore described. The method as hereinbefore described may be used in the use of the device.

Disclosed herein is the use of a device as hereinbefore described for diagnosing and/or managing diabetes in a patient, wherein the device is capable of determining a concentration of acetone in a breath sample of the patient, and the device comprises a sorbent material having a hydroxylamine salt and an indicator sorbed therein and/or thereon, whereby exposure of the breath sample to the sorbent material produces a change in absorption spectrum of the indicator; a detector for measuring the change in the absorption spectrum of the indicator; and a processor configured to calculate the concentration of acetone in the breath sample from the change in the absorption spectrum of the indicator.

The sorbent material, hydroxylamine salt, and indicator may be as hereinbefore described.

The use of the device may use the method as hereinbefore described. The method as hereinbefore described may be used in the use of the device.

**Methods of managing and diagnosing diabetes**

Disclosed herein is a method of managing diabetes in a patient on a diabetes treatment plan. The method comprises determining an acetone concentration of a breath sample of the patient using the method of determining a concentration of acetone in a fluid according to the second aspect; comparing the acetone concentration to a reference acetone concentration range; and if the acetone concentration is outside the acetone concentration reference range, adjusting the diabetes treatment plan.

The diabetes treatment plan may be adjusted, for example, by modifying the administration timing or dosage of a blood glucose management medication. It may be adjusted, for example, by an administration of insulin, or modification of the patient's total daily dose of insulin. It may be adjusted by modifying the fluid and/or nutrient intake of the patient. It may be adjusted by an administration of glucagon. It may be adjusted, for example, by initiating immediate medical attention, in particular in the case of ketoacidosis.

The method may use the device disclosed herein. The device disclosed herein may be used in the method.

Disclosed herein is a method to assist in diagnosing diabetes in a patient. The method comprises determining an acetone concentration of a breath sample of the patient using the method of determining a concentration of acetone in a fluid according to the second aspect; and comparing the acetone concentration to a reference acetone concentration range to determine if the patient has diabetes.

The method may be a component of a diabetes diagnosis method. For example, it may be possible to diagnose diabetes in a patient by using the method and additionally considering other factors which may affect the concentration of ketones in the breath of the patient, such as the diet of the patient.

The method may use the device disclosed herein. The device disclosed herein may be used in the method.

Disclosed herein is a method of managing diabetes in a patient on a diabetes treatment plan comprising use of the method of the second aspect. The method comprises determining an acetone concentration of a breath sample of the patient using the method of determining a concentration of acetone in a fluid as described above; comparing the acetone concentration to a reference acetone concentration range; and if the acetone concentration is outside the acetone concentration reference range, adjusting the diabetes treatment plan.

The diabetes treatment plan may be adjusted, for example, by modifying the administration timing or dosage of a blood glucose management medication. It may be adjusted, for example, by an administration of insulin, or modification of the patient's total daily dose of insulin. It may be adjusted by modifying the fluid and/or nutrient intake of the patient. It may be adjusted by an administration of glucagon.

The method may use the device disclosed herein. The device disclosed herein may be used in the method.

**Examples**

The present invention will now be further described in greater detail with reference to the following specific examples, which should not be construed as in any way limiting the scope of the invention.

*Example 1: A sensor for the measurement of breath acetone*

Diabetes mellitus, commonly shortened to diabetes, is a serious health concern globally, and its prevalence and diabetes-related deaths have been increasing with rapid transitions of lifestyle resulting in factors such as obesity. Furthermore, recent estimates have shown the prevalence of diabetes is likely to continue to grow substantially in coming decades.

Diabetes is caused by the lack of the hormone insulin, or the body's inability to use insulin. Diabetics can suffer from low insulin levels, and the elevated levels of blood glucose results in an increase in the body's ketone concentration. This can cause diabetic ketoacidosis, a state of insulin deficiency that can progress rapidly and which if left untreated can result in coma or death. Therefore, the ability to monitor ketone levels is beneficial as it allows self-monitoring which can help prevent diabetic ketoacidosis.

Blood testing currently provides accurate and quantitative measurements, using blood glucose meters. However, the invasive nature of blood testing can be painful for patients, is expensive, and presents risk of infection as well as injury if performed incorrectly. Urine testing is also available but is inaccurate. Acetone levels in human breath have been correlated with ketone bodies in plasma, and therefore in this embodiment, as sensor is provided that is able to sense a concentration of acetone in human breath.

In this example, the differential sensitivity to acetone on the sensor is produced by a varying concentration or amount of a selectively reactive chemical, such as hydroxylamine hydrochloride, hydroxylammonium sulfate, or a combination thereof at positions of the sensor.

The sensor produces a colour change response upon exposure of the sensor to acetone in a breath sample. The colour change at different sensor positions depends upon the reaction between acetone and the selectively reactive chemical. The colour-metric detection method is utilised to detect the degree of reaction between the breath acetone and the reagent. This reaction causes a colour change across the sensor, to varying degrees, divided into segment A and B as depicted in Figure 5(a). Segment A consists of the area with a noticeable colour gradient, while segment B consists of the area of the sensor with a homogenous colour.

The change in sensitivity across the sensor can be achieved through multiple methods. In this case, the concentration of hydroxylamine hydrochloride is adjusted across the sensor, such that there is a gradient from low concentration to high concentration. This change in concentration causes a change in sensitivity along the length of the sensor, both through changes in the degree of reaction due to the equilibrium between acetone and hydroxylamine, and the initial concentration of $H^+$ due to partial dissociation of hydroxylamine hydrochloride leading to a difference in the signal produced by the colour change of the secondary reaction.

In this case, the 0% response for a given local section of the sensor, is defined as the colour of the media before any reaction with acetone. The steady state response level is the point at which there is no further colour change, despite further increases in $H^+$ caused by the reaction. The colour change may be assessed visually. Alternatively, spectroscopic methods may be used to accurately quantify the change.

*Example 2: Spatially differential response acetone sensor*

The sensors depicted in Figure 18 each have two discrete triangular shaped regions with higher (top right triangle) and lower (bottom left triangle) sensitivity to acetone. A concentration of 0.633 molL$^{-1}$ and 1.27 molL$^{-1}$ of hydroxylamine hydrochloride solution was used for the higher and lower sensitivity regions respectively. Figure 18(a) shows the colour change in the sensor after exposure to 10 ppm acetone. At this acetone concentration the sensor clearly produces distinct colours at each region. Figure 18(b) shows the colour change in the sensor after exposure to 50 ppm acetone. At this acetone concentration the two regions of the sensor produce the same colour, due to a saturated detector response at both regions.

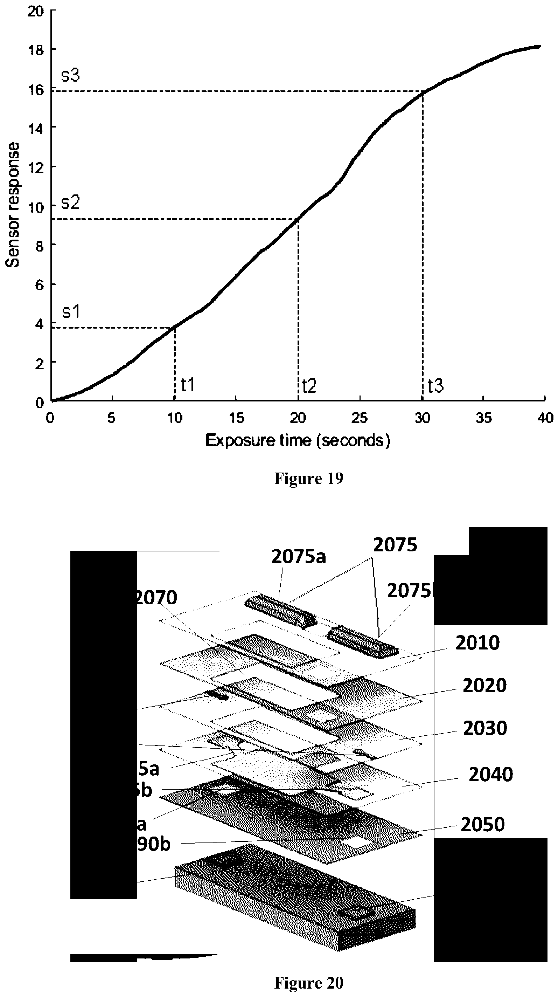

Figure 19 shows a sensor response plotted as a function of time for a position of an example sensor having a rectangular shape and using a 1.08 molL$^{-1}$ hydroxylamine hydrochloride solution. A TCS230 colour sensor was used to detect the red colour change as a function of time for the first 40 seconds after exposure of the sensor to a fluid with a 50 ppm concentration of acetone.

Prototype structures to achieve breath ketone measurement based on the invention may vary. In general, they can be divided into two categories, with air storage and without air storage i.e. a pass-through approach. The former requires the user to breath into an intermediate storage bag, to contain a specified volume of breath. After this bag is filled, the breath is passed over the sensor at a controlled flow rate, to produce a signal response as previously described. The latter requires the user to breathe directly over the sensor, without the use of intermediate storage, the volume of breath that the patient supplies can either be controlled, or measured, for calibration with the signal response produced by the sensor.

*Example 3: Lab on chip device design*

An example of the device in a lab on chip design is shown in an exploded view in Figure 20. The device **2000** comprises five layers: liquid layer **2010,** exposure layer **2020,** electrode layer **2030,** reaction layer **2040** and sensing layer **2050.** The reaction layer **2040** consists of a hydrophilic sorbent material, impregnated with hydroxylamine hydrochloride and a halochromic indicator. The sorbent material is selected to allow for fast diffusivity of solution and ion migration. The sensing layer is opaque and contains optically transparent windows positioned beneath the electrode areas, such that spectrometric measurement of these two critical areas can be performed. A spectrophotometric sensor **2060** is positioned below the sensing layer such that it can analyse the optical change at position **2055** through the transparent windows of the sensing layer during the reaction. A processor (not shown) is used to calculate the concentration of the carbonyl containing compound in the fluid from the optical change measured by the spectrophotometric sensor **2060.**

The liquid layer **2010** contains on-chip storage of suitable reaction liquids **2075** that can be dispensed on demand into the reaction layer via mechanical actuation (not shown). The device has an opening **2070** in it such that the gas-liquid interface area is exposed for reaction with breath analyte, and has small openings **2095a, 2095b** for dispensing liquids to the sensor regions **2090a, 2090b.** By dispensing the solvent through these distinct openings separated from the exposure area, meniscus formation is reduced at the edges of the gas-liquid interface within the sensing region. This reduces interference in the detector measurement of the change in absorption spectrum of the indicator.

The electrode layer has both a cathode **2080** and an anode **2085,** positioned above opposite ends of the reaction layer for applying an electric field. When the electric field is applied to the reaction layer, the charged reaction products formed after exposure of the fluid to the sorbent material of the reaction layer migrate and concentrate to a localised area of the sensor based on the charge of the species. This causes all the charged species to concentrate to a smaller sensing area, relative to the total reaction area, and consequently the localised sensor response is increased.

Immediately prior to exposing the fluid to the device, a suitable solvent, such as water, may be dispensed and distributed into the sensing region of the chip. Moreover, liquid neutralising reagents may be utilised to react with the chemical formulation present in the device after use, reducing the environmental impact associated with waste disposal of used devices. An additional benefit of this structure shown in Figure 20, is an on-chip method of storing and dispensing these fluids, whereby two containments **2075a, 2075b** for each respective fluid are positioned on the liquid layer. The fluids stored in these containers are dispensed on demand by proper actuation such as mechanical actuation, and are directed into the reaction layer where they diffuse across the hydrophilic sensing region.

Prior to breathing into the device **2000,** a liquid such as water or a solution comprising a weak base is dispensed from liquid storage **2075a** on the liquid storage layer **2010,** thereby pre-wetting and/or adjusting the pH at the reaction layer **2040.** When a user breathes into the device **2000,** the breath sample passes through opening **2070** in exposure layer **2020,** and moves to reaction layer **2040,** where it is exposed to the hydroxylamine salt and halochromic indicator sorbed therein and/or thereon. The hydroxylamine salt reacts with a carbonyl containing compound in the breath sample, reducing the local pH and resulting in a change in absorption spectrum of the indicator. A potential is applied across the electrodes **2080, 2085** on the electrode layer **2030,** which causes charged species formed as the indicator is exposed to the reduced local pH to migrate through small openings **2095a, 2095b** to sensor areas **2090a, 2090b,** thereby concentrating the charged species in these areas. The spectrophotometric sensor **2060** measures the absorption spectrum change at position **2055** compared to a baseline level, and the processor (not shown) determines the concentration of carbonyl containing compound from the absorption spectrum change. After use, a neutralising agent is dispensed from liquid storage **2075b** that neutralises the chemical formulation present in the device after use, thereby reducing the environmental impact associated with waste disposal of the used device and/or layers thereof.

*Example 4: Lab on chip device design with multiple exposure regions having different sensitivities to the carbonyl containing compound*

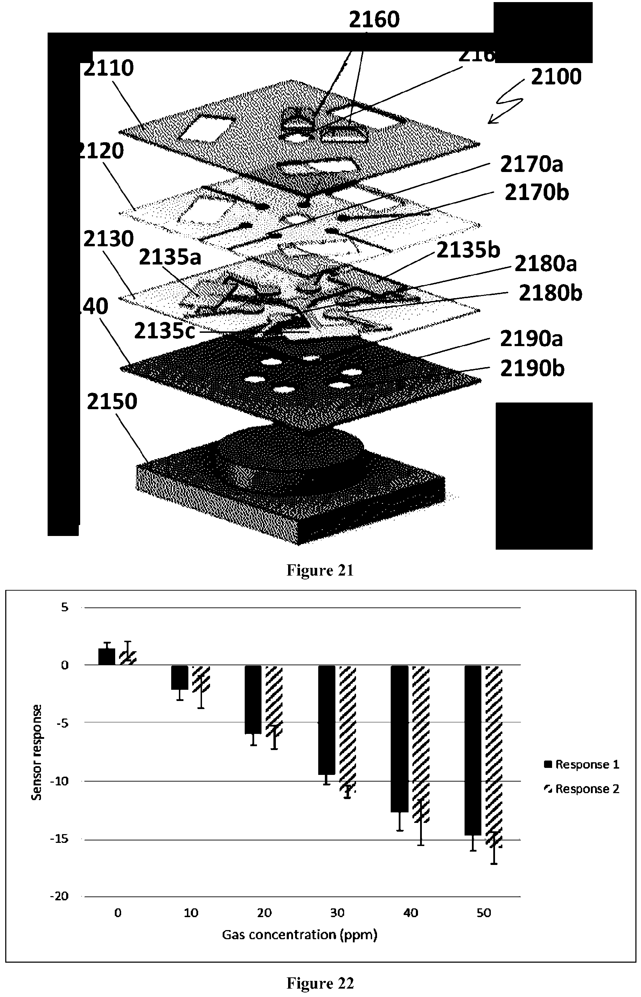

Figure 21 shows an example of a lab on chip device design depicted in an exploded view, where the sorbent material has multiple exposure regions having different sensitivities to the carbonyl compound. Each layer of device **2100** has a similar function to that described in Example 3 (i.e. exposure layer **2110,** electrode layer **2120,** reaction layer **2130** and sensing layer **2140).** However, in this case the reaction layer contains multiple distinct exposure regions which provide for an increase in working range of the device, and the electrode layer **2120** contains additional electrodes (two of which are labelled as **2170a, 2170b)** such that an individual electric field can be applied to each distinct sensing region, enabling an increase in sensitivity through concentrating charged species in the respective sensor regions. The example device is also depicted with a detector **2150** and having liquid dispenser containers **2160,** both similar to those described in Example 3. The device also comprises a processor (not shown) configured to calculate the concentration of the carbonyl containing compound in the fluid from the change in the absorption spectrum of the indicator.

In use, a breath sample is passed through opening **2165** and is directed through the device to three sorbent material comprising reaction areas **2135a, 2135b,** and **2135c** where the breath sample is exposed to the hydroxylamine salt and halochromic indicator. An electric field applied across each reaction zone by the electrodes in the electrode layer **2120** concentrates the charged species in sensing zones (two of which are labelled as **2190a, 2190b),** where the change in absorption spectrum is measured by the detector **2150.** The processor (not shown) calculates the carbonyl containing compound concentration in the breath sample from the change in absorption spectrum at one or more of the sensing zones (two of which are labelled as **2190a, 2190b).** Such a device configuration allows determination of a wide concentration of analyte due to the different sensitivity exposure areas of the sorbent material. For example, a high concentration of analyte may be determined more accurately from the change in absorption spectrum at a position of lower sensitivity to the analyte, whereas a lower concentration of analyte may be determined more accurately from the change in absorption spectrum at a position of higher sensitivity to the analyte.

*Example 5: Acetone detection system with hydroxylamine hydrochloride and a halochromic indicator in solution*

A reagent solution comprising hydroxylamine hydrochloride and a halochromic indicator was added to an absorbent material. Gas samples comprising various concentrations of acetone were exposed to the absorbent material for a period of 30 seconds, which approximates the duration of a standard human breath. The sensor response was measured at two different wavelengths (response 1 and 2) using a spectrophotometer. The results in Figure 22 show that the response changed as a function of the concentration of acetone in the gas.

It is clear that the ketone detection system could be used to provide a different response with different concentration of acetone. With higher concentration of acetone, the sensor could provide greater negative response. The resolution of this system is defined as

$$Resolution = Acetone\ concentration\ (ppm) / Sensor\ response$$

The resolution of the system is around 2 ppm for both responses (i.e. wavelength measurements) in a low concentration range, and 5 ppm for high concentration gas. It is thought that the negative value at concentration of 0 ppm is related to the drying effect that concentrates the reagent on the testing strip and provides a greater response for both channels.

*Example 6: Acetone detection system with hydroxylamine hydrochloride and a halochromic indicator pre-impregnated in the testing strip*

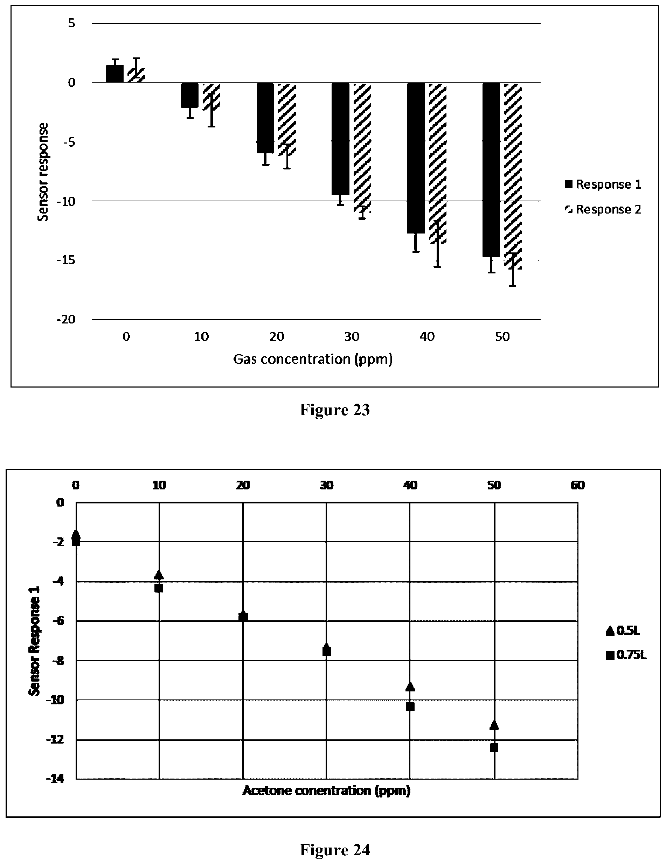

Hydroxylamine hydrochloride and a halochromic indicator were pre-impregnated in a testing strip. The strip was wetted with water to form a solution before the test. Gas samples comprising various concentrations of acetone were exposed to the absorbent material for a period of 30 seconds, which approximates the duration of a standard human breath. The sensor response was measured at two different wavelengths (response 1 and 2) using a spectrophotometer. The results in Figure 23 show that the response changed as a function of the concentration of acetone in the gas.

Figure 23 shows that the sensor response was greater for this device when comparing the results for the same acetone concentration exposure for the device described in Example 5 above. Although the resolution was higher in the low concentration range of the device results shown in Figure 23, there was some cross over between the responses at the higher concentration range, presumably because the chemical sensor was operating close to its saturation limit.

*Example 7: Acetone detection system with varying gas volume/flow rate*

Hydroxylamine hydrochloride and a halochromic indicator were added to a testing strip. The strip was prewetted with water. Gas samples comprising various concentrations of acetone were exposed to the absorbent material for a period of 30 seconds, at a flow rate of 0.5 L/min or 0.75 L/min. The sensor response was measured using a spectrophotometer. The results in Figure 24 show that the response changed as a function of the concentration of acetone in the gas, but the response did not substantially change as a function of the flow rate (or volume) of gas.

The chart illustrates that increasing the volume of gas by 50% will not significantly change the sensor response, and the response at the different concentrations does not overlap (for a particular flow rate). Although different gas volumes did not produce identical results, a higher volume sample with a lower acetone concentration will clearly not produce the same response as a lower volume sample with a higher ketone concentration. In other words, the ketone detection system is substantially fluid volume independent.

*Example 8: Acetone detection system with a weak base*

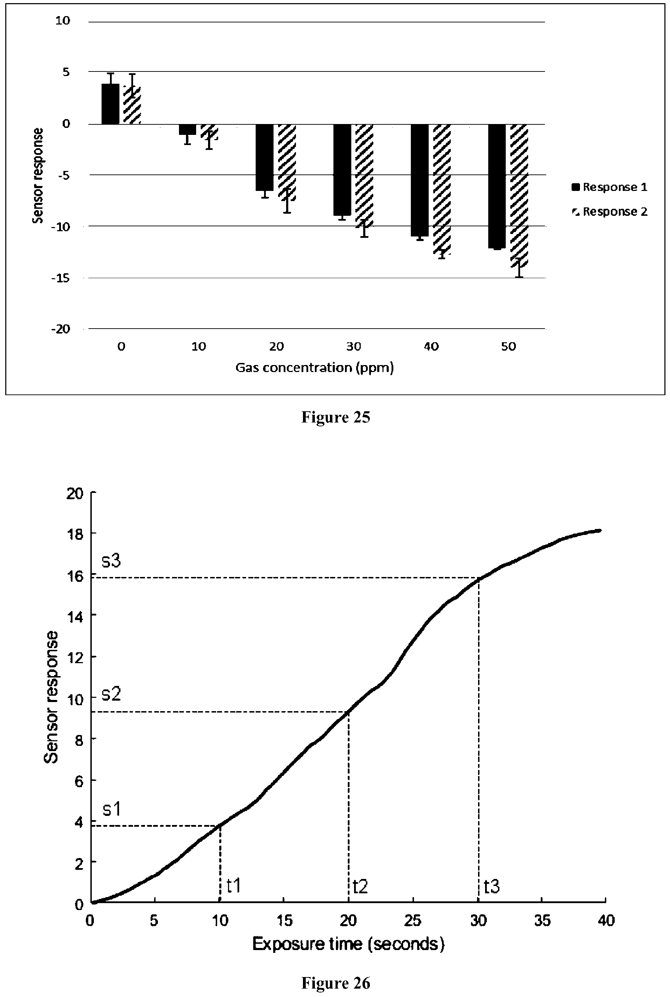

The acetone detection system described at Example 6 can be adjusted with the addition of a weak base to the testing strip. Base adjustment using sodium bicarbonate increased the resolution and accuracy for samples in the higher concentration range. Figure 25 shows the sensor response with base adjustment on the testing strip. The accuracy of the test could be estimated by the extent or presence of error bars crossing each other with samples having different gas concentrations, in which case the system cannot distinguish the concentration based on the sensor reading. The results in Figure 25 show that the addition of the weak base allows a higher accuracy at a high concentration range could be achieved when compared with the results of Figure 23 which show some cross over at 40 and 50 ppm. The resolution in the example system with the base adjustment (Figure 25) is 2 ppm - in the low concentration range and 5 ppm- in the higher concentration range tested.

*Example 9: Acetone sensor*

Figure 26 shows a response (i.e. a change in absorption spectrum of the indicator) plotted as a function of time for a position of an example sensor having a rectangular shape and using a 1.08 molL$^{-1}$ hydroxylamine hydrochloride solution. A TCS230 colour sensor was used to detect the red colour change as a function of time for the first 40 seconds after exposure of the sensor to a fluid with a 50 ppm - concentration of acetone.

Prototype structures to achieve breath ketone measurement based on the invention may vary. In general, it can be divided into two categories, with air storage and without air storage i.e. a pass-through approach. The former requires the user to breath into an intermediate storage bag, to contain a specified volume of breath. After this bag is filled, the breath is passed over the sorbent material at a controlled flow rate, to produce a signal response (i.e. a change in absorption spectrum of the indicator) as previously described. The latter requires the user to breathe directly over the sensor, without the use of intermediate storage, the volume of breath that the patient supplies can either be controlled, or measured, for calibration with the signal response produced by the device.

*Example 10: Acetone detection system with hydroxylamine hydrochloride and an ISFET pH sensor.*

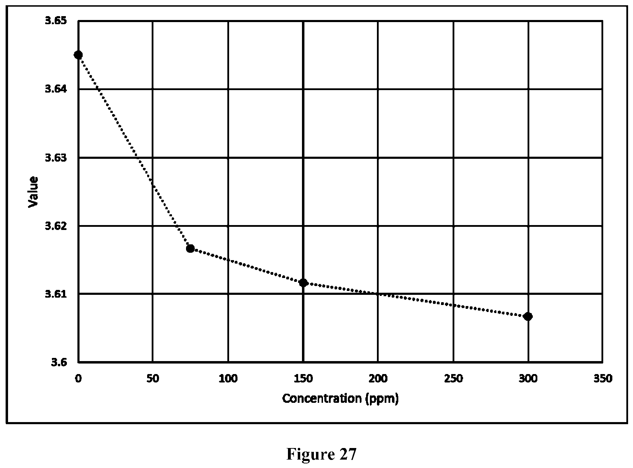

An experimental study was carried out based on the usage of an ISFEST pH sensor. The hydrogen ion sensing layer was made of silicon nitride, and the reference electrode was made of Ag/AgCl contained in potassium chloride solution (similar to the device design depicted in Figure 15(a)). The test was carried out at 26 °C at 65% humidity. In the experiment, hydroxylamine hydrochloride solution was added on a sorbent material strip before being exposed to a gas consisting of a mixture of air and acetone. Three concentrations of acetone gas (75, 150 and 300 ppm) were used in the testing at a gas flowrate of 1.2L/min for an exposure period of 30 seconds. After the reaction was finished, distilled water was added onto the strip. Both the ISFET sensor and the reference electrode were attached to the strip so as to produce a voltage reading. For each gas concentration, six measurements were performed and the average value was taken so as to minimize any experimental errors. The test results at different acetone concentrations are shown in Figure 27.

As can be seen from the results at Figure 27, the ISFET sensor shows a voltage drop with an increasing amount of acetone. This is due to an increasing amount of $H_3O^+$ through the reaction of acetone with the hydroxylamine hydro-chloride to produce $H_3O^+$. The sensor exhibits a voltage difference which is dependent upon the concentration of acetone in the gas sample. The resolution of the device was defined as follows:

$$Resolution = Acetone\ concentration\ (ppm)\ /\ Sensor\ response$$

Yielding a resolution for the device of around 2.65ppm/mV.

The skilled person will appreciate that the examples disclosed herewith with respect to sensors, systems and methods for determining the concentration of acetone samples could be adapted without undue burden to sensors, systems and methods for determining the concentration of a variety of other analytes.

**Claims**

**1.** A system for determining a concentration of acetone in a fluid, said system comprising:

a sensor configured to generate a differential response to a constant concentration of acetone, wherein the sensor comprises a hydroxylammonium salt, and whereby exposure of the fluid to the hydroxylammonium salt in the presence of a liquid produces a change in a variable, said variable being dependent upon the pH of the liquid;

and

an acquisition device configured to determine the concentration of the acetone from the differential response, wherein the acquisition device comprises:

> a detector for measuring the change in the variable; and
> a processor configured to calculate the concentration of the acetone in the fluid from the change in the variable, optionally wherein the fluid is breath;
> wherein the differential response is spatially differential, whereby the sensor is configured to respond to the acetone at a plurality of detection positions on the sensor, said detection positions having different sensitivities to the acetone; and
> wherein:
> the sensor comprises a spatially differential local concentration or local amount of the hydroxylammonium salt at different points of the sensor so as to provide spatially differential sensitivity to the sensor; or
> the sensor comprises a coating on a detection surface, and the coating has spatially differential permeability to the analyte so as to provide spatially differential sensitivity to the sensor.

2. The system of claim 1, further comprising a flow channel or an analysis chamber, wherein the sensor is disposed within said flow channel or analysis chamber.

3. The system of claim 1 or 2, wherein the variable is an absorbance; the sensor comprises a sorbent material, whereby the hydroxylammonium salt is sorbed in and/or on the sorbent material, and the sensor further comprises a halochromic indicator which is sorbed in and/or on the sorbent material; and the detector is a spectroscopic sensor;

> optionally wherein the sorbent material further comprises a weak base sorbed thereon and/or therein,
> optionally wherein the halochromic indicator is selected from the group consisting of methyl orange, methyl red, methyl yellow, chlorophenol red, bromocresol green, congo red, thymol blue, bromophenol blue, cresol red, metacresol purple, malachite green, ethyl violet, crystal violet, 2,4-dinitrophenol, orange IV, erythrosin B, p-(phenylazo)diphenylamine, p-phenylazoaniline and mixtures thereof.

4. The system of any one of claims 1 to 3, wherein the hydroxylammonium salt is hydroxylammonium chloride.

5. A sensor for sensing a concentration of acetone in a fluid, the sensor being as defined according to any one of claims 1 to 4.

6. A method for determining a concentration of acetone in a fluid, the method comprising:

> (a) exposing the fluid to a sensor as defined according to any one of claims 1 to 4, which is configured to generate a differential response to a constant concentration of acetone;
> (b) measuring a differential response of the sensor to the acetone; and
> (c) calculating the concentration of the acetone in the fluid utilising the differential response of the sensor to the acetone; wherein the method further comprises:
>
>> (i) exposing the fluid to a hydroxylammonium salt in the presence of a liquid, so as to produce a change in a variable, said variable being dependent upon the pH of the liquid;
>> (ii) measuring the change in the variable; and
>> (iii) determining the concentration of the acetone in the fluid from the change in the variable;

optionally wherein the fluid is breath;
wherein the differential response is spatially differential, whereby the sensor is configured to respond to the acetone at a plurality of detection positions on the sensor; said detection positions having different sensitivities to the acetone; and wherein said calculating utilises at least the following:

> • if the sensor reaches a saturated response level at all of the plurality of detection positions on the sensor: a response of the sensor to the acetone as a function of time at a detection position on the sensor having the least sensitivity to the acetone;
> • if the sensor does not reach a saturated response level at any of the plurality of detection positions on the sensor: a response of the sensor to the acetone at a detection position on the sensor having the greatest sensitivity to the acetone; or

• if the sensor responds to the acetone to reach a saturated response level at some, but not all, of the plurality of detection positions on the sensor:
one or more of:

a ratio of the number of detection positions on the sensor that reach a saturated response level to the number of detection positions on the sensor that do not reach a saturated response level,
a time required to reach a saturated response level for at least one detection position on the sensor that reaches a saturated response level, and
a response of the sensor to the acetone for at least one detection position on the sensor that does not reach a saturated response level.

7. The method of claim 6, wherein the variable is an absorbance; the sensor comprises a sorbent material, whereby the hydroxylammonium salt is sorbed in and/or on the sorbent material, and the sensor further comprises a halochromic indicator which is sorbed in and/or on the sorbent material; and the detector is a spectroscopic sensor;
optionally wherein the hydroxylammonium salt and/or the halochromic indicator are contacted with a weak base.

8. The method of claim 7, wherein the change in the absorbance is substantially independent of the volume of the fluid exposed to the hydroxylammonium salt, and/or the change in the absorbance is substantially independent of the flow rate of the fluid exposed to the hydroxylammonium salt.

9. The method of any one of claims 6 to 8, wherein the liquid volume is about 1 mL or less.

10. Use of the system of any one of claims 1 to 4 for diagnosing and/or managing diabetes in a patient, wherein the system is capable of determining a concentration of acetone in a breath sample of the patient, and the system comprises:
a hydroxylammonium salt, whereby exposure of the breath to the hydroxylammonium salt in the presence of a liquid produces a change in a variable, said variable being dependent upon the pH of the liquid;

a detector for measuring the change in the variable; and
a processor configured to calculate the concentration of the acetone in the breath from the change in the variable.

11. A method of managing diabetes in a patient on a diabetes treatment plan, said method comprising:

(a) determining an acetone concentration of a breath sample of the patient using the method of claim 6;
(b) comparing the acetone concentration to a reference acetone concentration range; and
(c) if the acetone concentration is outside the acetone concentration reference range, adjusting the diabetes treatment plan.

12. A method to assist in diagnosing diabetes in a patient, said method comprising:

(a) determining an acetone concentration of a breath sample of the patient using the method of claim 6; and
(b) comparing the acetone concentration to a reference acetone concentration range to determine if the patient has diabetes.

**Patentansprüche**

1. System zur Bestimmung einer Acetonkonzentration in einem Fluid, wobei das System umfasst:

einen Sensor, der so konfiguriert ist, dass er ein differentielles Ansprechen auf eine konstante Acetonkonzentration erzeugt, wobei der Sensor ein Hydroxylammoniumsalz umfasst und wobei die Einwirkung des Hydroxylammoniumsalzes auf das Fluid in Gegenwart einer Flüssigkeit eine Änderung einer Variablen erzeugt, wobei die Variable vom pH-Wert der Flüssigkeit abhängig ist; und
eine Erfassungsvorrichtung, die so konfiguriert ist, dass sie die Konzentration des Analyten aus dem differentiellen Ansprechen bestimmt, wobei die Erfassungsvorrichtung umfasst:

einen Detektor zum Messen der Änderung der Variablen und
einen Prozessor, der so konfiguriert ist, dass er die Konzentration des Acetons in dem Fluid aus der Änderung der Variablen berechnet, wobei das Fluid optional Atem ist,

wobei das differentielle Ansprechen räumlich differentiell ist, wobei der Sensor so konfiguriert ist, dass er auf das Aceton an einer Mehrzahl von Detektionspositionen auf dem Sensor anspricht, wobei die Detektionspositionen unterschiedliche Sensitivitäten für das Aceton aufweisen, und wobei:

der Sensor eine räumlich differentielle lokale Konzentration oder lokale Menge des Hydroxylammoniumsalzes an unterschiedlichen Punkten des Sensors umfasst, um so dem Sensor eine räumlich differentielle Sensitivität zu verleihen, oder
der Sensor eine Beschichtung auf einer Detektionsfläche umfasst, und die Beschichtung eine räumlich differentielle Durchlässigkeit für den Analyten aufweist, um so dem Sensor eine räumlich differentielle Sensitivität zu verleihen.

2. System nach Anspruch 1, welches ferner einen Strömungskanal oder eine Analysekammer umfasst, wobei der Sensor innerhalb des Strömungskanals oder der Analysekammer angeordnet ist.

3. System nach Anspruch 1 oder 2, wobei die Variable eine Absorption ist; der Sensor ein Sorptionsmaterial umfasst, wobei das Hydroxylammoniumsalz in und/oder auf dem Sorptionsmaterial sorbiert ist, und der Sensor ferner einen halochromen Indikator umfasst, der in und/oder auf dem Sorptionsmaterial sorbiert ist; und der Detektor ein spektroskopischer Sensor ist;

optional, wobei das Sorptionsmaterial weiterhin eine darauf und/oder darin sorbierte schwache Base umfasst, optional, wobei der halochrome Indikator ausgewählt ist aus der Gruppe bestehend aus Methylorange, Methylrot, Methylgelb, Chlorphenolrot, Bromkresolgrün, Kongorot, Thymolblau, Bromphenolblau, Kresolrot, Metakresolviolett, Malachitgrün, Ethylviolett, Kristallviolett, 2,4-Dinitrophenol, Orange IV, Erythrosin B, p-(Phenylazo)diphenylamin, p-Phenylazoanilin und Mischungen davon.

4. System nach einem der Ansprüche 1 bis 3, wobei das Hydroxylammoniumsalz Hydroxylammoniumchlorid ist.

5. Sensor zum Erfassen einer Acetonkonzentration in einem Fluid, wobei der Sensor gemäß einem der Ansprüche 1 bis 4 definiert ist.

6. Verfahren zur Bestimmung einer Acetonkonzentration in einem Fluid, wobei das Verfahren umfasst:

(a) Aussetzen des Fluids gegenüber einem Sensor wie gemäß einem der Ansprüche 1 bis 4 definiert, der so konfiguriert ist, dass er ein differentielles Ansprechen auf eine konstante Acetonkonzentration erzeugt;
(b) Messen eines differentiellen Ansprechens des Sensors auf das Aceton; und
c) Berechnen der Konzentration des Acetons in dem Fluid unter Verwendung des differentiellen Ansprechens des Sensors auf das Aceton;
wobei das Verfahren ferner umfasst:

(i) Aussetzen des Fluids gegenüber einem Hydroxylammoniumsalz in Gegenwart einer Flüssigkeit, um eine Änderung einer Variablen zu erzeugen, wobei die Variable vom pH-Wert der Flüssigkeit abhängig ist;
(ii) Messen der Änderung der Variablen; und
(iii) Bestimmen der Konzentration des Acetons in dem Fluid aus der Änderung der Variablen;

optional, wobei das Fluid Atem ist;
wobei das differentielle Ansprechen räumlich differentiell ist, wobei der Sensor so konfiguriert ist, dass er auf das Aceton an einer Mehrzahl von Detektionspositionen auf dem Sensor anspricht, wobei die Detektionspositionen unterschiedliche Sensitivitäten für das Aceton aufweisen; und wobei das Berechnen mindestens das Folgende verwendet:

• wenn der Sensor ein gesättigtes Ansprechniveau an allen der Mehrzahl von Detektionspositionen auf dem Sensor erreicht:
ein Ansprechen des Sensors auf das Aceton als Funktion der Zeit an einer Detektionsposition auf dem Sensor mit der geringsten Sensitivität gegenüber dem Aceton;
• wenn der Sensor an keiner der Mehrzahl von Detektionspositionen auf dem Sensor ein gesättigtes Ansprechniveau erreicht:
ein Ansprechen des Sensors auf das Aceton an einer Detektionsposition auf dem Sensor mit der größten Sensitivität gegenüber dem Aceton; oder
• wenn der Sensor auf das Aceton anspricht, um ein gesättigtes Ansprechniveau an einigen, aber nicht allen, der Mehrzahl von Detektionspositionen auf dem Sensor zu erreichen:
eines oder mehrere von:

einem Verhältnis der Anzahl von Detektionspositionen auf dem Sensor, die ein gesättigtes Ansprechniveau erreichen, zu der Anzahl von Detektionspositionen auf dem Sensor, die kein gesättigtes Ansprechniveau erreichen,
eine Zeit, die erforderlich ist, um ein gesättigtes Ansprechniveau für mindestens eine Detektionsposition auf dem Sensor zu erreichen, die ein gesättigtes Ansprechniveau erreicht, und
ein Ansprechen des Sensors auf das Aceton für mindestens eine Detektionsposition auf dem Sensor, die kein gesättigtes Ansprechniveau erreicht

7. Verfahren nach Anspruch 6, wobei die Variable eine Absorption ist; der Sensor ein Sorptionsmaterial umfasst, wobei das Hydroxylammoniumsalz in und/oder auf dem Sorptionsmaterial sorbiert ist, und der Sensor ferner einen halochromen Indikator umfasst, der in und/oder auf dem Sorptionsmaterial sorbiert ist; und der Detektor ein spektroskopischer Sensor ist,
optional wobei das Hydroxylammoniumsalz und/oder der halochrome Indikator mit einer schwachen Base in Kontakt gebracht werden.

8. Verfahren nach Anspruch 7, wobei die Änderung der Absorption im Wesentlichen unabhängig vom Volumen des dem Hydroxylammoniumsalz ausgesetzten Fluids ist und/oder die Änderung der Absorption im Wesentlichen unabhängig von der Flussrate des dem Hydroxylammoniumsalz ausgesetzten Fluids ist.

9. Verfahren nach einem der Ansprüche 6 bis 8, wobei das Flüssigkeitsvolumen etwa 1 mL oder weniger beträgt.

10. Verwendung des Systems nach einem der Ansprüche 1 bis 4 zur Diagnose und/oder Verwaltung von Diabetes bei einem Patienten, wobei das System in der Lage ist, eine Acetonkonzentration in einer Atemprobe des Patienten zu bestimmen, und das System umfasst:

ein Hydroxylammoniumsalz, wobei die Einwirkung des Hydroxylammoniumsalzes auf den Atem in Gegenwart einer Flüssigkeit eine Änderung einer Variablen erzeugt, wobei die Variable vom pH-Wert der Flüssigkeit abhängig ist;
einen Detektor zum Messen der Änderung der Variablen; und
einen Prozessor, der so konfiguriert ist, dass er die Acetonkonzentration in dem Atem aus der Änderung der Variablen berechnet.

11. Verfahren zur Verwaltung von Diabetes bei einem Patienten mit einem Diabetes-Behandlungsplan, wobei das Verfahren umfasst:

(a) Bestimmen einer Acetonkonzentration einer Atemprobe des Patienten unter Verwendung des Verfahrens von Anspruch 6;
(b) Vergleichen der Acetonkonzentration mit einem Acetonkonzentrations-Referenzbereich; und
(c) wenn die Acetonkonzentration außerhalb des Acetonkonzentrations-Referenzbereichs liegt, Anpassen des Diabetes-Behandlungsplans.

12. Verfahren zur Unterstützung bei der Diagnose von Diabetes bei einem Patienten, wobei das Verfahren umfasst:

(a) Bestimmen einer Acetonkonzentration einer Atemprobe des Patienten unter Verwendung des Verfahrens von Anspruch 6; und
(b) Vergleichen der Acetonkonzentration mit einem Acetonkonzentrations-Referenzbereich, um festzustellen, ob der Patient Diabetes hat.

## Revendications

1. Système pour déterminer une concentration d'acétone dans un fluide, ledit système comprenant :

un capteur configuré pour générer une réponse différentielle à une concentration constante d'acétone, dans lequel le capteur comprend un sel d'hydroxylammonium, et moyennant quoi une exposition du fluide au sel d'hydroxylammonium en présence d'un liquide produit un changement dans une variable, ladite variable dépendant du pH du liquide ; et

un dispositif d'acquisition configuré pour déterminer la concentration de l'acétone à partir de la réponse différentielle, dans lequel le dispositif d'acquisition comprend :

un détecteur pour mesurer le changement de la variable ; et

un processeur configuré pour calculer la concentration de l'acétone dans le fluide à partir du changement de la variable, facultativement dans lequel le fluide est l'haleine ;

dans lequel la réponse différentielle est spatialement différentielle, moyennant quoi le capteur est configuré pour répondre à l'acétone dans une pluralité de positions de détection sur le capteur, lesdites positions de détection présentant des sensibilités différentes à l'acétone ; et

dans lequel :

le capteur comprend une concentration locale spatialement différentielle ou une quantité locale du sel d'hydroxylammonium en différents points du capteur de manière à fournir une sensibilité spatialement différentielle au capteur ; ou

le capteur comprend un revêtement sur une surface de détection, et le revêtement présente une perméabilité spatialement différentielle à l'analyte de manière à fournir une sensibilité spatialement différentielle au capteur.

2. Système selon la revendication 1, comprenant en outre un canal d'écoulement ou une chambre d'analyse, dans lequel le capteur est disposé à l'intérieur dudit canal d'écoulement ou de ladite chambre d'analyse.

3. Système selon la revendication 1 ou 2, dans lequel la variable est une absorbance ; le capteur comprend un matériau sorbant, moyennant quoi le sel d'hydroxylammonium est sorbé dans et/ou sur le matériau sorbant, et le capteur comprend en outre un indicateur halochromique qui est sorbé dans et/ou sur le matériau sorbant ; et le détecteur est un capteur spectroscopique ;

facultativement dans lequel le matériau sorbant comprend en outre une base faible sorbée sur et/ou dans celui-ci, facultativement dans lequel l'indicateur halochromique est choisi dans le groupe constitué de l'orange de méthyle, du rouge de méthyle, du jaune de méthyle, du rouge de chlorophénol, du vert de bromocrésol, du rouge de congo, du bleu de thymol, du bleu de bromophénol, du rouge de crésol, du violet de métacrésol, du vert de malachite, du violet d'éthyle, du violet de cristal, du 2,4-dinitrophénol, de l'orange IV, de l'érythrosine B, de la p-(phénylazo)diphénylamine, de la p-phénylazoaniline, et de mélanges de ceux-ci.

4. Système selon l'une quelconque des revendications 1 à 3, dans lequel le sel d'hydroxylammonium est le chlorure d'hydroxylammonium.

5. Capteur pour détecter une concentration d'acétone dans un fluide, le capteur étant tel que défini selon l'une quelconque des revendications 1 à 4.

6. Procédé de détermination d'une concentration d'acétone dans un fluide, le procédé comprenant les étapes consistant à :

(a) exposer le fluide à un capteur selon l'une quelconque des revendications 1 à 4, qui est configuré pour générer une réponse différentielle à une concentration constante d'acétone ;

(b) mesurer une réponse différentielle du capteur à l'acétone ; et

(c) calculer la concentration de l'acétone dans le fluide en utilisant la réponse différentielle du capteur à l'acétone ;

dans lequel le procédé comprend en outre les étapes consistant à :

(i) exposer le fluide à un sel d'hydroxylammonium en présence d'un liquide, de manière à produire un changement dans une variable, ladite variable dépendant du pH du liquide ;

(ii) mesurer la variation de la variable ; et

(iii) déterminer la concentration de l'acétone dans le fluide à partir du changement de la variable ;

facultativement dans lequel le fluide est l'haleine ;

dans lequel la réponse différentielle est spatialement différentielle, moyennant quoi le capteur est configuré pour répondre à l'acétone dans une pluralité de positions de détection sur le capteur ; lesdites positions de détection présentant des sensibilités différentes à l'acétone ; et dans lequel ledit calcul utilise au moins les éléments suivants :

• si le capteur atteint un niveau de réponse saturé dans la totalité de la pluralité de positions de détection sur le capteur :
une réponse du capteur à l'acétone en fonction du temps dans une position de détection sur le capteur présentant la moindre sensibilité à l'acétone ;
• si le capteur n'atteint pas un niveau de réponse saturé dans l'une quelconque de la pluralité de positions de détection sur le capteur :
une réponse du capteur à l'acétone dans une position de détection sur le capteur présentant la plus grande sensibilité à l'acétone ; ou
• si le capteur répond à l'acétone pour atteindre un niveau de réponse saturé dans certaines, mais pas toutes, de la pluralité de positions de détection sur le capteur :
un ou plusieurs parmi :

un rapport du nombre de positions de détection sur le capteur qui atteignent un niveau de réponse saturé au nombre de positions de détection sur le capteur qui n'atteignent pas un niveau de réponse saturé, un temps nécessaire pour atteindre un niveau de réponse saturé pour au moins une position de détection sur le capteur qui atteint un niveau de réponse saturé, et une réponse du capteur à l'acétone pour au moins une position de détection sur le capteur qui n'atteint pas un niveau de réponse saturé.

7. Procédé selon la revendication 6, dans lequel la variable est une absorbance ; le capteur comprend un matériau sorbant, moyennant quoi le sel d'hydroxylammonium est sorbé dans et/ou sur le matériau sorbant, et le capteur comprend en outre un indicateur halochromique qui est sorbé dans et/ou sur le matériau sorbant ; et le détecteur est un capteur spectroscopique ;
facultativement dans lequel le sel d'hydroxylammonium et/ou l'indicateur halochromique sont mis en contact avec une base faible.

8. Procédé selon la revendication 7, dans lequel le changement de l'absorbance est sensiblement indépendant du volume du fluide exposé au sel d'hydroxylammonium, et/ou le changement de l'absorbance est sensiblement indépendant du débit d'écoulement du fluide exposé au sel d'hydroxylammonium.

9. Procédé selon l'une quelconque des revendications 6 à 8, dans lequel le volume de liquide est d'environ 1 mL ou moins.

10. Utilisation du système selon l'une quelconque des revendications 1 à 4 pour diagnostiquer et/ou gérer le diabète chez un patient, dans lequel le système est capable de déterminer une concentration d'acétone dans un échantillon d'haleine du patient, et le système comprend :

un sel d'hydroxylammonium, dans lequel l'exposition de l'haleine au sel d'hydroxylammonium en présence d'un liquide produit un changement dans une variable, ladite variable dépendant du pH du liquide ;
un détecteur pour mesurer le changement de la variable ; et
un processeur configuré pour calculer la concentration de l'acétone dans l'haleine à partir du changement de la variable.

11. Procédé de gestion du diabète chez un patient sur un plan de traitement du diabète, ledit procédé comprenant les étapes consistant à :

(a) déterminer une concentration d'acétone d'un échantillon d'haleine du patient en utilisant le procédé de la revendication 6 ;
b) comparer la concentration d'acétone à une plage de concentration d'acétone de référence ; et
(c) si la concentration d'acétone est en dehors de la plage de concentration d'acétone de référence, ajuster le plan de traitement du diabète.

12. Procédé pour aider à diagnostiquer le diabète chez un patient, ledit procédé comprenant les étapes consistant à :

(a) déterminer une concentration d'acétone d'un échantillon d'haleine du patient en utilisant le procédé de la revendication 6 ; et

b) comparer la concentration d'acétone à une plage de concentration d'acétone de référence pour déterminer si le patient est diabétique.

# Continous arrangement

# Discrete arrangement

High sensitivity

Low sensitivity

Figure 1

Analyte input

Treatment

Local analyte concentration

Low

Medium

High

Sensor

0 difference

Figure 2

Figure 3

**a)**

Sensor
Response

Saturated level —

$R_p$

0 —

Time

**b)**

Sensor
Response

Saturated level —

0 —

Time

**c)**

Sensor
Response

Saturated level —

$R_{ss}=R_p$

*Steady state*

0 —

Time

**d)**

Sensor
Response

Saturated level —

$R_p$
$R_{ss}$

*Steady state*

0 —

Time

**e)**

Sensor
Response

Saturated level —

0 —

Time

Figure 4

(a)

Segment A          Segment B

Response

Low          Sensitivity          High

(b)

100%

L

Segment A (d)                    Segment B (L-d)

Signal

m1

0%

d

Low          Sensor sensitivity/distance across sensor          High

(c)

Segment A                    Segment B

Time to
reach 100%

Response is
N/A since
100% has not
been reached

m2

Low          Sensitivity/distance across sensor          High

Figure 5

Figure 6

Figure 7

Figure 8

900

Breath inlet

901

902

903

904

910

905

906

907

908

909

912

913

911

914

915

Cloud storage data
Publicly distributed ledger
Blockchain technology
Record date/time/user info/ketone level
Available to medical practitioners
globally

User ketone level
Smart phone
display

Computational
Neural Network

Clinical training
data

Software updates

Smart
device

Wired or wireless

Figure 9

a)

1010

1000

1020

1030

b)

1040

1050a

1001

1050

1050b

1060

1050c

Figure 10

Figure 11

Figure 12

a)

Analyte
Concentration
——— high
- - - - med
.......... low

b)

Figure 13

a)

b)

Figure 14

Figure 15

Figure 16

Figure 17

Figure 18

Figure 19

Figure 20

**Figure 21**

**Figure 22**

Figure 23

Figure 24

Figure 25

Figure 26

Figure 27

**EP 3 847 459 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- SU 877425 A1
- BR 8704258 A

- WO 2018081877 A